# EUROPEAN PATENT APPLICATION

(11) **EP 2 527 337 A1**
(43) Date of publication of application: **28.11.2012**
(21) Application number: 12175530.0
(22) Date of filing: 12.04.2006
(51) Int. Cl.: C07D 401/12, C07D 401/14, C07D 401/04, A61K 31/444, A61P 3/00, A61P 9/00, A61P 25/00, C07D 213/70, C07D 213/71, C07D 213/81, C07D 213/82, C07D 401/06, C07D 405/04, C07D 405/14, C07D 409/04, C07D 413/14, C07D 487/04, C07D 487/08

(54) **Inhibitors of 11-beta hydroxysteroid dehydrogenase type I**

(30) Priority: 14.04.2005 US 671174 P
(62) Divisional of application: 06749854.3
(71) Applicant: Bristol-Myers Squibb Company, Princeton, NJ 08543 (US)
(72) Inventor: Li, James, J., Pennington, NJ New Jersey 08534 (US); Hamann, Lawrence, G., North Grafton, MA Massachusetts 01536 (US); Ruan, Zheming, Dayton, NJ New Jersey 08810 (US); Cooper, Christopher, B., Lawrenceville, NJ New Jersey 08648 (US); Wu, Shung, C., Princeton, NJ New Jersey 08540 (US); Simpkins, Ligaya, M., Titusville, NJ New Jersey 08540 (US); Wang, Haixia, Princeton Junction, NJ New Jersey 08550 (US); Nayeem, Akbar, Newtown, PA Pennsylvania 18940 (US); Krystek, Stanley, R., Jr., Ringoes, NJ New Jersey 08551 (US)
(74) Representative: Beacham, Annabel Rose

(57) **Abstract**

Novel compounds are provided which are 11-beta-hydroxysteroid dehydrogenase type I inhibitors. 11-beta-hydroxysteroid dehydrogenase type I inhibitors are useful in treating, preventing, or slowing the progression of diseases requiring 11-beta-hydroxysteroid dehydrogenase type I inhibitor therapy. These novel compounds have the structure: 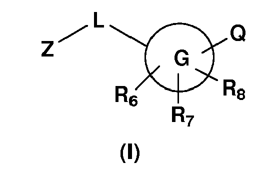 or stereoisomers or prodrugs or pharmaceutically acceptable salts thereof, wherein G, L, Q, Z, R₆, R₇, and R₈ are defined herein.

## Description

### BACKGROUND OF THE INVENTION

The steroid hormone cortisol is a key regulator of many physiological processes. However, an excess of cortisol, as occurs in Cushing's Disease, provokes severe metabolic abnormalities including: type 2 diabetes, cardiovascular disease, obesity, and osteoporosis. Many patients with these diseases, however, do not show significant increases in plasma cortisol levels. In addition to plasma cortisol, individual tissues can regulate their glucocorticoid tone via the in situ conversion of inactive cortisone to the active hormone cortisol. Indeed, the normally high plasma concentration of cortisone provides a ready supply of precursor for conversion to cortisol via the intracellular enzyme 11-beta-hydroxysteroid dehydrogenase type I (11beta-HSD1).

11beta-HSD1 is a member of the short chain dehydrogenase superfamily of enzymes. By catalyzing the conversion of biologically inactive cortisone to cortisol, 11beta-HSD1 controls the intracellular glucocorticoid tone according to its expression and activity levels. In this manner, 11beta-HSD1 can determine the overall metabolic status of the organ. 11beta-HSD1 is expressed at high levels in the liver and at lower levels in many metabolically active tissues including the adipose, the CNS, the pancreas, and the pituitary. Taking the example of the liver, it is predicted that high levels of 11beta-HSD1 activity will stimulate gluconeogenesis and overall glucose output. Conversely, reduction of 11beta-HSD1 activity will downregulate gluconeogenesis resulting in lower plasma glucose levels.

Various studies have been conducted that support this hypothesis. For example, transgenic mice expressing 2X the normal level of 11beta-HSD1 in only the adipose tissue show abdominal obesity, hyperglycemia, and insulin resistance. (H. Masuzaki, J. Paterson, H. Shinyama, N.M. Morton, J.J. Mullins, J.R. Seckl, J.S. Flier, A Transgenic Model of Visceral Obesity and the Metabolic Syndrome, Science 294:2166-2170 (2001). Conversely, when the 11beta-HSD1 gene is ablated by homologous recombination, the resulting mice are resistant to diet induced obesity and the accompanying dysregulation of glucose metabolism (N.M. Morton, J.M. Paterson, H. Masuzaki, M.C. Holmes, B. Staels, C. Fievet, B.R. Walker, J.S. Flier, J.J. Mullings, J.R. Seckl, Novel Adipose Tissue-Mediated Resistance to Diet-induced Visceral Obesity in 11β-Hydroxysteroid Dehydrogenase Type 1-Deficient Mice. Diabetes 53: 931-938 (2004). In addition, treatment of genetic mouse models of obesity and diabetes (ob/ob, db/db and KKAy mice) with a specific inhibitor of 11beta-HSD1 causes a decrease in glucose output from the liver and an overall increase in insulin sensitivity (P. Alberts, C. Nilsson, G. Selen, L.O.M. Engblom, N.H.M. Edling, S. Norling, G. Klingstrom, C. Larsson, M. Forsgren, M. Ashkzari, C.E. Nilsson, M. Fiedler, E. Bergqvist, B. Ohman, E. Bjorkstrand, L.B. Abrahmsen, Selective Inhibition of 11β-Hydroxysteroid Dehydrogenase Type I Improves Hepatic Insuling Sensuitivity in Hyperglycemic Mice Strains, Endocrinology 144: 4755-4762 (2003)). Furthermore, inhibitors of 11beta-HSD1 have been shown to be effective in treating metabolic syndrome and atherosclerosis in high fat fed mice (Hermanowoki-Vosetka et. al., J. Eg. Med., 2002, 202(4), 517-527). Based in part on these studies, it is believed that local control of cortisol levels is important in metabolic diseases in these model systems. In addition, the results of these studies also suggest that inhibition of 11beta-HSD1 will be a viable strategy for treating metabolic diseases such as type 2 diabetes, obesity, and the metabolic syndrome.

Lending further support to this idea are the results of a series of preliminary clinical studies. For example, several reports have shown that adipose tissue from obese individuals has elevated levels of 11beta-HSD1 activity. In addition, studies with carbenoxolone, a natural product derived from licorice that inhibits both 11beta-HSD1 and 11beta-HSD2 (converts cortisol to cortisone in kidney) have shown promising results. A seven day, double blind, placebo controlled, cross over study with carbenoxolone in mildly overweight individuals with type 2 diabetes showed that patients treated with the inhibitor, but not the placebo group, displayed a decrease in hepatic glucose production (R.C. Andrews, O. Rooyackers, B.R. Walker, J. Clin. Endocrinol. Metab. 88: 285-291 (2003)). This observation is consistent with the inhibition of 11beta-HSD1 in the liver. The results of these preclinical and early clinical studies strongly support the concept that treatment with a potent and selective inhibitor of 11beta-HSD1 will be an efficacious therapy in patients afflicted with type 2 diabetes, obesity, and the metabolic syndrome.

### SUMMARY OF THE INVENTION

In accordance with the present invention, aryl and heteroaryl and related compounds are provided that have the general structure of formula I: 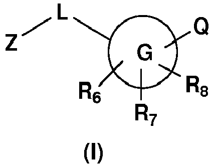 wherein G, L, Q, Z, R₆, R₇, and R₈ are defined below.

The compounds of the present invention inhibit the activity of the enzyme 11-beta-hydroxysteroid dehydrogenase type I. Consequently, the compounds of the present invention may be used in the treatment of multiple diseases or disorders associated with 11-beta-hydroxysteroid dehydrogenase type I, such as diabetes and related conditions, microvascular complications associated with diabetes, the macrovascular complications associated with diabetes, cardiovascular diseases, Metabolic Syndrome and its component conditions, and other maladies. Examples of diseases or disorders associated with the activity of the enzyme 11-beta-hydroxysteroid dehydrogenase type I that can be prevented, inhibited, or treated according to the present invention include, but are not limited to, diabetes, hyperglycemia, impaired glucose tolerance, insulin resistance, hyperinsulinemia, retinopathy, neuropathy, nephropathy, delayed wound healing, atherosclerosis and its sequelae, abnormal heart function, myocardial ischemia, stroke, Metabolic Syndrome, hypertension, obesity, dislipidemia, dylsipidemia, hyperlipidemia, hypertriglyceridemia, hypercholesterolemia, low HDL, high LDL, non-cardiac ischemia, infection, cancer, vascular restenosis, pancreatitis, neurodegenerative disease, lipid disorders, cognitive impairment and dementia, bone disease, HIV protease associated lipodystrophy and glaucoma.

The present invention provides for compounds of formula I, pharmaceutical compositions employing such compounds, and for use of such compounds in therapy. In particular, the present invention provides a pharmaceutical composition comprising a therapeutically effective amount of a compound of formula I, alone or in combination with a pharmaceutically acceptable carrier.

Further, in accordance with the present invention, a method is provided for preventing, inhibiting, or treating the progression or onset of diseases or disorders associated with the activity of the enzyme 11-beta-hydroxysteroid dehydrogenase type I, such as defined above and hereinafter, wherein a therapeutically effective amount of a compound of formula I is administered to a mammalian, i.e., human, patient in need of treatment.

The compounds of the invention can be used alone, in combination with other compounds of the present invention, or in combination with one or more other agent(s).

Further, the present invention provides a method for preventing, inhibiting, or treating the diseases as defined above and hereinafter, wherein a therapeutically effective amount of a combination of a compound of formula I and another compound of formula I and/or at least one other type of therapeutic agent, is administered to a mammalian, i.e., human, patient in need of treatment.

### DESCRIPTION OF THE INVENTION

In accordance with the present invention, compounds of formula I are provided 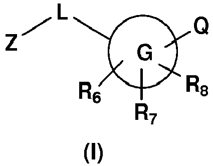 or stereoisomers or prodrugs or pharmaceutically acceptable salts thereof, wherein:
Z is aryl or heterocyclyl group, and may be optionally substituted with R₁, R₂, R₃, R₄, and R₅ at any available positions;
R₁, R₂, R₃, R₄, and R₅ are independently hydrogen, halo, cyano, haloalkyl, haloalkoxy, nitro, alkyl, alkenyl, alkynyl, cycloalkyl, alkoxy, alkylthio, alkylsulfonyl, arylsulfonyl, alkylamino, -C(O)R₉, -NR₉C(O)R₉ₐ, -NR₉R₉ₐ, aryl, arylalkyl, aryloxy, or heterocyclyl, wherein the haloalkyl, haloalkoxy, alkyl, alkenyl, alkynyl, cycloalkyl, alkoxy, alkylthio, alkylsulfonyl, arylsulfonyl, alkylamino, aryl, arylalkyl, or heterocyclyl, may be optionally substituted with R₉ and R₉ₐ;
   or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, alkenyl, alkynyl, cycloalkyl, -C(O)NR₉R₉ₐ, -C(O)R₉, -NR₉C(O)R₉ₐ, aryl, aryloxy, or heterocyclyl, wherein the haloalkyl, alkyl, alkenyl, alkynyl, cycloalkyl, aryl, aryloxy, or heterocyclyl may be optionally substituted with R₉ and R₉ₐ; and
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl;
L is a bond, O, S, SO₂, SO₂NR₄ₐ, NR₄ₐ, OCR₄ₐR_{4b}, CR₄ₐR_{4b}O, SCR₄ₐR_{4b}, CR₄ₐR_{4b}S, SO₂CR₄ₐR_{4b}, CR₄ₐR_{4b}SO₂, CR₄ₐR_{4b}CR_{4c}R_{4d}, CR₄ₐ=CR_{4b}, or OCONR_{4b};
R₄ₐ, R_{4b}, R_{4c}, and R_{4d} are independently hydrogen, alkyl or haloalkyl, wherein the alkyl and haloalkyl may be optionally substituted with R₁₀, R₁₀ₐ, R_{10b}, and R_{10c};
G is a 5- or 6-membered heteroaryl containing at least one nitrogen;
R₆, R₇, and R₈ are independently hydrogen, halo, haloalkyl, haloalkoxy, alkyl, aryl, heterocyclyl, alkoxy, aryloxy;
Q is CONR₁₁R₁₁ₐ, or OCONR₁₁R₁₁ₐ;
R₁₁ and R₁₁ₐ are independently hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c};
or R₁₁ and R₁₁ₐ may be taken together with the nitrogen to which they are attached to form a heterocyclyl ring, which may be optionally substituted with R₁₀, R₁₀ₐ, R_{10b}, and R_{10c}.

In another embodiment, compounds of formula I are those in which L is a bond, O, S, OCR₄ₐR_{4b}, SCR₄ₐR_{4b}, CR₄ₐR_{4b}S, SO₂CR₄ₐR_{4b}, CR₄ₐR_{4b}SO₂, CR₄ₐR_{4b}CR_{4c}R_{4d}, or CR₄ₐ=CR_{4b}.

In another embodiment, compounds of formula I are those in which L is a bond, OCR₄ₐR_{4b}, SCR₄ₐR_{4b}, CR₄ₐR_{4b}S, SO₂CR₄ₐR_{4b}, CR₄ₐR_{4b}SO₂, or CR₄ₐ=CR_{4b}.

In another embodiment, compounds of formula I are those in which L is OCR₄ₐR_{4b}, SCR₄ₐR_{4b}, CR₄ₐR_{4b}S, SO₂CR₄ₐR_{4b}, CR₄ₐR_{4b}SO₂, or CR₄ₐ=CR_{4b}.

In another embodiment, compounds of formula I are those in which L is CR₄ₐR_{4b}S, SO₂CR₄ₐR_{4b}, CR₄ₐR_{4b}SO₂, or CR₄ₐ=CR_{4b}.

In yet another embodiment, compounds of formula I are those in which L is CR₄ₐR_{4b}S, CR₄ₐR_{4b}SO₂, or CR₄ₐ=CR_{4b}.

In another embodiment, compounds of formula I are those in which:
Z is aryl or heterocyclyl group, and may be optionally substituted with R₁, R₂, R₃, R₄, and R₅ at any available positions;
R₁, R₂, R₃, R₄, and R₅ are independently hydrogen, halo, cyano, haloalkyl, haloalkoxy, nitro, alkyl, alkenyl, alkynyl, cycloalkyl, alkoxy, alkylthio, alkylsulfonyl, arylsulfonyl, alkylamino, -C(O)R₉, -NR₉C(O)R₉ₐ, -NR₉R₉ₐ, aryl, arylalkyl, aryloxy, or heterocyclyl, wherein the haloalkyl, haloalkoxy, alkyl, alkenyl, alkynyl, cycloalkyl, alkoxy, alkylthio, alkylsulfonyl, arylsulfonyl, alkylamino, aryl, arylalkyl, or heterocyclyl, may be optionally substituted with R₉ and R₉ₐ;
   or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R_{10c};
L is bond, O, S, SO₂, OCR₄ₐR_{4b}, CR₄ₐR_{4b}O, SCR₄ₐR_{4b}, CR₄ₐR_{4b}S, SO₂CR₄ₐR_{4b}, CR₄ₐR_{4b}SO₂, CR₄ₐR_{4b}CR_{4c}R_{4d}, CR₄ₐ=CR_{4b}, or OCONR_{4b};
R₄ₐ, R_{4b}, R_{4c}, and R_{4d} are independently hydrogen and alkyl, wherein the alkyl may be optionally substituted with R₁₀, R₁₀ₐ, R_{10b}, and R_{10c};
G is a 5- or 6-membered heteroaryl containing at least one nitrogen;
R₆, R₇, and R₈ are independently hydrogen, halo, haloalkyl, haloalkoxy, alkyl, aryl, heterocyclyl, alkoxy, aryloxy;
Q is CONR₁₁R₁₁ₐ, or OCONR₁₁R₁₁ₐ;
R₁₁ and R₁₁ₐ are independently hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c};
or R₁₁ and R₁₁ₐ may be taken together with the nitrogen to which they are attached to form a heterocyclyl ring, which may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, alkenyl, alkynyl, cycloalkyl, -C(O)NR₉R₉ₐ, -C(O)R₉, -NR₉C(O)R₉ₐ, aryl, aryloxy, or heterocyclyl, wherein the haloalkyl, alkyl, alkenyl, alkynyl, cycloalkyl, aryl, aryloxy, or heterocyclyl may be optionally substituted with R₉ and R₉ₐ; and
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl.

In still yet another embodiment, compounds of formula I are those in which:
Z is aryl or heterocyclyl group, and may be optionally substituted with R₁, R₂, R₃, R₄, and R₅ at any available positions;
R₁, R₂, R₃, R₄, and R₅ are independently hydrogen, halo, cyano, haloalkyl, haloalkoxy, nitro, alkyl, alkenyl, alkynyl, cycloalkyl, alkoxy, alkylthio, alkylsulfonyl, arylsulfonyl, alkylamino, -C(O)R₉, -NR₉C(O)R₉ₐ, -NR₉R₉ₐ, aryl, arylalkyl, aryloxy, or heterocyclyl, wherein the haloalkyl, haloalkoxy, alkyl, alkenyl, alkynyl, cycloalkyl, alkoxy, alkylthio, alkylsulfonyl, arylsulfonyl, alkylamino, aryl, arylalkyl, or heterocyclyl, may be optionally substituted with R₉ and R₉ₐ;
   or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R_{10c};
L is a bond, OCR₄ₐR_{4b}, CR₄ₐR_{4b}O, SCR₄ₐR_{4b}, CR₄ₐR_{4b}S, SO₂CR₄ₐR_{4b}, CR₄ₐR_{4b}SO₂, CR₄ₐR_{4b}CR_{4c}R_{4dc}, or CR₄ₐ=CR_{4b};
R₄ₐ, R_{4b}, R_{4c}, and R_{4d} are independently hydrogen, alkyl or haloalkyl, wherein the alkyl or haloalkyl may be optionally substituted with R₁₀, R₁₀ₐ, R_{10b}, and R_{10c};
G is a 5- or 6-membered heteroaryl containing at least one nitrogen;
R₆, R₇, and R₈ are independently hydrogen, halo, haloalkyl, haloalkoxy, alkyl, aryl, heterocyclyl, alkoxy, aryloxy;
Q is OCONR₁₁R₁₁ₐ;
R₁₁ and R₁₁ₐ are independently hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c};
   or R₁₁ and R₁₁ₐ may be taken together with the nitrogen to which they are attached to form a heterocyclyl ring, which may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, alkenyl, alkynyl, cycloalkyl, -C(O)NR₉R₉ₐ, -C(O)R₉, -NR₉C(O)R₉ₐ, aryl, aryloxy, or heterocyclyl, wherein the haloalkyl, alkyl, alkenyl, alkynyl, cycloalkyl, aryl, aryloxy, or heterocyclyl may be optionally substituted with R₉ and R₉ₐ; and
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl.

In one embodiment, compounds of formula I are those in which:
Z is aryl, and may be optionally substituted with R₁, R₂, R₃, R₄, and R₅ at any available positions;
R₁, R₂, R₃, R₄, and R₅ are independently hydrogen, halo, cyano, haloalkyl, haloalkoxy, nitro, alkyl, alkenyl, alkynyl, cycloalkyl, alkoxy, alkylthio, alkylsulfonyl, arylsulfonyl, alkylamino, -C(O)R₉, -NR₉C(O)R₉ₐ, -NR₉R₉ₐ, aryl, arylalkyl, aryloxy, or heterocyclyl, wherein the haloalkyl, haloalkoxy, alkyl, alkenyl, alkynyl, cycloalkyl, alkoxy, alkylthio, alkylsulfonyl, arylsulfonyl, alkylamino, aryl, arylalkyl, or heterocyclyl, may be optionally substituted with R₉ and R₉ₐ;
L is a bond, OCR₄ₐR_{4b}, SCR₄ₐR_{4b}, SO₂CR₄ₐR_{4b}, or CR₄ₐR_{4b}CR_{4c}R_{4d};
R₄ₐ, R_{4b}, R_{4c}, and R_{4d} are independently hydrogen and alkyl, wherein the alkyl may be optionally substituted with R₁₀, R₁₀ₐ, R_{10b}, and R_{10c};
G is a 5- or 6-membered heteroaryl containing at least one nitrogen;
R₆, R₇, and R₈ are independently hydrogen, halo, haloalkyl, haloalkoxy, alkyl, aryl, heterocyclyl, alkoxy, aryloxy;
Q is OCONR₁₁R₁₁ₐ;
R₁₁ and R₁₁ₐ are independently hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c};
or R₁₁ and R₁₁ₐ may be taken together with the nitrogen to which they are attached to form a heterocyclyl ring, which may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, alkenyl, alkynyl, cycloalkyl, -C(O)NR₉R₉ₐ, -C(O)R₉, -NR₉C(O)R₉ₐ, aryl, aryloxy, or heterocyclyl, wherein the haloalkyl, alkyl, alkenyl, alkynyl, cycloalkyl, aryl, aryloxy, or heterocyclyl may be optionally substituted with R₉ and R₉ₐ; and
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl.

In another embodiment, compounds of formula I are those in which:
Z is an aryl or heteroaryl of the following structure:

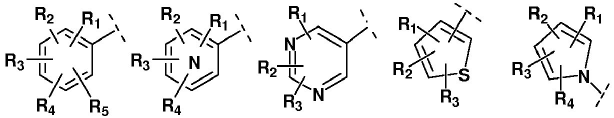

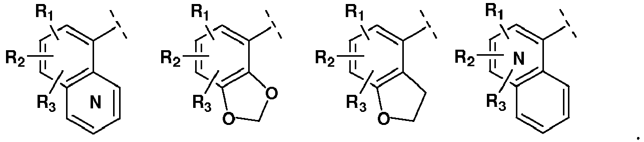

In yet another embodiment, compounds of formula I are those in which:
Z is an aryl or heteroaryl of the following structure:

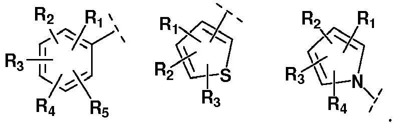

In still yet another embodiment, the compounds of formula I are those in which:
G is a 5- or 6-membered heteroaryl containing at least one nitrogen of the following structure:

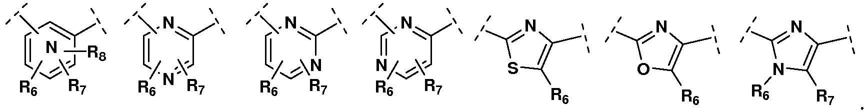

In one embodiment, compounds of formula I are those in which:
G is a 5- or 6-membered heteroaryl containing at least one nitrogen of the following structure:

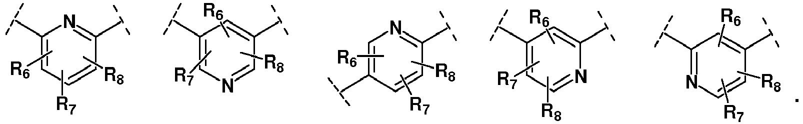

In another embodiment, compounds of formula I are those in which:
Z is an aryl or heteroaryl of the following structure: 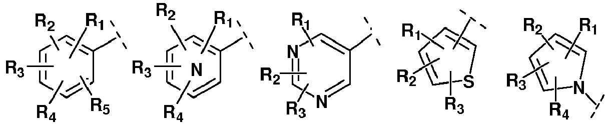 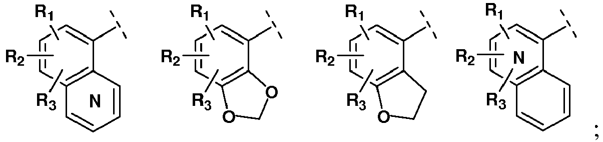
L is a bond, OCR₄ₐR_{4b}, CR₄ₐR_{4b}O, SCR₄ₐR_{4b}, CR₄ₐR_{4b}S, SO₂CR₄ₐR_{4b}, CR₄ₐR_{4b}SO₂, CR₄ₐR_{4b}CR_{4c}R_{4d}, or CR₄ₐ=CR_{4b}; and
G is a 5- or 6-membered heteroaryl containing at least one nitrogen of the following structure:

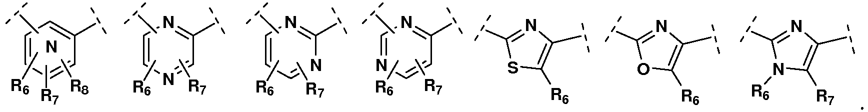

In another embodiment, compounds of formula I are those in which:
Z is an aryl or heteroaryl of the following structure: 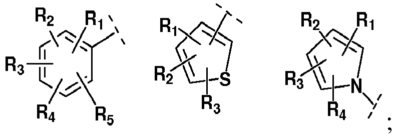
L is a bond, OCR₄ₐR_{4b}, SCR₄ₐR_{4b}, or SO₂CR₄ₐR_{4b};
G is a 5- or 6-membered heteroaryl containing at least one nitrogen of the following structure:

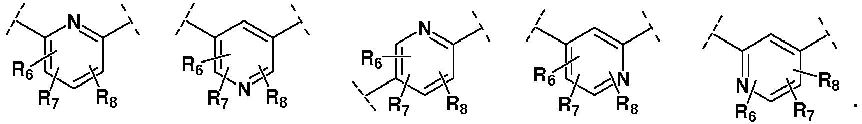

In another embodiment, compounds of formula I are those in which:
Z is 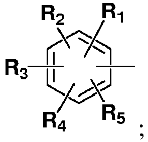 and
G is a 5- or 6-membered heteroaryl containing at least one nitrogen of the following structure:

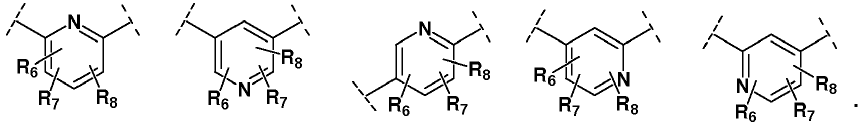

In another embodiment, compounds of formula I are those in which:
Z is 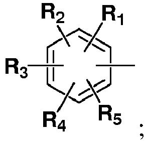
R₁, R₂, R₃, R₄, and R₅ are independently hydrogen, halo, cyano, haloalkyl, haloalkoxy, nitro, alkyl, alkenyl, alkynyl, cycloalkyl, alkoxy, alkylthio, alkylsulfonyl, arylsulfonyl, alkylamino, -C(O)R₉, -NR₉C(O)R₉ₐ, -NR₉R₉ₐ, aryl, arylalkyl, aryloxy, or heterocyclyl, wherein the haloalkyl, haloalkoxy, alkyl, alkenyl, alkynyl, cycloalkyl, alkoxy, alkylthio, alkylsulfonyl, arylsulfonyl, alkylamino, aryl, arylalkyl, or heterocyclyl, may be optionally substituted with R₉ and R₉ₐ;
   or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R_{10c};
L is a bond, OCR₄ₐR_{4b}, SCR₄ₐR_{4b}, or SO₂CR₄ₐR_{4b};
R₄ₐ and R_{4b} are independently hydrogen, alkyl, or haloalkyl;
G is a 5- or 6-membered heteroaryl containing at least one nitrogen of the following structure: 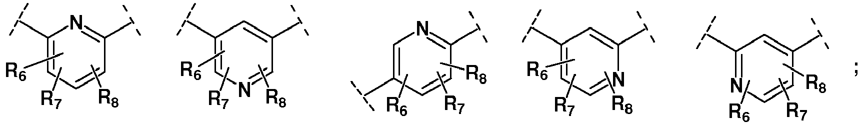
R₆, R₇, and R₈ are independently hydrogen, halo, haloalkyl, haloalkoxy, alkyl, aryl, heterocyclyl, alkoxy, aryloxy;
Q is OCONR₁₁R₁₁ₐ;
R₁₁ and R₁₁ₐ are independently hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c}; or R₁₁ and R₁₁ₐ may be taken together with the nitrogen to which they are attached to form a heterocyclyl ring, which may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, alkenyl, alkynyl, cycloalkyl, -C(O)NR₉R₉ₐ, -C(O)R₉, -NR₉C(O)R₉ₐ, aryl, aryloxy, or heterocyclyl, wherein the haloalkyl, alkyl, alkenyl, alkynyl, cycloalkyl, aryl, aryloxy, or heterocyclyl may be optionally substituted with R₉ and R₉ₐ; and
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl.

In yet another embodiment, compounds of formula I are those in which:
R₁, R₂, R₃, R₄, and R₅ are independently hydrogen, halo, cyano, haloalkyl, haloalkoxy, nitro, alkyl, cycloalkyl, alkoxy, alkylthio, alkylsulfonyl, arylsulfonyl, alkylamino, -C(O)R₉, -NR₉C(O)R₉ₐ, -NR₉R₉ₐ, aryl, arylalkyl, aryloxy, or heterocyclyl, wherein the haloalkyl, haloalkoxy, alkyl, alkenyl, alkynyl, cycloalkyl, alkoxy, alkylthio, alkylsulfonyl, arylsulfonyl, alkylamino, aryl, arylalkyl, or heterocyclyl, may be optionally substituted with R₉ and R₉ₐ;
   or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R_{10c};
L is OCR₄ₐR_{4b}, SCR₄ₐR_{4b}, or SO₂CR₄ₐR_{4b};
R₄ₐ and R_{4b} are independently hydrogen, alkyl or haloalkyl;
G is a 5- or 6-membered heteroaryl containing at least one nitrogen of the following structure: 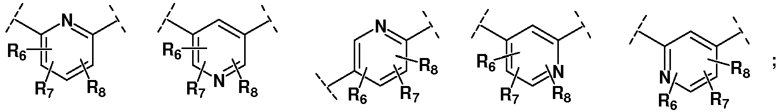
R₆, R₇, and R₈ are independently hydrogen, halo, haloalkyl, haloalkoxy, alkyl, aryl, heterocyclyl, alkoxy, aryloxy;
Q is OCONR₁₁R₁₁ₐ;
R₁₁ and R₁₁ₐ are independently hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c};
or R₁₁ and R₁₁ₐ may be taken together with the nitrogen to which they are attached to form a heterocyclyl ring, which may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, alkenyl, alkynyl, cycloalkyl, -C(O)NR₉R₉ₐ, - C(O)R₉, -NR₉C(O)R₉ₐ, aryl, aryloxy, or heterocyclyl, wherein the haloalkyl, alkyl, alkenyl, alkynyl, cycloalkyl, aryl, aryloxy, or heterocyclyl may be optionally substituted with R₉ and R₉ₐ; and
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl.

In still yet another embodiment, compounds of formula I are those in which:
R₁, R₂, R₃, R₄, and R₅ are independently hydrogen, halo, cyano, haloalkyl,
   haloalkoxy, nitro, alkyl, cycloalkyl, alkoxy, alkylthio, alkylsulfonyl, arylsulfonyl, alkylamino, -C(O)R₉, -NR₉C(O)R₉ₐ, -NR₉R₉ₐ, aryl, arylalkyl, aryloxy, or heterocyclyl, wherein the haloalkyl, haloalkoxy, alkyl, alkenyl, alkynyl, cycloalkyl, alkoxy, alkylthio, alkylsulfonyl, arylsulfonyl, alkylamino, aryl, arylalkyl, or heterocyclyl, may be optionally substituted with R₉ and R₉ₐ;
   or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R_{10c};
L is OCR₄ₐR_{4b} or SO₂CR₄ₐR_{4b};
R₄ₐ and R_{4b} are independently hydrogen, alkyl, or haloalkyl;
G is a 5- or 6-membered heteroaryl containing at least one nitrogen of the following structure: 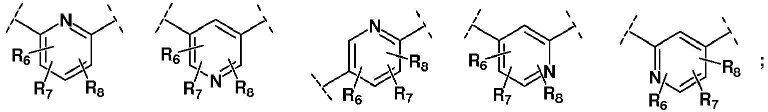
R₆, R₇, and R₈ are independently hydrogen, halo, haloalkyl, haloalkoxy, alkyl, aryl, heterocyclyl, alkoxy, aryloxy;
Q is OCONR₁₁R₁₁ₐ;
R₁₁ and R₁₁ₐ are independently hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c}; or R₁₁ and R₁₁ₐ may be taken together with the nitrogen to which they are attached to form a heterocyclyl ring, which may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, alkenyl, alkynyl, cycloalkyl, -C(O)NR₉R₉ₐ, -C(O)R₉, -NR₉C(O)R₉ₐ, aryl, aryloxy, or heterocyclyl, wherein the haloalkyl, alkyl, alkenyl, alkynyl, cycloalkyl, aryl, aryloxy, or heterocyclyl may be optionally substituted with R₉ and R₉ₐ; and
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl.

In one embodiment, compounds of formula I are those in which:
R₁, R₂, R₃, R₄, and R₅ are independently hydrogen, halo, cyano, haloalkyl, haloalkoxy, nitro, alkyl, alkenyl, alkynyl, cycloalkyl, alkoxy, alkylthio, alkylsulfonyl, arylsulfonyl, alkylamino, -C(O)R₉, -NR₉C(O)R₉ₐ, -NR₉R₉ₐ, aryl, arylalkyl, aryloxy, or heterocyclyl, wherein the haloalkyl, haloalkoxy, alkyl, alkenyl, alkynyl, cycloalkyl, alkoxy, alkylthio, alkylsulfonyl, arylsulfonyl, alkylamino, aryl, arylalkyl, or heterocyclyl, may be optionally substituted with R₉ and R₉ₐ;
   or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R_{10c};
L is OCR₄ₐR_{4b} or SO₂CR₄ₐR_{4b};
R₄ₐ and R_{4b} are independently hydrogen or alkyl;
G is a 5- or 6-membered heteroaryl containing at least one nitrogen of the following structure: 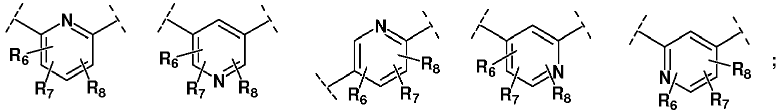
R₆, R₇, and R₈ are independently hydrogen, halo, haloalkyl, haloalkoxy, alkyl, aryl, heterocyclyl, alkoxy, aryloxy;
Q is OCONR₁₁R₁₁ₐ;
R₁₁ and R₁₁ₐ are independently hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c}; or R₁₁ and R₁₁ₐ may be taken together with the nitrogen to which they are attached to form a heterocyclyl ring, which may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, alkenyl, alkynyl, cycloalkyl, -C(O)NR₉R₉ₐ, -C(O)R₉, -NR₉C(O)R₉ₐ, aryl, aryloxy, or heterocyclyl, wherein the haloalkyl, alkyl, alkenyl, alkynyl, cycloalkyl, aryl, aryloxy, or heterocyclyl may be optionally substituted with R₉ and R₉ₐ; and
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl.

In another embodiment, compounds of formula I are those in which:
R₁, R₂, R₃, R₄, and R₅ are independently hydrogen, halo, cyano, haloalkyl,
   haloalkoxy, nitro, alkyl, cycloalkyl, alkoxy, alkylthio, alkylsulfonyl, arylsulfonyl, alkylamino, -C(O)R₉, -NR₉C(O)R₉ₐ, -NR₉R₉ₐ, aryl, arylalkyl, aryloxy, or heterocyclyl, wherein the haloalkyl, haloalkoxy, alkyl, alkenyl, alkynyl, cycloalkyl, alkoxy, alkylthio, alkylsulfonyl, arylsulfonyl, alkylamino, aryl, arylalkyl, aryloxy, or heterocyclyl, may be optionally substituted with R₉ and R₉ₐ;
   or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R_{10c};
L is OCR₄ₐR_{4b} or SO₂CR₄ₐR_{4b};
R₄ₐ and R_{4b} are independently hydrogen or alkyl;
G is a 5- or 6-membered heteroaryl containing at least one nitrogen of the following structure: 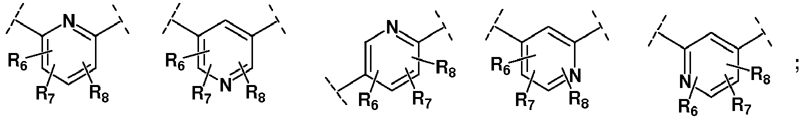
R₆, R₇, and R₈ are independently hydrogen, halo, haloalkyl, haloalkoxy, alkyl, aryl, heterocyclyl, alkoxy, aryloxy;
Q is OCONR₁₁R₁₁ₐ;
R₁₁ and R₁₁ₐ are independently hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c}; or R₁₁ and R₁₁ₐ may be taken together with the nitrogen to which they are attached to form a heterocyclyl ring, which may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, cycloalkyl, -C(O)NR₉R₉ₐ, -C(O)R₉, -NR₉C(O)R₉ₐ, aryl, aryloxy, or heterocyclyl, wherein the haloalkyl, alkyl, cycloalkyl, aryl, aryloxy, or heterocyclyl may be optionally substituted with R₉ and R₉ₐ; and
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl.

In yet another embodiment, compounds of formula I are those in which:
R₁, R₂, R₃, R₄, and R₅ are independently hydrogen, halo, cyano, haloalkyl,
   haloalkoxy, nitro, alkyl, cycloalkyl, alkoxy, alkylthio, alkylsulfonyl, arylsulfonyl, alkylamino, aryl, arylalkyl, aryloxy, or heterocyclyl, wherein the haloalkyl, haloalkoxy, alkyl, cycloalkyl, alkoxy, alkylthio, alkylsulfonyl, arylsulfonyl, alkylamino, aryl, arylalkyl, or heterocyclyl, may be optionally substituted with R₉ and R₉ₐ;
   or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R_{10c};
L is OCR₄ₐR_{4b} or SO₂CR₄ₐR_{4b};
R₄ₐ and R_{4b} are independently hydrogen or alkyl;
G is a 5- or 6-membered heteroaryl containing at least one nitrogen of the following structure: 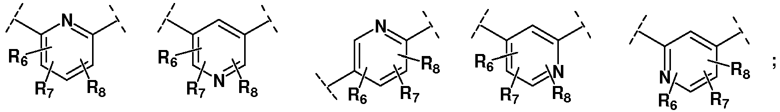
R₆, R₇, and R₈ are independently hydrogen, halo, haloalkyl, haloalkoxy, alkyl, aryl, or heterocyclyl;
Q is OCONR₁₁R₁₁ₐ;
R₁₁ and R₁₁ₐ are independently hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c};
   or R₁₁ and R₁₁ₐ may be taken together with the nitrogen to which they are attached to form a heterocyclyl ring, which may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, cycloalkyl, aryl, aryloxy, or heterocyclyl, wherein the haloalkyl, alkyl, cycloalkyl, aryl, aryloxy, or heterocyclyl may be optionally substituted with R₉ and R₉ₐ; and
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl.

In still yet another embodiment, compounds of formula I are those in which:
R₁, R₂, R₃, R₄, and R₅ are independently hydrogen, halo, haloalkyl, haloalkoxy, alkyl, cycloalkyl, alkoxy, alkylthio, alkylsulfonyl, arylsulfonyl, alkylamino, aryl, arylalkyl, aryloxy, or heterocyclyl, wherein the haloalkyl, haloalkoxy, alkyl, cycloalkyl, alkoxy, alkylthio, alkylsulfonyl, arylsulfonyl, alkylamino, aryl, arylalkyl, or heterocyclyl, may be optionally substituted with R₉ and R₉ₐ;
   or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R_{10c};
L is OCR₄ₐR_{4b} or SO₂CR₄ₐR_{4b};
R₄ₐ and R_{4b} are independently hydrogen or alkyl;
G is a 5- or 6-membered heteroaryl containing at least one nitrogen of the following structure: 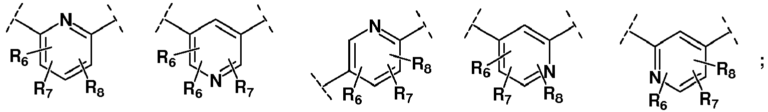
R₆, R₇, and R₈ are independently hydrogen, halo, alkyl, aryl, or heterocyclyl;
Q is OCONR₁₁R₁₁ₐ;
R₁₁ and R₁₁ₐ are independently hydrogen, alkyl, cycloalkyl, aryl or heterocyclyl, wherein the alkyl, cycloalkyl, aryl, or heterocyclyl may be optionally substituted with R₁₀, R₁₀ₐ, R_{10b}, and R_{10c};
or R₁₁ and R₁₁ₐ may be taken together with the nitrogen to which they are attached to form a heterocyclyl ring, which may be optionally substituted with R₁₀, R₁₀ₐ, R_{10b}, and R_{10c};
R₁₀, R₁₀ₐ, R_{10b}, and R_{10c} are independently selected from hydrogen, halo, haloalkyl, alkyl, cycloalkyl, aryl, aryloxy, or heterocyclyl, wherein the haloalkyl, alkyl, cycloalkyl, aryl, aryloxy, or heterocyclyl may be optionally substituted with R₉ and R₉ₐ; and
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl.

In an additional embodiment, compounds of formula I are those in which:
R₁, R₂, R₃, R₄, and R₅ are independently hydrogen, halo, haloalkyl, haloalkoxy, alkyl, cycloalkyl, alkoxy, aryl, arylalkyl, aryloxy, or heterocyclyl, wherein the haloalkyl, haloalkoxy, alkyl, cycloalkyl, alkoxy, aryl, arylalkyl, aryloxy, or heterocyclyl, may be optionally substituted with R₉ and R₉ₐ;
L is OCR₄ₐR_{4b} or SO₂CR₄ₐR_{4b};
R₄ₐ and R_{4b} are independently hydrogen or alkyl;
G is a 5- or 6-membered heteroaryl containing at least one nitrogen of the following structure: 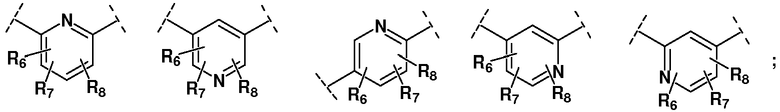
R₆, R₇, and R₈ are independently hydrogen, alkyl, aryl, or heterocyclyl;
Q is OCONR₁₁R₁₁ₐ;
R₁₁ and R₁₁ₐ are independently hydrogen, alkyl, cycloalkyl, aryl or heterocyclyl, wherein the alkyl, cycloalkyl, aryl or heterocyclyl may be optionally substituted with R₁₀, R₁₀ₐ, R_{10b}, and R_{10c};
   or R₁₁ and R₁₁ₐ may be taken together with the nitrogen to which they are attached to form a heterocyclyl ring, which may be optionally substituted with R₁₀, R₁₀ₐ, R_{10b}, and R_{10c};
R₁₀, R₁₀ₐ, R_{10b}, and R_{10c} are independently selected from hydrogen, halo, haloalkyl, alkyl, cycloalkyl, aryl, or heterocyclyl, wherein the haloalkyl, alkyl, cycloalkyl, aryl, or heterocyclyl may be optionally substituted with R₉ and R₉ₐ; and
R₉ and R₉ₐ are independently hydrogen, alkyl, cycloalkyl, aryl, or heterocyclyl, wherein the alkyl, cycloalkyl, aryl, or heterocyclyl may be optionally substituted with halo, haloalkyl, alkyl, aryl, or heterocyclyl.

In another additional embodiment, compounds of formula I are those in which:
R₁, R₂, R₃, R₄, and R₅ are independently hydrogen, halo, haloalkyl, alkyl, cycloalkyl, aryl, arylalkyl, aryloxy, or heterocyclyl, wherein the haloalkyl, haloalkoxy, alkyl, cycloalkyl, alkoxy, aryl, arylalkyl, aryloxy, or heterocyclyl, may be optionally substituted with R₉ and R₉ₐ;
L is OCH₂ or SO₂CH₂;
G is a 5- or 6-membered heteroaryl containing at least one nitrogen of the following structure: 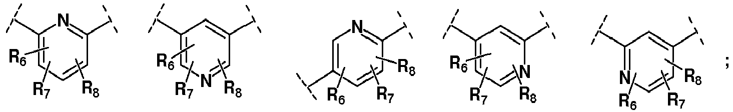
R₆, R₇, and R₈ are independently hydrogen or alkyl;
Q is OCONR₁₁R₁₁ₐ;
R₁₁ and R₁₁ₐ are independently hydrogen, alkyl, cycloalkyl, aryl or heterocyclyl, wherein the alkyl, cycloalkyl, aryl or heterocyclyl may be optionally substituted with R₁₀, R₁₀ₐ, R_{10b}, and R_{10c};
or R₁₁ and R₁₁ₐ may be taken together with the nitrogen to which they are attached to form a heterocyclyl ring, which may be optionally substituted with R₁₀, R₁₀ₐ, R_{10b}, and R_{10c};
R₁₀, R₁₀ₐ, R_{10b}, and R_{10c} are independently selected from hydrogen, halo, alkyl, cycloalkyl, aryl, or heterocyclyl, wherein the alkyl, cycloalkyl, aryl, or heterocyclyl may be optionally substituted with R₉ and R₉ₐ; and
R₉ and R₉ₐ are independently hydrogen, alkyl, cycloalkyl, aryl, or heterocyclyl, wherein the alkyl, cycloalkyl, aryl, or heterocyclyl may be optionally substituted with halo, haloalkyl, alkyl, aryl, or heterocyclyl.

In one embodiment, compounds of formula I are those in which:
G is a 5- or 6-membered heteroaryl containing at least one nitrogen of the following structure:

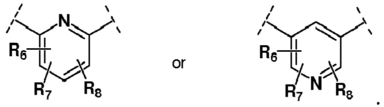

In another embodiment, compounds of formula I are those in which:
G is a 5- or 6-membered heteroaryl containing at least one nitrogen of the following structure:

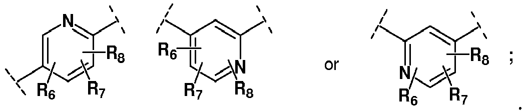

In another embodiment, compounds of the present invention are selected from the compounds exemplified in the examples.

In another embodiment, the present invention relates to pharmaceutical compositions comprised of a therapeutically effective amount of a compound of the present invention, alone or, optionally, in combination with a pharmaceutically acceptable carrier and/or one or more other agent(s).

In another embodiment, the present invention relates to methods of inhibiting the activity of the enzyme 11-beta-hydroxysteroid dehydrogenase type I comprising administering to a mammalian patient, for example, a human patient, in need thereof a therapeutically effective amount of a compound of the present invention, alone, or optionally, in combination with another compound of the present invention and/or at least one other type of therapeutic agent.

In another embodiment, the present invention relates to a method for preventing, inhibiting, or treating the progression or onset of diseases or disorders associated with the activity of the enzyme 11-beta-hydroxysteroid dehydrogenase type I comprising administering to a mammalian patient, for example, a human patient, in need of prevention, inhibition, or treatment a therapeutically effective amount of a compound of the present invention, alone, or, optionally, in combination with another compound of the present invention and/or at least one other type of therapeutic agent.

Examples of diseases or disorders associated with the activity of the enzyme 11-beta-hydroxysteroid dehydrogenase type I that can be prevented, inhibited, or treated according to the present invention include, but are not limited to, diabetes, hyperglycemia, impaired glucose tolerance, insulin resistance, hyperinsulinemia, retinopathy, neuropathy, nephropathy, delayed wound healing, atherosclerosis and its sequelae, abnormal heart function, myocardial ischemia, stroke, Metabolic Syndrome, hypertension, obesity, dislipidemia, dylsipidemia, hyperlipidemia, hypertriglyceridemia, hypercholesterolemia, low HDL, high LDL, non-cardiac ischemia, infection, cancer, vascular restenosis, pancreatitis, neurodegenerative disease, lipid disorders, cognitive impairment and dementia, bone disease, HIV protease associated lipodystrophy and glaucoma.

In another embodiment, the present invention relates to a method for preventing, inhibiting, or treating the progression or onset of diabetes, hyperglycemia, obesity,dyslipidemia, hypertension and cognitive impairment comprising administering to a mammalian patient, for example, a human patient, in need of prevention, inhibition, or treatment a therapeutically effective amount of a compound of the present invention, alone, or, optionally, in combination with another compound of the present invention and/or at least one other type of therapeutic agent.

In still another embodiment, the present invention relates to a method for preventing, inhibiting, or treating the progression or onset of diabetes, comprising administering to a mammalian patient, for example, a human patient, in need of prevention, inhibition, or treatment a therapeutically effective amount of a compound of the present invention, alone, or, optionally, in combination with another compound of the present invention and/or at least one other type of therapeutic agent.

In yet still another embodiment, the present invention relates to a method for preventing, inhibiting, or treating the progression or onset of hyperglycemia comprising administering to a mammalian patient, for example, a human patient, in need of prevention, inhibition, or treatment a therapeutically effective amount of a compound of the present invention, alone, or, optionally, in combination with another compound of the present invention and/or at least one other type of therapeutic agent.

In another embodiment, the present invention relates to a method for preventing, inhibiting, or treating the progression or onset of obesity comprising administering to a mammalian patient, for example, a human patient, in need of prevention, inhibition, or treatment a therapeutically effective amount of a compound of the present invention, alone, or, optionally, in combination with another compound of the present invention and/or at least one other type of therapeutic agent.

In one embodiment, the present invention relates to a method for preventing, inhibiting, or treating the progression or onset of dyslipidemia comprising administering to a mammalian patient, for example, a human patient, in need of prevention, inhibition, or treatment a therapeutically effective amount of a compound of the present invention, alone, or, optionally, in combination with another compound of the present invention and/or at least one other type of therapeutic agent.

In another embodiment, the present invention relates to a method for preventing, inhibiting, or treating the progression or onset of hypertension comprising administering to a mammalian patient, for example, a human patient, in need of prevention, inhibition, or treatment a therapeutically effective amount of a compound of the present invention, alone, or, optionally, in combination with another compound of the present invention and/or at least one other type of therapeutic agent.

In another embodiment, the present invention relates to a method for preventing, inhibiting, or treating the progression or onset of cognitive impairment comprising administering to a mammalian patient, for example, a human patient, in need of prevention, inhibition, or treatment a therapeutically effective amount of a compound of the present invention, alone, or, optionally, in combination with another compound of the present invention and/or at least one other type of therapeutic agent.

### DEFINITIONS

The compounds herein described may have asymmetric centers. Compounds of the present invention containing an asymmetrically substituted atom may be isolated in optically active or racemic forms. It is well known in the art how to prepare optically active forms, such as by resolution of racemic forms or by synthesis from optically active starting materials. Many geometric isomers of olefins, C=N double bonds, and the like can also be present in the compounds described herein, and all such stable isomers are contemplated in the present invention. Cis and trans geometric isomers of the compounds of the present invention are described and may be isolated as a mixture of isomers or as separated isomeric forms. All chiral, diastereomeric, racemic forms, and all geometric isomeric forms of a structure are intended, unless the specific stereochemistry or isomeric form is specifically indicated.

The term "substituted," as used herein, means that any one or more hydrogens on the designated atom or ring is replaced with a selection from the indicated group, provided that the designated atom's normal valency is not exceeded, and that the substitution results in a stable compound. When a substituent is keto (i.e., =O), then 2 hydrogens on the atom are replaced.

When any variable (e.g., R^{a}) occurs more than one time in any constituent or formula for a compound, its definition at each occurrence is independent of its definition at every other occurrence. Thus, for example, if a group is shown to be substituted with 0-2 R^{a}, then said group may optionally be substituted with up to two R^{a} groups and R^{a} at each occurrence is selected independently from the definition of R^{a}. Also, combinations of substituents and/or variables are permissible only if such combinations result in stable compounds.

When a bond to a substituent is shown to cross a bond connecting two atoms in a ring, then such substituent may be bonded to any atom on the ring. When a substituent is listed without indicating the atom via which such substituent is bonded to the rest of the compound of a given formula, then such substituent may be bonded via any atom in such substituent. Combinations of substituents and/or variables are permissible only if such combinations result in stable compounds.

Unless otherwise indicated, the term "lower alkyl," "alkyl," or "alk" as employed herein alone or as part of another group includes both straight and branched chain hydrocarbons, containing 1 to 20 carbons, preferably 1 to 10 carbons, more preferably 1 to 8 carbons, in the normal chain, such as methyl, ethyl, propyl, isopropyl, butyl, t-butyl, isobutyl, pentyl, hexyl, isohexyl, heptyl, 4,4-dimethylpentyl, octyl, 2,2,4-trimethyl-pentyl, nonyl, decyl, undecyl, dodecyl, the various branched chain isomers thereof, and the like as well as such groups may optionally include 1 to 4 substituents such as halo, for example F, Br, Cl, or I, or CF₃, alkyl, alkoxy, aryl, aryloxy, aryl(aryl) or diaryl, arylalkyl, arylalkyloxy, alkenyl, cycloalkyl, cycloalkylalkyl, cycloalkylalkyloxy, amino, hydroxy, hydroxyalkyl, acyl, heteroaryl, heteroaryloxy, heteroarylalkyl, heteroarylalkoxy, aryloxyalkyl, alkylthio, arylalkylthio, aryloxyaryl, alkylamido, alkanoylamino, arylcarbonylamino, nitro, cyano, thiol, haloalkyl, trihaloalkyl, and/or alkylthio.

Unless otherwise indicated, the term "cycloalkyl" as employed herein alone or as part of another group includes saturated or partially unsaturated (containing 1 or 2 double bonds) cyclic hydrocarbon groups containing 1 to 3 rings, including monocyclic alkyl, bicyclic alkyl (or bicycloalkyl) and tricyclic alkyl, containing a total of 3 to 20 carbons forming the ring, preferably 3 to 10 carbons, forming the ring and which may be fused to 1 or 2 aromatic rings as described for aryl, which includes cyclopropyl, cyclobutyl, cyclopentyl, cyclohexyl, cycloheptyl, cyclooctyl, cyclodecyl and cyclododecyl, cyclohexenyl, 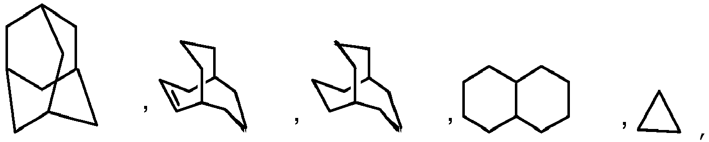 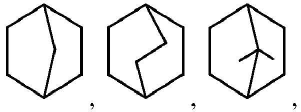 any of which groups may be optionally substituted with 1 to 4 substituents such as halogen, alkyl, alkoxy, hydroxy, aryl, aryloxy, arylalkyl, cycloalkyl, alkylamido, alkanoylamino, oxo, acyl, arylcarbonylamino, amino, nitro, cyano, thiol, and/or alkylthio, and/or any of the substituents for alkyl.

Unless otherwise indicated, the term "lower alkenyl" or "alkenyl" as used herein by itself or as part of another group refers to straight or branched chain radicals of 2 to 20 carbons, preferably 2 to 12 carbons, and more preferably 1 to 8 carbons in the normal chain, which include one to six double bonds in the normal chain, such as vinyl, 2-propenyl, 3-butenyl, 2-butenyl, 4-pentenyl, 3-pentenyl, 2-hexenyl, 3-hexenyl, 2-heptenyl, 3-heptenyl, 4-heptenyl, 3-octenyl, 3-nonenyl, 4-decenyl, 3-undecenyl, 4-dodecenyl, 4,8,12-tetradecatrienyl, and the like, and which may be optionally substituted with 1 to 4 substituents, namely, halogen, haloalkyl, alkyl, alkoxy, alkenyl, alkynyl, aryl, arylalkyl, cycloalkyl, amino, hydroxy, heteroaryl, cycloheteroalkyl, alkanoylamino, alkylamido, arylcarbonyl-amino, nitro, cyano, thiol, alkylthio, and/or any of the alkyl substituents set out herein.

Unless otherwise indicated, the term "lower alkynyl" or "alkynyl" as used herein by itself or as part of another group refers to straight or branched chain radicals of 2 to 20 carbons, preferably 2 to 12 carbons and more preferably 2 to 8 carbons in the normal chain, which include one triple bond in the normal chain, such as 2-propynyl, 3-butynyl, 2-butynyl, 4-pentynyl, 3-pentynyl, 2-hexynyl, 3-hexynyl, 2-heptynyl, 3-heptynyl, 4-heptynyl, 3-octynyl, 3-nonynyl, 4-decynyl,3-undecynyl, 4-dodecynyl, and the like, and which may be optionally substituted with 1 to 4 substituents, namely, halogen, haloalkyl, alkyl, alkoxy, alkenyl, alkynyl, aryl, arylalkyl, cycloalkyl, amino, heteroaryl, cycloheteroalkyl, hydroxy, alkanoylamino, alkylamido, arylcarbonylamino, nitro, cyano, thiol, and/or alkylthio, and/or any of the alkyl substituents set out herein.

Where alkyl groups as defined above have single bonds for attachment to other groups at two different carbon atoms, they are termed "alkylene" groups and may optionally be substituted as defined above for "alkyl".

Where alkenyl groups as defined above and alkynyl groups as defined above, respectively, have single bonds for attachment at two different carbon atoms, they are termed "alkenylene groups" and "alkynylene groups", respectively, and may optionally be substituted as defined above for "alkenyl" and "alkynyl".

The term "halogen" or "halo" as used herein alone or as part of another group refers to chlorine, bromine, fluorine, and iodine as well as CF₃, with chlorine or fluorine being preferred.

Unless otherwise indicated, the term "aryl" as employed herein alone or as part of another group refers to monocyclic and bicyclic aromatic groups containing 6 to 10 carbons in the ring portion (such as phenyl or naphthyl, including 1-naphthyl and 2-naphthyl) and may optionally include 1 to 3 additional rings fused to a carbocyclic ring or a heterocyclic ring (such as aryl, cycloalkyl, heteroaryl, or cycloheteroalkyl rings
for example 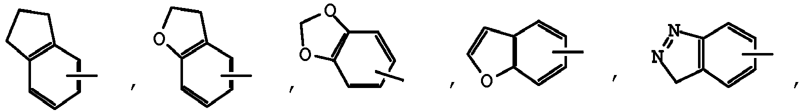 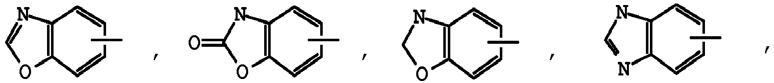 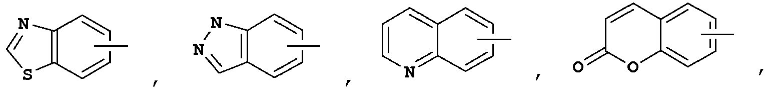 and may be optionally substituted through available carbon atoms with 1, 2, or 3 substituents, for example, hydrogen, halo, haloalkyl, alkyl, haloalkyl, alkoxy, haloalkoxy, alkenyl, trifluoromethyl, trifluoromethoxy, alkynyl, cycloalkyl-alkyl, cycloheteroalkyl, cycloheteroalkylalkyl, aryl, heteroaryl, arylalkyl, aryloxy, aryloxyalkyl, arylalkoxy, arylthio, arylazo, heteroarylalkyl, heteroarylalkenyl, heteroarylheteroaryl, heteroaryloxy, hydroxy, nitro, cyano, amino, substituted amino wherein the amino includes 1 or 2 substituents (which are alkyl, aryl, or any of the other aryl compounds mentioned in the definitions), thiol, alkylthio, arylthio, heteroarylthio, arylthioalkyl, alkoxyarylthio, alkylcarbonyl, arylcarbonyl, alkylaminocarbonyl, arylaminocarbonyl, alkoxycarbonyl, aminocarbonyl, alkylcarbonyloxy, arylcarbonyloxy, alkylcarbonylamino, arylcarbonylamino, arylsulfinyl, arylsulfinylalkyl, arylsulfonylamino, or arylsulfon-aminocarbonyl, and/or any of the alkyl substituents set out herein.

Unless otherwise indicated, the term "lower alkoxy", "alkoxy", "aryloxy" or "aralkoxy" as employed herein alone or as part of another group includes any of the above alkyl, aralkyl, or aryl groups linked to an oxygen atom.

Unless otherwise indicated, the term "amino" as employed herein alone or as part of another group refers to amino that may be substituted with one or two substituents, which may be the same or different, such as alkyl, aryl, arylalkyl, heteroaryl, heteroarylalkyl, cycloheteroalkyl, cycloheteroalkylalkyl, cycloalkyl, cycloalkylalkyl, haloalkyl, hydroxyalkyl, alkoxyalkyl, or thioalkyl. These substituents may be further substituted with a carboxylic acid and/or any of the R¹ groups or substituents for R¹ as set out above. In addition, the amino substituents may be taken together with the nitrogen atom to which they are attached to form 1-pyrrolidinyl, 1-piperidinyl, 1-azepinyl, 4-morpholinyl, 4-thiamorpholinyl, 1-piperazinyl, 4-alkyl-1-piperazinyl, 4-arylalkyl-1-piperazinyl, 4-diarylalkyl-1-piperazinyl, 1-pyrrolidinyl, 1-piperidinyl, or 1-azepinyl, optionally substituted with alkyl, alkoxy, alkylthio, halo, trifluoromethyl, or hydroxy.

Unless otherwise indicated, the term "lower alkylthio," "alkylthio," "arylthio," or "aralkylthio" as employed herein alone or as part of another group includes any of the above alkyl, aralkyl, or aryl groups linked to a sulfur atom.

Unless otherwise indicated, the term "lower alkylamino," "alkylamino," "arylamino," or "arylalkylamino" as employed herein alone or as part of another group includes any of the above alkyl, aryl, or arylalkyl groups linked to a nitrogen atom.

As used herein, the term " heterocyclyl " or "heterocyclic system" is intended to mean a stable 5- to 12-membered monocyclic or bicyclic heterocyclic ring which is saturated, partially unsaturated, or unsaturated (aromatic), and which consists of carbon atoms and 1, 2, 3, or 4 heteroatoms independently selected from the group consisting of N, NH, O, and S, and including any bicyclic group in which any of the above-defined heterocyclic rings is fused to a benzene ring. The nitrogen and sulfur heteroatoms may optionally be oxidized. The heterocyclic ring may be attached to its pendant group at any heteroatom or carbon atom which results in a stable structure. The heterocyclic rings described herein may be substituted on carbon or on a nitrogen atom if the resulting compound is stable. If specifically noted, a nitrogen in the heterocycle may optionally be quatemized. It is preferred that when the total number of S and O atoms in the heterocycle exceeds 1, then these heteroatoms are not adjacent to one another. As used herein, the term "aromatic heterocyclic system" is intended to mean a stable 5- to 12-membered monocyclic or bicyclic heterocyclic aromatic ring, which consists of carbon atoms and from 1 to 4 heterotams independently selected from the group consisting of N, O, and S.

Unless otherwise indicated, the term "heteroaryl" as used herein alone or as part of another group refers to a 5- or 12- membered aromatic ring, prefereably, a 5-or 6- membered aromatic ring, which includes 1, 2, 3, or 4 hetero atoms such as nitrogen, oxygen, or sulfur, and such rings fused to an aryl, cycloalkyl, heteroaryl, or cycloheteroalkyl ring (e.g. benzothiophenyl, indolyl), and includes possible N-oxides. The heteroaryl group may optionally include 1 to 4 substituents such as any of the substituents set out above for alkyl. Examples of heteroaryl groups include the following: 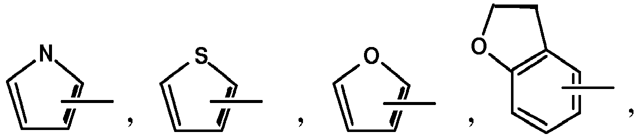 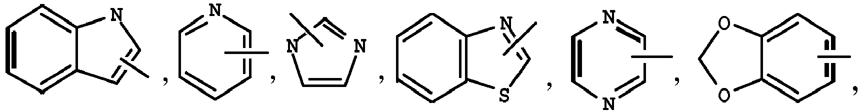 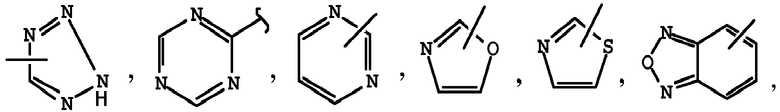 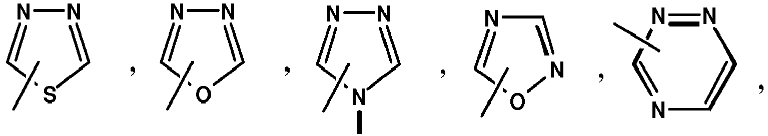 and the like.

The term " heterocyclylalkyl" or "heterocyclyl" as used herein alone or as part of another group refers to heterocyclyl groups as defined above linked through a C atom or heteroatom to an alkyl chain.

The term "heteroarylalkyl" or "heteroarylalkenyl" as used herein alone or as part of another group refers to a heteroaryl group as defined above linked through a C atom or heteroatom to an alkyl chain, alkylene, or alkenylene as defined above.

The term "cyano" as used herein, refers to a -CN group.

The term "nitro" as used herein, refers to an ―NO₂ group.

The term "hydroxy" as used herein, refers to an -OH group.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418, the disclosure of which is hereby incorporated by reference.

Any compound that can be converted in vivo to provide the bioactive agent (i.e., the compound of formula I) is a prodrug within the scope and spirit of the invention.

The term "prodrugs" as employed herein includes esters and carbonates formed by reacting one or more hydroxyls of compounds of formula I with alkyl, alkoxy, or aryl substituted acylating agents employing procedures known to those skilled in the art to generate acetates, pivalates, methylcarbonates, benzoates, and the like.

Various forms of prodrugs are well known in the art and are described in:
a) The Practice of Medicinal Chemistry, Camille G. Wermuth et al., Ch. 31, (Academic Press, 1996);
b) Design of Prodrugs, edited by H. Bundgaard, (Elsevier, 1985);
c) A Textbook of Drug Design and Development, P. Krogsgaard-Larson and H. Bundgaard, eds. Ch. 5, pgs 113 - 191 (Harwood Academic Publishers, 1991); and
d) Hydrolysis in Drug and Prodrug Metabolism, Bernard Testa and Joachim M. Mayer, (Wiley-VCH, 2003).
Said references are incorporated herein by reference.

In addition, compounds of the formula I are, subsequent to their preparation, preferably isolated and purified to obtain a composition containing an amount by weight equal to or greater than 99% formula I compound ("substantially pure" compound I), which is then used or formulated as described herein. Such "substantially pure" compounds of the formula I are also contemplated herein as part of the present invention.

All stereoisomers of the compounds of the instant invention are contemplated, either in admixture or in pure or substantially pure form. The compounds of the present invention can have asymmetric centers at any of the carbon atoms including any one of the R substituents and/or exhibit polymorphism. Consequently, compounds of formula I can exist in enantiomeric, or diastereomeric forms, or in mixtures thereof. The processes for preparation can utilize racemates, enantiomers, or diastereomers as starting materials. When diastereomeric or enantiomeric products are prepared, they can be separated by conventional methods for example, chromatographic or fractional crystallization.

"Stable compound" and "stable structure" are meant to indicate a compound that is sufficiently robust to survive isolation to a useful degree of purity from a reaction mixture, and formulation into an efficacious therapeutic agent. The present invention is intended to embody stable compounds.

"Therapeutically effective amount" is intended to include an amount of a compound of the present invention alone or an amount of the combination of compounds claimed or an amount of a compound of the present invention in combination with other active ingredients effective to inhibit MIP-1α or effective to treat or prevent inflammatory disorders.

As used herein, "treating" or "treatment" cover the treatment of a disease-state in a mammal, particularly in a human, and include: (a) preventing the disease-state from occurring in a mammal, in particular, when such mammal is predisposed to the disease-state but has not yet been diagnosed as having it; (b) inhibiting the disease-state, i.e., arresting it development; and/or (c) relieving the disease-state, i.e., causing regression of the disease state.

### SYNTHESIS

Compounds of formula I of may be prepared as shown in the following reaction schemes and description thereof, as well as relevant literature procedures that may be used by one skilled in the art. Exemplary reagents and procedures for these reactions appear hereinafter and in the working Examples.

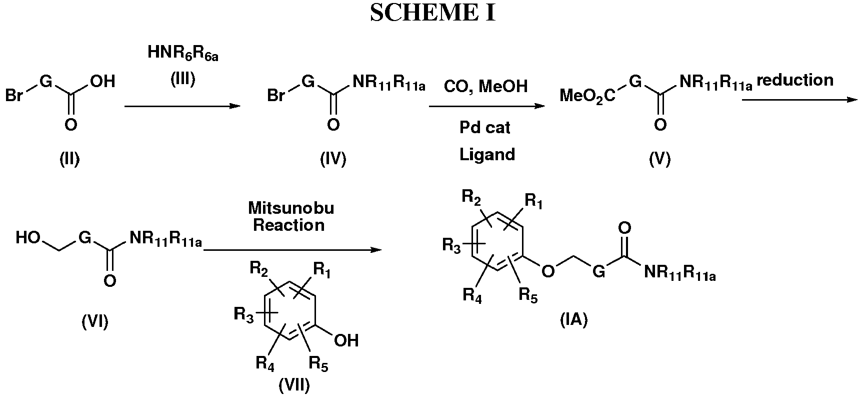

Scheme I describes a method for preparing compounds of formula **IA** (a subset of compounds of formula **I).** An acid intermediate **II** can be obtained commercially, prepared by methods known in the literature or other methods used by one skilled in the art. Formation of an amide **IV** can be obtained from an acid **II** and an amine **III** using appropriate amide coupling reagents, such as EDAC/HOBT, EDAC/HOAT, PyBOP, or those reagents described in "The Practice of Peptide Synthesis" (Spring-Verlag, 2nd Ed., Bodanszy, Miklos, 1993), to yield an amide intermediate **IV.** Carbonylation of an intermediate **IV** with an appropriate catalyst and ligand provides an ester intermediate **V.** Reduction of an ester **V** using an appropriate reducing reagent such as sodium borohydride or other reagents used by one skilled in the art provides an alcohol **VI.** Mitsunobu Reaction of an alcohol **VI** with a phenol **VII** provides compounds of formula **IA**.

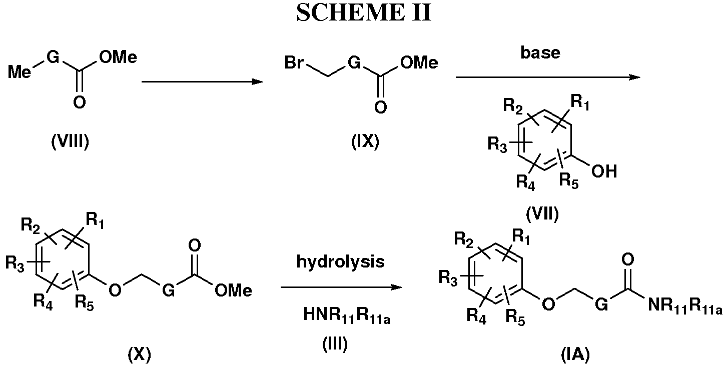

Scheme II describes another method for preparing compounds of formula **IA** (a subset of compounds of formula **I).** An intermediate **VIII** can be obtained commercially, prepared by methods known in the literature or other methods used by one skilled in the art. Bromination of an intermediate **VIII** can be obtained using NBS with an appropriate radical reaction initiator such as AIBN to provide a bromo-intermediate **IX.** Alkylation of a phenol intermediate **VII** with a bromo-intermediate **IX** provides an ester intermediate **X.** Hydrolysis of an ester **X** under basic condition followed by amide formation with an amine **III** provides compounds of formula **IA**.

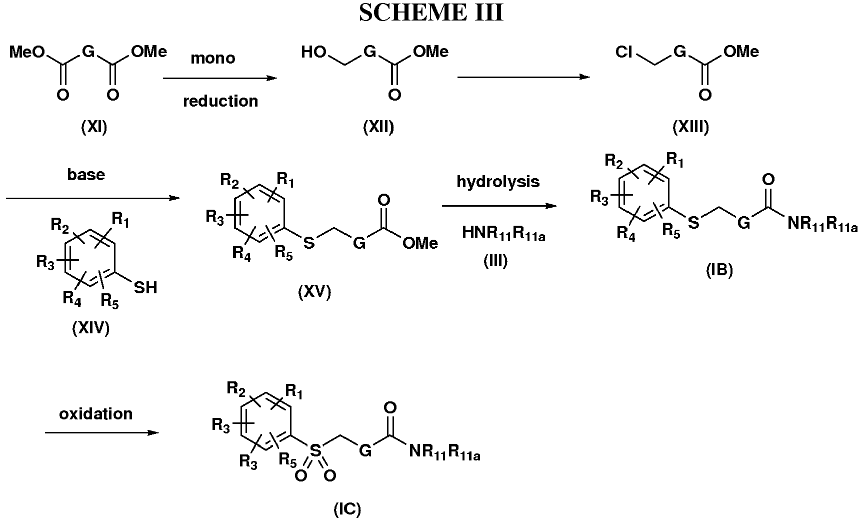

Scheme III describes a method for preparing compounds of formula **IB** and **IC** (subsets of compounds of formula **I).** A diester intermediate **XI** can be obtained commercially, prepared by methods known in the literature or other methods used by one skilled in the art. Reduction of one ester group can be obtained using an appropriate reducing reagent such as sodium borohydride or other reagents used by one skilled in the art. Chlorination of an alcohol intermediate **XII** using thionyl chloride or carbon tetrachloride/triphenyl phosphine provides an intermediate **XIII.** Alkylation of a thiophenol **XIV** with an intermediate **XIII** provides an ester intermediate **XV.** Hydrolysis of an ester **XV** under basic conditions followed by amide formation with an amine **III** provides compounds of formula **IB**. Subsequent oxidation of compounds **IB** with an appropriate oxidizing reagent such as mCPBA, Oxone^{®}, p-toluenesulfonic peracid generated in situ *(*Tetrahedron, 1996, 52, 5773-5787), or other reagents used by one skilled in the art provides compounds of formula **IC**.

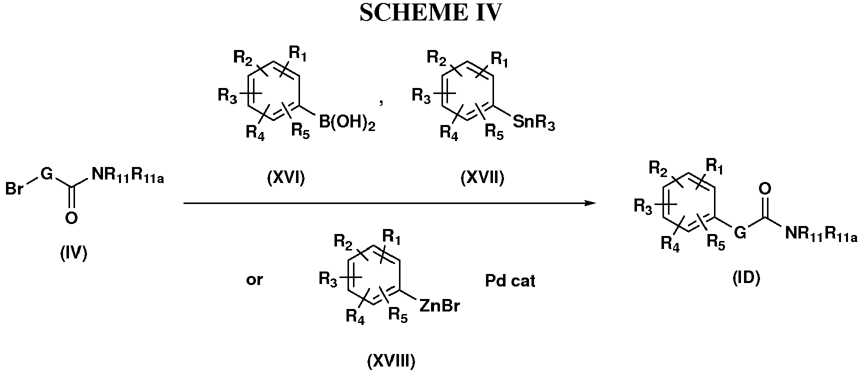

Scheme IV describes a method for preparing compounds of formula **ID** (a subset of compounds of formula **I).** A cross-coupling reaction of a bromo-intermediate **IV** (Scheme I) with a boronic acid **XVI,** an organostannane **XVII,** or an organozinc reagent **XVIII** using an appropriate catalyst and ligand provides compounds of formula **ID**.

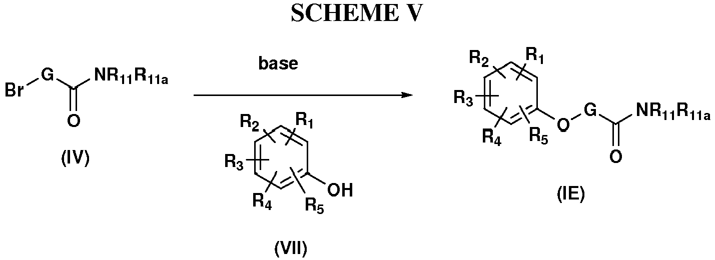

Scheme V describes a method for preparing compounds of formula **IE** (a subset of compounds of formula **I).** Nucleophilic aromatic substitution of an intermediate **IV** (Scheme I) by a phenol intermediate **VII** provides compounds of formula **IE.**

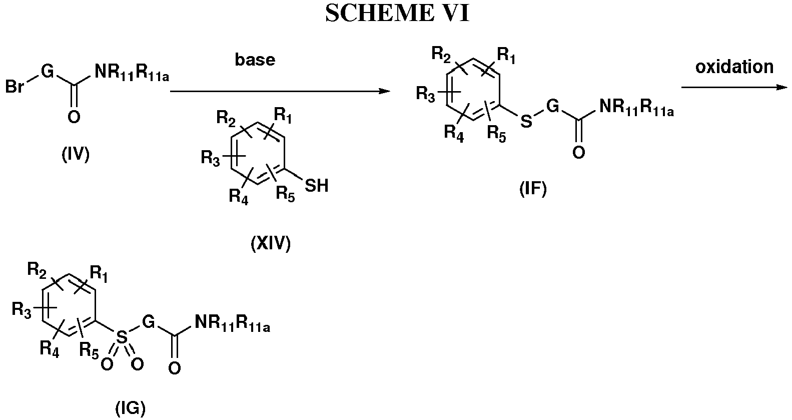

Scheme VI describes a method for preparing compounds of formula **IF** and **IG** (subsets of compounds of formula **I).** Nucleophilic aromatic substitution of an intermediate **IV** (Scheme I) by a thiophenol intermediate **XIV** provides compounds of formula **IF.** Subsequent oxidation of a compound **IF** with an appropriate oxidizing reagent such as mCPBA, Oxone^{®}, p-toluenesulfonic peracid generated in situ *(*Tetrahedron, 1996, 52, 5773-5787), or other reagents used by one skilled in the art provides a compound of formula **IG.**

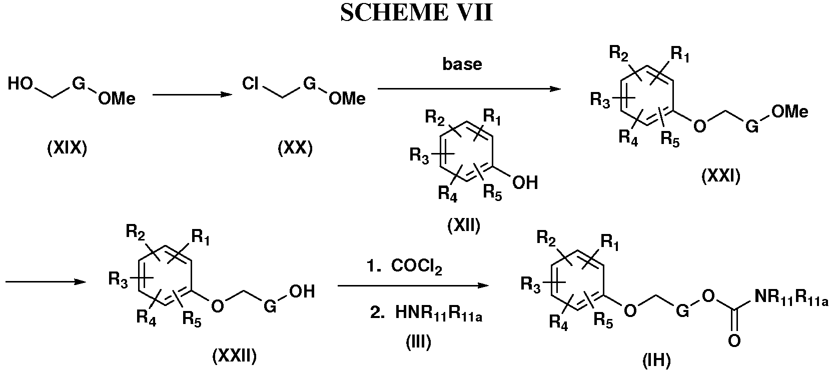

Scheme VII describes a method for preparing compounds of formula **IH** and **IJ** (subsets of compounds of formula **I).** An alcohol intermediate **XIX** can be obtained commercially, prepared by methods known in the literature, or by other methods used by one skilled in the art. Chlorination of an alcohol intermediate **XIX** using thionyl chloride or carbon tetrachloride/ triphenyl phosphine provides an intermediate XX. Alkylation of a phenol XII with an intermediate XX provides an intermediate XXI. Demethylation of an intermediate XXI can be obtained using tribromoborane or other reagents used by one skilled in the art to provide an intermediate XXII. Reaction of an intermediate XXII with phosgene followed by reaction with an amine III provides compounds of formula **IH**.

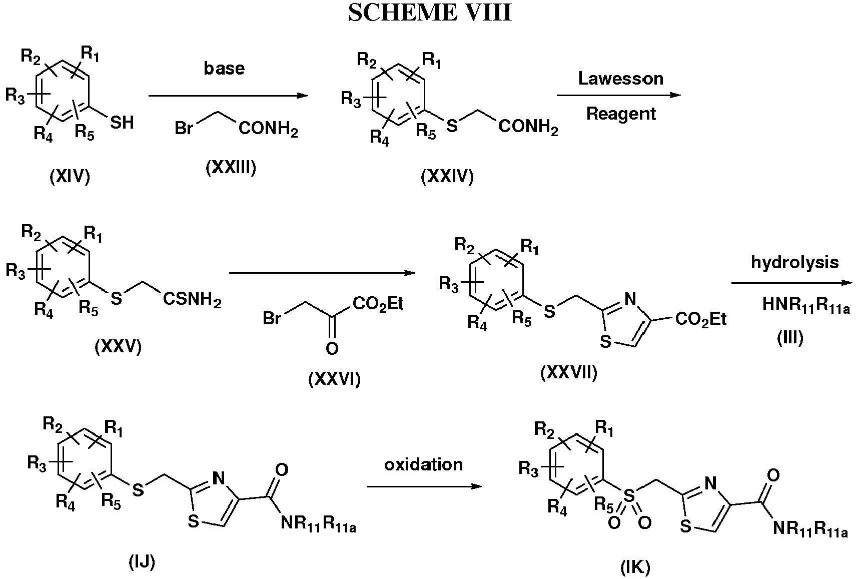

Scheme VIII describes a method for preparing compounds of formula IK and IL (subsets of compounds of formula I where G is a thiazole group). Alkylation of a thiophenol XIV with a 2-bromoacetoamide XXIII provides an amide intermediate XXIV. Reaction of an amide XXIV with Lawesson Reagent provides a thioamide intermediate XXV. Thiazole formation can be obtained from reaction of a thioamide **XXV** and a bromopyruvate **XXVI** or by other methods used by one skilled in the art. Hydrolysis of an ester **XXVII** under basic conditions followed by amide formation with an amine **III** provides compounds of formula **IK.** Subsequent oxidation of compounds **IK** with an appropriate oxidizing reagent such as mCPBA, Oxone^{®}, *p*-toluenesulfonic peracid generated in situ *(*Tetrahedron, 1996, 52, 5773-5787), or other reagents used by one skilled in the art provides compounds of formula **IL.**

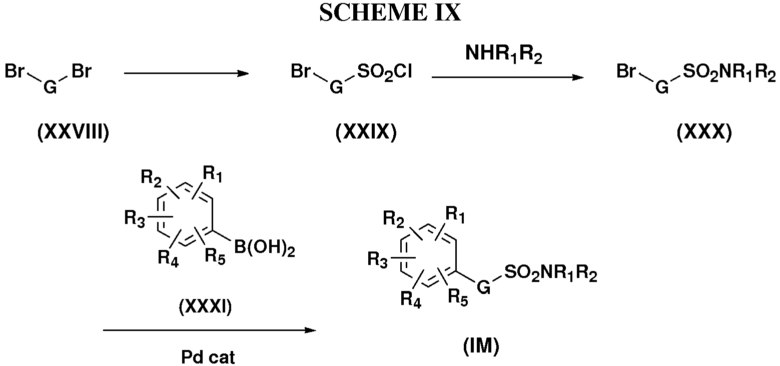

Scheme IX describes a method for preparing compounds of formula **IM.** Monolithiation *(*Tetrahedron Lett., 1996, 37, 2537-2540) of commerically available (**XXVIII**) followed by sulfinylation of the lithiated species and subsequent oxidative sulfonylation with sulfuryl chloride provides intermediate **(XXIX).** Reaction of amine with intermediate (**XXIX**) provides intermediate **(XXX).** Suzuki cross-coupling with bromo intermediate **(XXX)** using the appropriate ligand and catalyst provides compounds of formula **(IM).**

### UTILITIES AND COMBINATIONS

### A. Utilities

The compounds of the present invention possess activity as inhibitors of the enzyme 11-beta-hydroxysteroid dehydrogenase type I, and, therefore, may be used in the treatment of diseases associated with 11-beta-hydroxysteroid dehydrogenase type I activity. Via the inhibition of 11-beta-hydroxysteroid dehydrogenase type I, the compounds of the present invention may preferably be employed to inhibit glucocorticoid, thereby interrupting or modulating cortisone or cortisol production.

Accordingly, the compounds of the present invention can be administered to mammals, preferably humans, for the treatment of a variety of conditions and disorders, including, but not limited to, treating, preventing, or slowing the progression of diabetes and related conditions, microvascular complications associated with diabetes, macrovascular complications associated with diabetes, cardiovascular diseases, Metabolic Syndrome and its component conditions, and other maladies. Consequently, it is believed that the compounds of the present invention may be used in preventing, inhibiting, or treating diabetes, hyperglycemia, impaired glucose tolerance, insulin resistance, hyperinsulinemia, retinopathy, neuropathy, nephropathy, delayed wound healing, atherosclerosis and its sequelae, abnormal heart function, myocardial ischemia, stroke, Metabolic Syndrome, hypertension, obesity, dislipidemia, dylsipidemia, hyperlipidemia, hypertriglyceridemia, hypercholesterolemia, low HDL, high LDL, non-cardiac ischemia, infection, cancer, vascular restenosis, pancreatitis, neurodegenerative disease, lipid disorders, cognitive impairment and dementia, bone disease, HIV protease associated lipodystrophy and glaucoma.

Metabolic Syndrome or "Syndrome X" is described in Ford, et al., J. Am. Med. Assoc. 2002, 287, 356-359 and Arbeeny, et al., Curr. Med. Chem. - Imm., Endoc. & Metab. Agents 2001, 1, 1-24.

### B. Combinations

The present invention includes within its scope pharmaceutical compositions comprising, as an active ingredient, a therapeutically effective amount of at least one of the compounds of formula I, alone or in combination with a pharmaceutical carrier or diluent. Optionally, compounds of the present invention can be used alone, in combination with other compounds of the invention, or in combination with one or more other therapeutic agent(s), e.g., an antidiabetic agent or other pharmaceutically active material.

The compounds of the present invention may be employed in combination with other 11-beta-hydroxysteroid dehydrogenase type I inhibitors or one or more other suitable therapeutic agents useful in the treatment of the aforementioned disorders including: anti-diabetic agents, anti-hyperglycemic agents, anti-hyperinsulinemic agents, anti-retinopathic agents, anti-neuropathic agents, anti-nephropathic agents, anti-atherosclerotic agents, anti-infective agents, anti-ischemic agents, anti-hypertensive agents, anti-obesity agents, anti-dislipidemic agents, anti-dylsipidemic agents, anti-hyperlipidemic agents, anti-hypertriglyceridemic agents, anti-hypercholesterolemic agents, anti-ischemic agents, anti-cancer agents, anti-cytotoxic agents, anti-restenotic agents, anti-pancreatic agents, lipid lowering agents, appetite suppressants, memory enhancing agents and cognitive agents.

Examples of suitable anti-diabetic agents for use in combination with the compounds of the present invention include insulin and insulin analogs: LysPro insulin, inhaled formulations comprising insulin; glucagon-like peptides; sulfonylureas and analogs: chlorpropamide, glibenclamide, tolbutamide, tolazamide, acetohexamide, glypizide, glyburide, glimepiride, repaglinide, meglitinide; biguanides: metformin, phenformin, buformin; alpha2-antagonists and imidazolines: midaglizole, isaglidole, deriglidole, idazoxan, efaroxan, fluparoxan; other insulin secretagogues: linogliride, insulinotropin, exendin-4, BTS-67582, A-4166; thiazolidinediones: ciglitazone, pioglitazone, troglitazone, rosiglitazone; PPAR-gamma agonists; PPAR-alpha agonists; PPAR alpha/gamma dual agonists; SGLT2 inhibitors; dipeptidyl peptidase-IV (DPP4) inhibitors; aldose reductase inhibitors; RXR agonists: JTT-501, MCC-555, MX-6054, DRF2593, GI-262570, KRP-297, LG100268; fatty acid oxidation inhibitors: clomoxir, etomoxir; α-glucosidase inhibitors: precose, acarbose, miglitol, emiglitate, voglibose, MDL-25,637, camiglibose, MDL-73,945; beta-agonists: BRL 35135, BRL 37344, Ro 16-8714, ICI D7114, CL 316,243, TAK-667, AZ40140; phosphodiesterase inhibitors, both cAMP and cGMP type: sildenafil, L686398: L-386,398; amylin antagonists: pramlintide, AC-137; lipoxygenase inhibitors: masoprocal; somatostatin analogs: BM-23014, seglitide, octreotide; glucagon antagonists: BAY 276-9955; insulin signaling agonists, insulin mimetics, PTP1B inhibitors: L-783281, TER17411, TER17529; gluconeogenesis inhibitors: GP3034; somatostatin analogs and antagonists; antilipolytic agents: nicotinic acid, acipimox, WAG 994; glucose transport stimulating agents: BM-130795; glucose synthase kinase inhibitors: lithium chloride, CT98014, CT98023; and galanin receptor agonists.

Other suitable thiazolidinediones include Mitsubishi's MCC-555 (disclosed in U.S. Patent No. 5,594,016), Glaxo-Wellcome's GL-262570, englitazone (CP-68722, Pfizer), or darglitazone (CP-86325, Pfizer, isaglitazone (MIT/J&J), JTT-501 (JPNT/P&U), L-895645 (Merck), R-119702 (Sankyo/WL), NN-2344 (Dr. Reddy/NN), or YM-440 (Yamanouchi).

Suitable PPAR alpha/gamma dual agonists include AR-H039242 (Astra/Zeneca), GW-409544 (Glaxo-Wellcome), KRP297 (Kyorin Merck), as well as those disclosed by Murakami et al, "A Novel Insulin Sensitizer Acts As a Coligand for Peroxisome Proliferation - Activated Receptor Alpha (PPAR alpha) and PPAR gamma; Effect of PPAR alpha Activation on Abnormal Lipid Metabolism in Liver of Zucker Fatty Rats", Diabetes 47, 1841-1847 (1998), and WO 01/21602, the disclosure of which is incorporated herein by reference, employing dosages as set out therein, which compounds designated as preferred are preferred for use herein.

Suitable alpha2 antagonists also include those disclosed in WO 00/59506, employing dosages as set out herein.

Suitable SGLT2 inhibitors include T-1095, phlorizin, WAY-123783, and those described in WO 01/27128.

Suitable DPP4 inhibitors include those disclosed in WO99/38501, WO99/46272, WO99/67279 (PROBIODRUG), WO99/67278 (PROBIODRUG), WO99/61431 (PROBIODRUG), NVP-DPP728A (1-[[[2-[(5-cyanopyridin-2-yl)amino]ethyl]amino]acetyl]-2-cyano-(S)-pyrrolidine) (Novartis) as disclosed by Hughes et al, Biochemistry, 38 (36), 11597-11603, 1999, TSL-225 (tryptophyl-1,2,3,4-tetrahydroisoquinoline-3-carboxylic acid (disclosed by Yamada et al, Bioorg. & Med. Chem. Lett. 8 (1998) 1537-1540, 2-cyanopyrrolidides and 4-cyanopyrrolidides, as disclosed by Ashworth et al, Bioorg. & Med. Chem. Lett., Vol. 6, No. 22, pp 1163-1166 and 2745-2748 (1996), employing dosages as set out in the above references.

Suitable aldose reductase inhibitors include those disclosed in WO 99/26659.

Suitable meglitinides include nateglinide (Novartis) or KAD1229 (PF/Kissei).

Examples of glucagon-like peptide-1 (GLP-1) include GLP-1(1-36) amide, GLP-1(7-36) amide, GLP-1(7-37) (as disclosed in U.S. Patent No. 5,614,492 to Habener), as well as AC2993 (Amylen), and LY-315902 (Lilly).

Other anti-diabetic agents that can be used in combination with compounds of the invention include ergoset and D-chiroinositol.

Suitable anti-ischemic agents include, but are not limited to, those described in the Physician's Desk Reference and NHE inhibitors, including those disclosed in WO 99/43663.

Examples of suitable anti-infective agents are antibiotic agents, including, but not limited to, those described in the Physicians' Desk Reference.

Examples of suitable lipid lowering agents for use in combination with the compounds of the present invention include one or more MTP inhibitors, HMG CoA reductase inhibitors, squalene synthetase inhibitors, fibric acid derivatives, ACAT inhibitors, lipoxygenase inhibitors, cholesterol absorption inhibitors, ileal Na⁺/bile acid cotransporter inhibitors, upregulators of LDL receptor activity, bile acid sequestrants, cholesterol ester transfer protein inhibitors (e.g., CP-529414 (Pfizer)), and/or nicotinic acid and derivatives thereof.

MTP inhibitors which may be employed as described above include those disclosed in U.S. Patent No. 5,595,872, U.S. Patent No. 5,739,135, U.S. Patent No. 5,712,279, U.S. Patent No. 5,760,246, U.S. Patent No. 5,827,875, U.S. Patent No. 5,885,983, and U.S. Patent No. 5,962,440.

The HMG CoA reductase inhibitors which may be employed in combination with one or more compounds of formula I include mevastatin and related compounds, as disclosed in U.S. Patent No. 3,983,140, lovastatin, (mevinolin) and related compounds, as disclosed in U.S. Patent No. 4,231,938, pravastatin, and related compounds, such as disclosed in U.S. Patent No. 4,346,227, simvastatin, and related compounds, as disclosed in U.S. Patent Nos. 4,448,784 and 4,450,171. Other HMG CoA reductase inhibitors which may be employed herein include, but are not limited to, fluvastatin, disclosed in U.S. Patent No. 5,354,772; cerivastatin, as disclosed in U.S. Patent Nos. 5,006,530 and 5,177,080; atorvastatin, as disclosed in U.S. Patent Nos. 4,681,893, 5,273,995, 5,385,929 and 5,686,104; atavastatin (Nissan/Sankyo's nisvastatin (NK-104)), as disclosed in U.S. Patent No. 5,011,930; visastatin (Shionogi-Astra/Zeneca (ZD-4522)) as disclosed in U.S. Patent No. 5,260,440; and related statin compounds disclosed in U.S. Patent No. 5,753,675; pyrazole analogs of mevalonolactone derivatives, as disclosed in U.S. Patent No. 4,613,610; indene analogs of mevalonolactone derivatives, as disclosed in PCT application WO 86/03488; 6-[2-(substituted-pyrrol-1-yl)alkyl)pyran-2-ones and derivatives thereof, as disclosed in U.S. Patent No. 4,647,576; Searle's SC-45355 (a 3-substituted pentanedioic acid derivative) dichloroacetate; imidazole analogs of mevalonolactone, as disclosed in PCT application WO 86/07054; 3-carboxy-2-hydroxy-propane-phosphonic acid derivatives, as disclosed in French Patent No. 2,596,393; 2,3-disubstituted pyrrole, furan and thiophene derivatives, as disclosed in European Patent Application No. 0221025; naphthyl analogs of mevalonolactone, as disclosed in U.S. Patent No. 4,686,237; octahydronaphthalenes, such as disclosed in U.S. Patent No. 4,499,289; keto analogs of mevinolin (lovastatin), as disclosed in European Patent Application No.0142146 A2; and quinoline and pyridine derivatives, as disclosed in U.S. Patent Nos. 5,506,219 and 5,691,322.

Preferred hypolipidemic agents are pravastatin, lovastatin, simvastatin, atorvastatin, fluvastatin, cerivastatin, atavastatin, and ZD-4522.

In addition, phosphinic acid compounds useful in inhibiting HMG CoA reductase, such as those disclosed in GB 2205837, are suitable for use in combination with the compounds of the present invention.

The squalene synthetase inhibitors suitable for use herein include, but are not limited to, α-phosphono-sulfonates disclosed in U.S. Patent No. 5,712,396, those disclosed by Biller et al, J. Med. Chem., 1988, Vol. 31, No. 10, pp 1869-1871, including isoprenoid (phosphinyl-methyl)phosphonates, as well as other known squalene synthetase inhibitors, for example, as disclosed in U.S. Patent No. 4,871,721 and 4,924,024 and in Biller, S.A., Neuenschwander, K., Ponpipom, M.M., and Poulter, C.D., Current Pharmaceutical Design, 2, 1-40 (1996).

In addition, other squalene synthetase inhibitors suitable for use herein include the terpenoid pyrophosphates disclosed by P. Ortiz de Montellano et al, J. Med. Chem., 1977, 20, 243-249, the farnesyl diphosphate analog A and presqualene pyrophosphate (PSQ-PP) analogs as disclosed by Corey and Volante, J. Am. Chem. Soc., 1976, 98, 1291-1293, phosphinylphosphonates reported by McClard, R.W. et al, J.A.C.S., 1987, 109, 5544 and cyclopropanes reported by Capson, T.L., Ph.D. dissertation, June, 1987, Dept. Med. Chem. U of Utah, Abstract, Table of Contents, pp. 16, 17, 40-43, 48-51, Summary.

The fibric acid derivatives which may be employed in combination with one or more compounds of formula I include fenofibrate, gemfibrozil, clofibrate, bezafibrate, ciprofibrate, clinofibrate, and the like, probucol, and related compounds, as disclosed in U.S. Patent No. 3,674,836, probucol and gemfibrozil being preferred, bile acid sequestrants, such as cholestyramine, colestipol and DEAE-Sephadex (Secholex^{®}, Policexide^{®}), as well as lipostabil (Rhone-Poulenc), Eisai E-5050 (an N-substituted ethanolamine derivative), imanixil (HOE-402), tetrahydrolipstatin (THL), istigmastanylphosphorylcholine (SPC, Roche), aminocyclodextrin (Tanabe Seiyoku), Ajinomoto AJ-814 (azulene derivative), melinamide (Sumitomo), Sandoz 58-035, American Cyanamid CL-277,082 and CL-283,546 (disubstituted urea derivatives), nicotinic acid, acipimox, acifran, neomycin, p-aminosalicylic acid, aspirin, poly(diallylmethylamine) derivatives, such as disclosed in U.S. Patent No. 4,759,923, quaternary amine poly(diallyldimethylammonium chloride) and ionenes, such as disclosed in U.S. Patent No. 4,027,009, and other known serum cholesterol lowering agents.

The ACAT inhibitor which may be employed in combination with one or more compounds of formula I include those disclosed in Drugs of the Future 24, 9-15 (1999), (Avasimibe); "The ACAT inhibitor, Cl-1011 is effective in the prevention and regression of aortic fatty streak area in hamsters", Nicolosi et al, Atherosclerosis (Shannon, Irel). (1998), 137(1), 77-85; "The pharmacological profile of FCE 27677: a novel ACAT inhibitor with potent hypolipidemic activity mediated by selective suppression of the hepatic secretion of ApoB 100-containing lipoprotein", Ghiselli, Giancarlo, Cardiovasc. Drug Rev. (1998), 16(1), 16-30; "RP 73163: a bioavailable alkylsulfinyl-diphenylimidazole ACAT inhibitor", Smith, C., et al, Bioorg. Med. Chem. Lett. (1996), 6(1), 47-50; "ACAT inhibitors: physiologic mechanisms for hypolipidemic and anti-atherosclerotic activities in experimental animals", Krause et al, Editor(s): Ruffolo, Robert R., Jr.; Hollinger, Mannfred A., Inflammation: Mediators Pathways (1995), 173-98, Publisher: CRC, Boca Raton, Fla.; "ACAT inhibitors: potential anti-atherosclerotic agents", Sliskovic et al, Curr. Med. Chem. (1994), 1(3), 204-25; "Inhibitors of acyl-CoA:cholesterol O-acyl transferase (ACAT) as hypocholesterolemic agents. 6. The first water-soluble ACAT inhibitor with lipid-regulating activity. Inhibitors of acyl-CoA:cholesterol acyltransferase (ACAT). 7. Development of a series of substituted N-phenyl-N'-[(1-phenylcyclopentyl)methyl]ureas with enhanced hypocholesterolemic activity", Stout et al, Chemtracts: Org. Chem. (1995), 8(6), 359-62, or TS-962 (Taisho Pharmaceutical Co. Ltd.).

The hypolipidemic agent may be an upregulator of LD2 receptor activity, such as MD-700 (Taisho Pharmaceutical Co. Ltd) and LY295427 (Eli Lilly).

Examples of suitable cholesterol absorption inhibitors for use in combination with the compounds of the invention include SCH48461 (Schering-Plough), as well as those disclosed in Atherosclerosis 115, 45-63 (1995) and J. Med. Chem. 41, 973 (1998).

Examples of suitable ileal Na⁺/bile acid cotransporter inhibitors for use in combination with the compounds of the invention include compounds as disclosed in Drugs of the Future, 24, 425-430 (1999).

The lipoxygenase inhibitors which may be employed in combination with one or more compounds of formula I include 15-lipoxygenase (15-LO) inhibitors, such as benzimidazole derivatives, as disclosed in WO 97/12615, 15-LO inhibitors, as disclosed in WO 97/12613, isothiazolones, as disclosed in WO 96/38144, and 15-LO inhibitors, as disclosed by Sendobry et al "Attenuation of diet-induced atherosclerosis in rabbits with a highly selective 15-lipoxygenase inhibitor lacking significant antioxidant properties", Brit. J. Pharmacology (1997) 120, 1199-1206, and Cornicelli et al, "15-Lipoxygenase and its Inhibition: A Novel Therapeutic Target for Vascular Disease", Current Pharmaceutical Design, 1999, 5, 11-20.

Examples of suitable anti-hypertensive agents for use in combination with the compounds of the present invention include beta adrenergic blockers, calcium channel blockers (L-type and T-type; e.g. diltiazem, verapamil, nifedipine, amlodipine and mybefradil), diuretics (e.g., chlorothiazide, hydrochlorothiazide, flumethiazide, hydroflumethiazide, bendroflumethiazide, methylchlorothiazide, trichloromethiazide, polythiazide, benzthiazide, ethacrynic acid tricrynafen, chlorthalidone, furosemide, musolimine, bumetanide, triamtrenene, amiloride, spironolactone), renin inhibitors, ACE inhibitors (e.g., captopril, zofenopril, fosinopril, enalapril, ceranopril, cilazopril, delapril, pentopril, quinapril, ramipril, lisinopril), AT-1 receptor antagonists (e.g., losartan, irbesartan, valsartan), ET receptor antagonists (e.g., sitaxsentan, atrsentan, and compounds disclosed in U.S. Patent Nos. 5,612,359 and 6,043,265), Dual ET/AII antagonist (e.g., compounds disclosed in WO 00/01389), neutral endopeptidase (NEP) inhibitors, vasopepsidase inhibitors (dual NEP-ACE inhibitors) (e.g., omapatrilat and gemopatrilat), and nitrates.

Examples of suitable anti-obesity agents for use in combination with the compounds of the present invention include a cannabinoid receptor 1 antagonist or inverse agonist, a beta 3 adrenergic agonist, a lipase inhibitor, a serotonin (and dopamine) reuptake inhibitor, a thyroid receptor beta drug, and/or an anorectic agent.

Cannabinoid receptor 1 antagonists and inverse agonists which may be optionally employed in combination with compounds of the present invention include rimonabant, SLV 319, and those discussed in D. L. Hertzog, Expert Opin. Ther. Patents 2004, 14, 1435-1452.

The beta 3 adrenergic agonists which may be optionally employed in combination with compounds of the present invention include AJ9677 (Takeda/Dainippon), L750355 (Merck), or CP331648 (Pfizer,) or other known beta 3 agonists, as disclosed in U.S. Patent Nos. 5,541,204, 5,770,615, 5,491,134, 5,776,983, and 5,488,064, with AJ9677, L750,355, and CP331648 being preferred.

Examples of lipase inhibitors which may be optionally employed in combination with compounds of the present invention include orlistat or ATL-962 (Alizyme), with orlistat being preferred.

The serotonin (and dopoamine) reuptake inhibitor which may be optionally employed in combination with a compound of formula I may be sibutramine, topiramate (Johnson & Johnson), or axokine (Regeneron), with sibutramine and topiramate being preferred.

Examples of thyroid receptor beta compounds which may be optionally employed in combination with compounds of the present invention include thyroid receptor ligands, such as those disclosed in WO97/21993 (U. Cal SF), WO99/00353 (KaroBio), and WO00/039077 (KaroBio), with compounds of the KaroBio applications being preferred.

The anorectic agent which may be optionally employed in combination with compounds of the present invention include dexamphetamine, phentermine, phenylpropanolamine, or mazindol, with dexamphetamine being preferred.

Other compounds that can be used in combination with the compounds of the present invention include CCK receptor agonists (e.g., SR-27895B); galanin receptor antagonists; MCR-4 antagonists (e.g., HP-228); leptin or mimentics; 11-beta-hydroxysteroid dehydrogenase type-1 inhibitors; urocortin mimetics, CRF antagonists, and CRF binding proteins (e.g., RU-486, urocortin).

Further, the compounds of the present invention may be used in combination with anti-cancer and cytotoxic agents, including but not limited to alkylating agents such as nitrogen mustards, alkyl sulfonates, nitrosoureas, ethylenimines, and triazenes; antimetabolites such as folate antagonists, purine analogues, and pyrimidine analogues; antibiotics such as anthracyclines, bleomycins, mitomycin, dactinomycin, and plicamycin; enzymes such as L-asparaginase; farnesyl-protein transferase inhibitors; 5α reductase inhibitors; inhibitors of 17β-hydroxy steroid dehydrogenase type 3; hormonal agents such as glucocorticoids, estrogens/antiestrogens, androgens/antiandrogens, progestins, and luteinizing hormone-releasing hormone antagonists, octreotide acetate; microtubule-disruptor agents, such as ecteinascidins or their analogs and derivatives; microtubule-stabilizing agents such as taxanes, for example, paclitaxel (Taxol^{®}), docetaxel (Taxotere^{®}), and their analogs, and epothilones, such as epothilones A-F and their analogs; plant-derived products, such as vinca alkaloids, epipodophyllotoxins, taxanes; and topiosomerase inhibitors; prenyl-protein transferase inhibitors; and miscellaneous agents such as hydroxyurea, procarbazine, mitotane, hexamethylmelamine, platinum coordination complexes such as cisplatin and carboplatin; and other agents used as anti-cancer and cytotoxic agents such as biological response modifiers, growth factors; immune modulators; and monoclonal antibodies. Additional anti-cancer agents are disclosed in EP 1177791. The compounds of the invention may also be used in conjunction with radiation therapy.

Examples of suitable memory enhancing agents, anti-dementia agents, or cognitive agents for use in combination with the compounds of the present invention include, but are not limited to, donepezil, rivastigmine, galantamine, memantine, tacrine, metrifonate, muscarine, xanomelline, deprenyl and physostigmine.

The aforementioned patents and patent applications are incorporated herein by reference.

The above other therapeutic agents, when employed in combination with the compounds of the present invention may be used, for example, in those amounts indicated in the Physician's Desk Reference, as in the patents set out above, or as otherwise determined by one of ordinary skill in the art.

The compounds of formula I can be administered for any of the uses described herein by any suitable means, for example, orally, such as in the form of tablets, capsules, granules or powders; sublingually; bucally; parenterally, such as by subcutaneous, intravenous, intramuscular, or intrasternal injection, or infusion techniques (e.g., as sterile injectable aqueous or non-aqueous solutions or suspensions); nasally, including administration to the nasal membranes, such as by inhalation spray; topically, such as in the form of a cream or ointment; or rectally such as in the form of suppositories; in dosage unit formulations containing non-toxic, pharmaceutically acceptable vehicles or diluents.

In carrying out the method of the invention for treating diabetes and related diseases, a pharmaceutical composition will be employed containing the compounds of formula I, with or without other antidiabetic agent(s) and/or antihyperlipidemic agent(s) and/or other type therapeutic agents in association with a pharmaceutical vehicle or diluent. The pharmaceutical composition can be formulated employing conventional solid or liquid vehicles or diluents and pharmaceutical additives of a type appropriate to the mode of desired administration, such as pharmaceutically acceptable carriers, excipients, binders, and the like. The compounds can be administered to a mammalian patient, including humans, monkeys, dogs, etc. by an oral route, for example, in the form of tablets, capsules, beads, granules or powders. The dose for adults is preferably between 1 and 2,000 mg per day, which can be administered in a single dose or in the form of individual doses from 1-4 times per day.

A typical capsule for oral administration contains compounds of structure I (250 mg), lactose (75 mg), and magnesium stearate (15 mg). The mixture is passed through a 60 mesh sieve and packed into a No. 1 gelatin capsule.

A typical injectable preparation is produced by aseptically placing 250 mg of compounds of structure I into a vial, aseptically freeze-drying and sealing. For use, the contents of the vial are mixed with 2 mL of physiological saline, to produce an injectable preparation.

### ASSAY(S) FOR 11-BETA-HYDROXYSTEROID DEHYDROGENASE ACTIVITY

The in vitro inhibition of recombinant human 11beta-HSD1 was determined as follows.

Recombinant human 11beta-HSD1 was expressed stably in HEK 293 EBNA cells. Cells were grown in DMEM (high glucose) containing MEM non-essential amino acids, L-glutamine, hygromycine B (200ug/ml), and G418(200ug/ml). The cell pellets were homogenized, and the microsomal fraction was obtained by differential centrifugation. 11beta-HSD1 over expressed microsomes were used as the enzyme source for the Scintillation Proximity Assay (SPA). The test compounds at the desired concentration were incubated at room temperature with 12.5 µg of microsomal enzyme, 250 nM [³H]-cortisone, 500 µM NADPH, 50 mM MES, pH 6.5, and 5 mM EDTA in 96-well OptiPlates. The reaction was terminated with the addition of 1 mM 18β-glycerrhentic acid. SPA reagent mixture (YSi anti-rabbit IgG, anti-cortisol antibody in 50 mM Tris, pH 8.0 containing 1% CHAPS and 1% glycerol) was added and the reaction was further incubated at room temperature over night and counted in TopCount. The IC₅₀ (concentration of compound required for 50% inhibition of cortisol formation) was determined using XLfit.

As a means of confirming selectivity for 11betaHSD1, the compounds of the present invention were also screened for 11betaHSD2 activity. The in vitro inhibition of recombinant human 11betaHSD2 was determined as follows:

Recombinant human 11betaHSD2 was expressed stably in HEK 293 EBNA cells. The microsomal fraction over expressing 11betaHSD2 was prepared from the cell homogenate. The test compounds at the desired concentration were incubated at 37°C with 10 µg of microsomal enzyme, 100 nM-cortisol, 1 mM NAD, and 20 mM Tris, pH 7.5 in 96-well plates for 3h. The reaction was stopped with the addition of equal volume of acetonitrile containing 200 ng/mL triamcinolone (internal standard). The plate was centrifuged and the supernatant was transferred to another 96-well assay plate. Cortisone in the samples was analyzed by LC/MS/MS (Micromass Quattro Ultima Triple Quadrupole Mass Spectrometer). From the MS response (ratio of compound to the internal standard), cortisone formation was calculated using the cortisone standard curve determined on each plate. The IC₅₀ (concentration of compound required for 50% inhibition of cortisone formation) was determined using XLfit.

In general, preferred compounds of the present invention, such as particular compounds disclosed in the following examples, have been identified to inhibit the catalytic activity of 11-beta-hydroxysteroid dehydrogenase type I at concentrations equivalent to, or more potently than, 10 µM, preferably 5 µM, more preferably 3 µM, thereby demonstrating compounds of the present invention as especially effective inhibitors of 11-beta-hydroxysteroid dehydrogenase type I. Potencies can be calculated and expressed as either inhibition constants (Ki values) or as IC50 (inhibitory concentration 50%) values, and refer to activity measured employing the assay system described above.

### EXAMPLES

The following working Examples serve to better illustrate, but not limit, some of the preferred embodiments of the present invention.

### GENERAL

The term HPLC refers to a Shimadzu high performance liquid chromatography with one of following methods:
**Method A**: YMC or Phenomenex C18 5 micron 4.6 X 50mm column using a 4 minute gradient of 0-100% solvent B [90% MeOH:10% H₂O:0.2% H₃PO₄] and 100-0% solvent A [10% MeOH:90% H₂O:0.2% H₃PO₄] with 4 mL/min flow rate and a 1 min. hold, an ultra violet (uv) detector set at 220 nm.
**Method B:** Phenomenex S5 ODS 4.6 x 30 mm column, gradient elution 0-100% B/A over 2 min (solvent A = 10% MeOH/H₂O containing 0.1% TFA, solvent B = 90% MeOH/H₂O containing 0.1% TFA), flow rate 5 mL/min, UV detection at 220 nm.
**Method C:** YMC S7 ODS 3.0 x 50 mm column, gradient elution 0-100% B/A over 2 min (solvent A = 10% MeOH/H₂O containing 0.1% TFA, solvent B = 90% MeOH/H₂O containing 0.1% TFA), flow rate 5 mL/min, UV detection at 220 nm.

The term prep HPLC refers to an automated Shimadzu HPLC system using a mixture of solvent A (10% MeOH/90%H₂O/0.2%TFA) and solvent B (90% MeOH/10%H₂O/0.2% TFA). The preparative columns are packed with YMC or Phenomenex ODS C18 5 micron resin or equivalent.

### ABBREVIATIONS

The following abbreviations are employed in the Examples and elsewhere herein:
Ph = phenyl
Bn = benzyl
i-Bu = iso-butyl
Me = methyl
Et = ethyl
Pr = propyl
Bu = butyl
AIBN = 2,2'-Azobisisobutyronitrile
TMS = trimethylsilyl
FMOC = fluorenylmethoxycarbonyl
Boc or BOC = tert-butoxycarbonyl
Cbz = carbobenzyloxy or carbobenzoxy or benzyloxycarbonyl
HOAc or AcOH = acetic acid
DCM = dichloromethane
DIEA = N,N-diisopropylethylamine
DMA = N,N-dimethylacetylamide
DMF = N,N-dimethylformamide
DMSO = dimethylsulfoxide
EtOAc = ethyl acetate
THF = tetrahydrofuran
TFA = trifluoroacetic acid
mCPBA = 3-Chloroperoxybenzoic acid
NMM = N-methyl morpholine
NBS = N-Bromosuccinimide
n-BuLi = n-butyllithium
Oxone^{®} = Monopersulfate
Pd/C = palladium on carbon
PtO₂ = platinum oxide
TEA = triethylamine
EDAC = 3-ethyl-3'-(dimethylamino)propyl-carbodiimide hydrochloride (or 1-[(3-(dimethyl)amino)propyl])-3-ethylcarbodiimide hydrochloride)
HOBT or HOBT•H₂O = 1-hydroxybenzotriazole hydrate
HOAT = 1-hydroxy-7-azabenzotriazole
PyBOP reagent = benzotriazol-1-yloxy-tripyrrolidino phosphonium hexafluorophosphate
equiv = equivalent(s)
min = minute(s)
h or hr = hour(s)
L = liter
mL = milliliter
µL = microliter
g = gram(s)
mg = milligram(s)
mol = mole(s)
mmol = millimole(s)
meq = milliequivalent
RT or R.T. = room temperature
sat or sat'd = saturated
aq. = aqueous
TLC = thin layer chromatography
HPLC = high performance liquid chromatography
HPLC Rₜ = HPLC retention time
LC/MS = high performance liquid chromatography/mass spectrometry
MS or Mass Spec = mass spectrometry
NMR = nuclear magnetic resonance
mp = melting point
PXPd₂ = Dichloro(chlorodi-tert-butylphosphine)palladium (II) dimer or [PdCl₂(*t-*Bu)₂PCl]₂

### EXAMPLE 1 (5-((2,6-Dichlorophenylthio)methyl)pyridin-3-yl)(4-methylpiperidin-1-yl)methanone

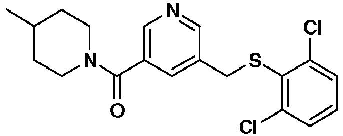

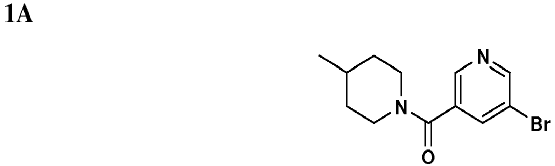

To a solution of 5-bromonicotinic acid (4.7 g, 23.27 mmol) in THF (90 mL) was added 4-methylmorpholine (2.56 ml, 23.27 mmol) and isobutyl chloroformate (3.03 ml, 23.27 mmol) at 0°C. The mixture was stirred at 0°C for 1.5 hours and then 4-methyl piperidine (9.7 g, 97.73 mmol) was added at 0°C. The suspension was stirred at 0°C to room temperature for 2 hours. The white precipitate was filtered off, and the liquid portion was concentrated under vacuum. The residue was purified by column chromatography to yield compound **1A** (5.36 g) as a white powder. HPLC Rₜ (Method A): 2.75 min. LCMS: m/z 283 (M + H⁺).

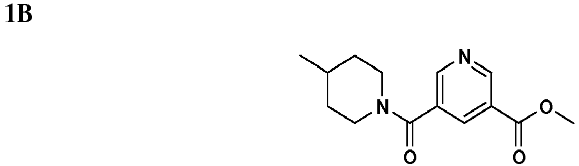

To a solution of compound **1A** (2 g, 7.063 mmol) in DMF (14 mL) was added palladium acetate (791 mg, 3.53 mmol), 1,3-bis(diphenylphosphino)-propane (1.163 g, 2.83 mmol), DBU (1.29 g, 8.48 mmol), and methanol (14 mL) in a steel auto clave container. The mixture was stirred and heated at 85°C for 14 hours under carbon monoxide (70 psi). After cooling the container, the methanol was concentrated via vacuum, and the residue was diluted with ethyl acetate. The powders were filtered off, and the mixture was washed with brine and water. Drying over MgSO₄, followed by concentration and column chromatography purification yielded compound **1B** (1.6 g) as a yellow oil. HPLC Rₜ (Method A) 2.497 min. LCMS: m/z 263 (M + H⁺).

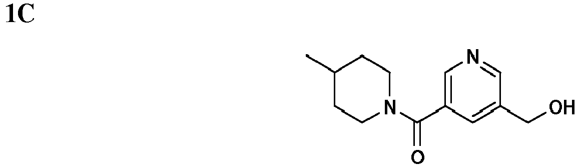

Compound **1B** (1.6 g, 6.1 mmol) in ethanol (20 mL) was treated with sodium borohydride (462 mg, 12.2 mmol) at room temperature and stirred for 1 hour. The solution was quenched with water and neutralized to pH=7. The mixture was stripped of most of the ethanol, basified with 1N NaOH solution, and extracted 3 times with ethyl acetate. The combined organic extracts were dried over MgSO₄, filtered, and concentrated via vacuum to yield compound **1C** (310 mg) as a yellow oil. HPLC Rₜ (Method A): 1.218 min, LCMS: m/z 235 (M + H⁺).

### Example 1

Compound 1C (200 mg, 0.853 mmol) in DCM (10 mL) was treated with 1N PBr₃ (0.64 mL, 0.64 mmol) at 0°C for 1.5 hours. The mixture was quenched with 5 mL saturated NaHCO₃ solution at 0°C. The solution was diluted with DCM. The organic layer was separated, washed with brine, and dried over MgSO₄. The drying agent was filtered, and the filtrate was concentrated via vacuum to yield the bromide as a colorless oil. The bromide was dissolved in THF (10 mL) and treated with 2,6-dichlorothiophenol (153 mg, 0.853 mmol) and N,N-diisopropyl-ethylamine (331mg, 2.56 mmol) at room temperature overnight. The mixture was concentrated and purified by column chromatography to yield **Example 1** (76.7 mg) as a white powder. HPLC Rₜ (Method A: 3.618 min. LCMS: m/z 395 (M + H⁺). HPLC purity: 99%. ¹H NMR: δ 8.42 (s, 1H), 8.31 (s, 1H), 7.58 (s, 1H), 7.30 (d, J = 8.2 Hz, 2H), 7.15 (t, J = 8.2 Hz, 1H), 4.70-4.55 (m, 1H), 4.08 (s, 2H), 3.60-3.48 (m, 1H), 3.08-2.86 (m, 1H), 2.85-2.70 (m, 1H), 1.80-1.57 (m, 3H), 1.30-1.09 (m, 2H), 0.97 (d, J = 6 Hz, 3H).

### EXAMPLE 2

### (5-((2,6-Dichlorophenylsulfonyl)methyl)pyridin-3-yl)(4-methylpiperidin-1-yl)methanone

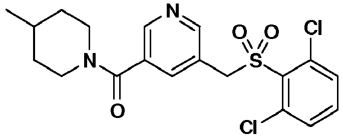

To a solution of **Example 1** (58 mg, 0.147 mmol) in THF (2 mL) and MeOH (2 mL) was added 1-(p-toluenesulfonyl)imidazole (261 mg, 1.18 mmol), 30% aqueous H₂O₂ (240 µL, 2.352 mmol), and 1 N NaOH (2.7 mL, 2.7 mmol). The mixture was stirred at room temperature for 2.5 hours. The organic solvents were removed *in vacuo,* and the aqueous portion was diluted with brine and ethyl acetate. The organic portion was separated, and the aqueous layer was extracted again with ethyl acetate. The organic extracts were combined, dried over MgSO₄, and concentrated. The residue was subjected to preparative HPLC to yield **Example 2** (41 mg) as a white powder. HPLC Rₜ (Method A): 2.868 min. LCMS: m/z 427 (M + H₊). HPLC purity: 99%. ¹H NMR δ 8.57 (s, 1H), 8.33 (s, 1H), 7.82 (s, 1H), 7.40-7.32 (m, 3H), 4.64 (s, 2H), 4.57-4.54 (m, 1H), 3.62-3.48 (m, 1H), 3.05-2.97 (m, 1H), 2.82-2.70 (m, 1H), 1.80-1.70 (m, 1H), 1.70-1.52 (m, 2H), 1.27-0.99 (m, 2H), 0.97 (d, J = 6Hz, 3H).

### EXAMPLE 3 (Reference) 2-((2,6-Dichlorophenylthio)methyl)-5-(4-methylpiperidin-1-ylsulfonyl)-pyridine

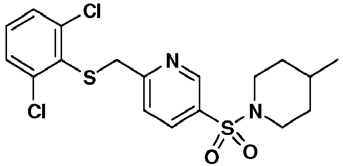

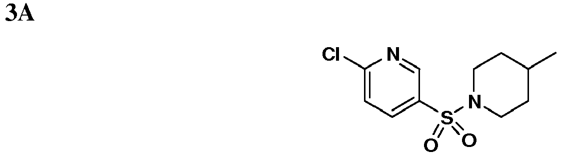

To a solution of 6-chloropyridine-3-sulfonyl chloride (600 mg, 2.83 mmol) in DCM (10 mL) was added DIEA (1.5 mL, 8.49 mmol) and 4-methylpiperidine (281 mg, 2.83 mmol) at RT. The mixture stirred for 2 hours. The solvent was removed under reduced pressure, and the residue was purified by column chromatography to yield compound **3A** (746 mg) as a white powder. HPLC Rₜ (Method A): 2.982 min. LCMS: m/z 275 (M + H⁺).

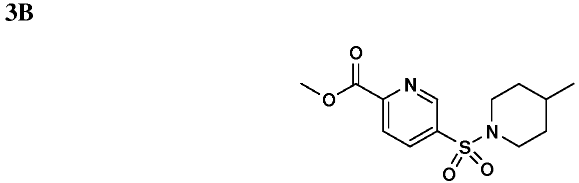

Compound **3B** was prepared in a similar manner as compound **1B**. Carbonylation of compound 3A (550 mg) gave compound **3B** (580 mg) as a white powder. HPLC Rₜ (Method A): 2.682 min. LCMS: m/z 299 (M + H⁺).

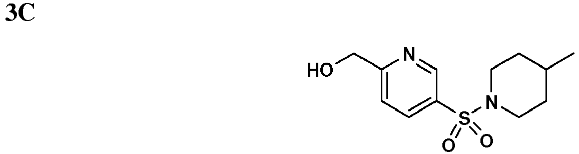

To a solution of compound **3B** (400 mg, 1.34 mmol) in THF (8 mL) was added 1N LiAlH₄ (0.67 mL, 0.67 mmol) solution in THF at RT. The mixture stirred for 2 hours, was quenched with H₂O, and was extracted 3 times with ethyl acetate. The combined organic extracts were dried over MgSO₄, filtered, and concentrated. The residue was purified by silical gel chromatography to yield compound **3C** (120 mg) as a light pink powder. HPLC Rₜ (Method A): 2.315min. LCMS: m/z 271 (M + H⁺).

### Example 3

To a solution of compound **3C** (80 mg, 0.296 mmol) in THF (2 mL) at RT was added 2,6-dichlorobenzenethiol (212 mg, 1.184 mmol), and PPh₃ (233 mg, 0.888 mmol). After the solution became homogeneous, diisopropyl azodicarboxylate (180 mg, 0.888 mmol) was added via syringe. After 5 minutes of stirring at RT, the mixture became cloudy. DCM (1.5 mL) was added and stirring was continued for another 2 hours. The precipitate was filtered off, and the solvents were removed at reduced pressure. The residue was purified by silical gel chromatography, followed by prep HPLC to give **Example 3.** HPLC Rₜ (Method A): 3.788 min. LCMS: m/z 431 (M + H⁺). HPLC purity: 97%. ¹H NMR: δ 8.80 (s, 1H), 7.93-7.88 (m, 1H), 7.36-7.20 (m, 4H), 4.28 (s, 2H), 3.80-3.73 (m, 2H), 2.36-2.22 (m, 2H), 1.81-1.63 (m, 2H), 1.45-1.26 (m, 3H), 0.97 (d, J = 5.1 Hz, 3H).

### EXAMPLE 4 (Reference) 2-((2,6-Dichlorophenylsulfonyl)methyl)-5-(4-methylpiperidin-1-ylsulfonyl)pyridine

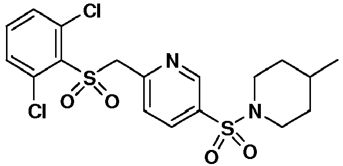

Example 4 was prepared in a similar manner as Example 2, and obtained as a white powder. HPLC Rₜ (Method A): 3.127 min. LCMS: m/z 463 (M + H⁺). HPLC purity: 95%. ¹H NMR: δ 8.61 (s, 1H), 7.96-7.93 (m, 1H), 7.63-7.61 (m, 1H), 7.34-7.31 (m, 3H), 4.83 (s, 2H), 3.64 (d, J = 11.6 Hz, 2H), 2.22-2.10 (m, 2H), 1.69-1.52 (m, 2H), 1.31-1.12 (m, 3H), 0.85 (d, J = 5.7 Hz, 3H)

### EXAMPLE 5 (Reference) 2-((2,6-Dichlorophenoxy)methyl)-5-(4-methylpiperidin-1-ylsulfonyl)-pyridine

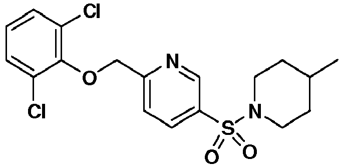

To a solution of compound 3C (10 mg, 0.037 mmol) in THF (1 mL) was added 2,6-dichlorophenol (18.1 mg, 0.111 mmol) and PPh₃ (29 mg, 0.111 mmol). After 1 minute of stirring, diisopropyl azodicarboxylate (22.4 mg, 0.111 mmol) was added. The mixture was stirred at room temperature for 1.5 hours. The solvent was removed at reduced pressure, and the mixture was purified by preparative HPLC (solvent: CH₃OH-H₂O-TFA) to yield Example 5 (17 mg) as a white powder. HPLC Rₜ (Method A): 3.923 min. LCMS: m/z 415 (M + H⁺). HPLC purity: 98%. ¹H NMR: δ 8.95 (d, J = 1.7 Hz, 1H), 8.18-8.16 (m, 1H), 8.07-8.05 (m, 1H), 7.39-7.37 (m, 2H), 7.12-7.08 (m, 1H), 5.29 (s, 2H), 3.84 (d, J = 11.7 Hz, 2H), 2.40-2.34 (m, 2H), 1.75-1.72 (m, 2H), 1.37-1.32 (m, 3H), 0.96 (d, J =5.7 Hz, 3H).

### EXAMPLE 6 (Reference) 5-((2,6-Dichlorophenylthio)methyl)-2-(4-methylpiperidin-1-ylsulfonyl)-pyridine

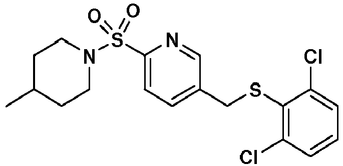

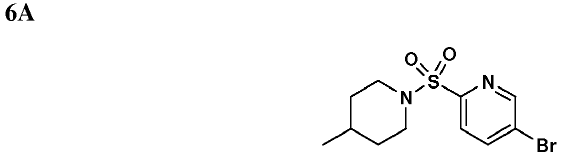

To a solution of 2,5-dibromopyridine (5 g, 21.10 mmol) in toluene (300 mL) at -78°C was added 2.5 N (in hexane) n-BuLi solution (10.1 mL, 25.33 mmol). After the addition, the solution was stirred at -78°C for 2.5 hours. The reaction mixture was added slowly, via a steel cannula, to a saturated SO₂ solution in THF (200 mL) at -78°C. After the addition, the solution was stirred at -78°C° for 20 minutes, then was warmed to RT over 1 hour. The solution was concentrated under reduced pressure to about 100 mL, and was then treated with sulfuryl chloride (2.85 g, 21.10 mmol) at 0°C to RT for 20 minutes. The solution was concentrated under reduced pressure to yield 5-bromopyridine-2-sulfonyl chloride. A portion (3/5) of the crude intermediate was dissolved in DCM (100 mL) and was treated with 4-methylpiperidine (10 g, 101.3 mmol) at room temperature for 20 minutes. The solution was concentrated and purified by column chromatography to yield compound 6A (1.86 g) as a white powder. HPLC Rₜ (Method A): 3.108 min. LCMS: m/z 319 (M + H⁺).

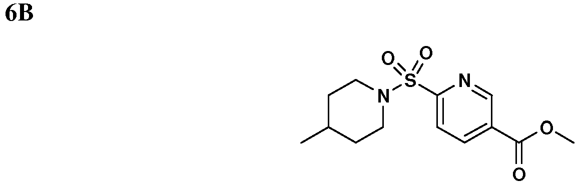

Compound 6B was prepared in a similar manner as compound 1B. Carbonylation of compound 6A (1.10 g) gave compound 6B (960 mg) as a white power. LC/MS m/z 299 (M+H⁺).

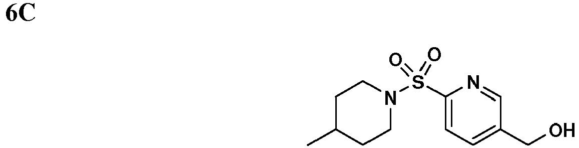

To a solution of compound 6B (801 mg, 2.69 mmol) in EtOH (12 mL) and THF (20 mL) was added NaBH₄ (203 mg, 5.38 mmol). The mixture stirred at RT overnight. The reaction was quenched with water and was neutralized to pH=7 using 1N HCl. The mixture was stripped of the organic solvents, was made slightly basic using 1N NaOH, and was extracted several times with ethyl acetate. The organic extracts were combined, dried over MgSO₄, concentrated, and purified by column chromatography to yield compound 6C (507 mg) as a white powder. HPLC Rₜ (Method A): 2.297 min. LCMS: m/z 271 (M + H⁺).

### Example 6

To a solution of compound 6C (250 mg, 0.925 mmol) in DCM (10 mL) was added thionyl chloride (0.547 mL, 7.40 mmol). The solution was stirred at room temperature for 3.5 hours and was then concentrated to yield a white powder. The powder was dissolved in DCM (10 mL) and was treated with 2,6-dichlorobenzenethiol (166 mg, 0.925 mmol) and N,N-diisopropylethylamine (0.644 mL, 3.7 mmol) at RT for 40 minutes. The solvent was removed under reduced pressure, and the residue was purified by column chromatography to yield Example 6 (385 mg) as a white powder. HPLC Rₜ (Method A): 3.785 min. LCMS: m/z 431 (M + H⁺). HPLC purity: 96%. ¹H NMR: δ 8.43 (s, 1H), 7.77-7.75 (m, 1H), 7.64-7.62 (m, 1H), 7.35-7.33 (m, 2H), 7.22-7.18 (m, 1H), 4.15 (s, 2H), 3.84 (d, J = 12.1 Hz, 2H), 2.61-2.55 (m, 2H), 1.70-1.67 (m, 2H), 1.50-1.26 (m, 3H), 0.96 (d, J = 6.3 Hz, 3H).

### EXAMPLE 7 (Reference) 5-((2,6-Dichlorophenylsulfonyl)methyl)-2-(4-methylpiperidin-1-ylsulfonyl)pyridine

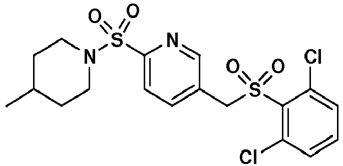

Example 7 was prepared in a similar manner as Example 2. Oxidation of Example 6 (188 mg) gave Example 7 (205 mg) as a white powder. HPLC Rₜ (Method A): 3.030 min. LCMS: m/z 463 (M + H⁺). HPLC purity: 97%. ¹H NMR: δ 8.42 (s, 1H), 7.91-7.72 (m, 2H), 7.48-7.32 (m, 3H), 4.68 (s, 2H), 3.76 (d, J = 11.3 Hz, 2H), 2.52 (t, J = 11.7 Hz, 2H), 1.70-1.49 (m, 2H), 1.40-1.09 (m, 3H), 0.86 (d, J = 6.2 Hz, 3H)

### EXAMPLE 8 (Reference) 5-((2,6-Dichlorophenoxy)methyl)-2-(4-methylpiperidin-1-ylsulfonyl)-pyridine

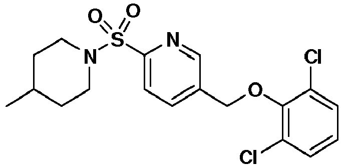

Example 8 was prepared in a similar manner as Example 5. Reaction of compound 6C (32 mg) and other appropriate reagents gave Example 8 (54.9 mg) as a white powder. HPLC Rₜ (Method A): 3.842min. LCMS: m/z 415 (M + H⁺). HPLC purity: 97%. ¹H NMR: δ 8.88 (d, J = 1.6 Hz,1H), 8.16-8.13 (m, 1H), 8.01-7.99 (m, 1H), 7.38-7.36 (m, 2H), 7.09 (t, J = 8.1 Hz , 1H), 5.17 (s, 2H), 3.94 (d, J = 12.2 Hz, 2H), 2.75-2.68 (m, 2H), 1.73-1.69 (m, 2H), 1.50-1.23 (m, 3H), 0.96 (d, J = 6.3 Hz, 3H).

### EXAMPLE 9 (6-(2-Chlorophenoxy)pyridin-2-yl)(4-methylpiperidin-1-yl)methanone

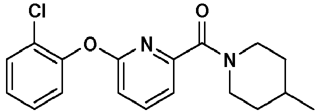

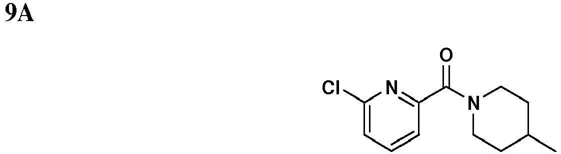

To a solution of 6-chloropyridine-2-carboxylic acid (1.0 g, 6.3 mmol) and 4-methylpiperidine (1.1 mL, 9.5 mmol) in DCM (20 mL) was added EDAC (1.8 g, 9.5 mmol), HOAT (0.5M in DMF, 1.9 mL, 0.95 mmol), and 4-DMAP (116 mg, 0.95 mmol). The solution was stirred at RT for 18hr, and then was concentrated *in vacuo.* The residue was partitioned between EtOAc and Brine. The organic phase was dried (MgSO₄) and concentrated *in vacuo.* The crude product was purified via column chromatography (30%EtOAc/70%Hexane, flow rate: 30 mL/min, detection wavelength: 254 nm) to provide compound 9A (1.3 g, 88% yield) as a white solid. HPLC Rₜ (Method A): 2.91 min. LCMS: m/z 239 (M + H⁺). HPLC purity: 95%.

### Example 9

To a solution of compound 9A (100 mg, 0.42 mmol) in DMF (4 mL) was added 2-chlorophenol (81 mg, 0.63 mmol) and cesium carbonate (409 mg, 1.26 mmol). The reaction mixture was placed on the microwave reactor at 200°C for 40 min and was then partitioned between EtOAc and a 10% LiCl solution. The organic phase was dried (MgSO₄) and concentrated *in vacuo.* The residue was purified via preparative HPLC (Phenomenex LUNA 5u C18 21.1 x 100 mm column; detection at 220 nm; flow rate = 25 mL/min; continuous gradient from 80% A to 100% B over 8 min, where A = 90:10:0.1 H₂O:MeOH:TFA and B = 90:10:0.1 MeOH:H₂O:TFA) to provide Example 9 (44.7 mg, 32% yield) as an oil ¹H NMR (400 MHz, CD₃OD): δ 0.68-0.78 (m, 1H), 0.84 (d, J = 6.6 Hz, 3H), 0.95-1.05 (m,1H), 1.34 (d, J = 13.2 Hz, 1H), 1.50-1.60 (m, 1H), 1.65 (d, J = 13.2 Hz, 1H), 2.65-2.75 (m, 1H), 2.78-2.88 (m,1H), 3.74 (d, J = 13.2 Hz, 1H), 4.45 (d, J = 13.2 Hz, 1H), 7.13-7.51 (m, 6H), 7.93 (d, J = 8.4 Hz, 1H).

### EXAMPLES 10 TO 12

**Examples 10 to 12** in Table 1 were synthesized according to the procedures described in **Example 9** utilizing the appropriate starting materials.

**TABLE 1**

| Example | Structure | Mass [M+H] | HPLC Purity (%) |
|---|---|---|---|
| 10 | 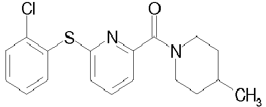 | 347 | 99 |
| 11 | 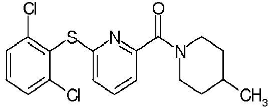 | 381 | 95 |
| 12 | 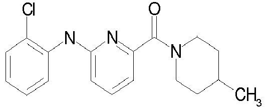 | 330 | 97 |

### EXAMPLE 13

### (6-((2,6-Dichlorophenylthio)methyl)pyridin-2-yl)(4-(trifluoromethyl)piperidin-1-yl)methanone

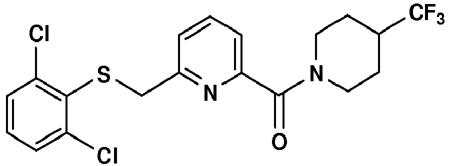

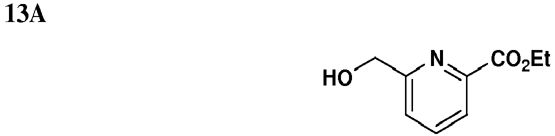

A solution of diethyl 2,6-pyridine dicarboxylate (25 g, 112 mmol) in ethanol (250 mL) was treated with sodium borohydride (2.33 g, 0.55 equiv) and was refluxed for 2 h. After being cooled to RT, the solution was concentrated to a volume of 50 mL and water (50 mL) was added. The solution was further concentrated to a final volume of about 50 mL and extracted with several 50 mL portions of DCM. The combined DCM extracts were dried with sodium sulfate and concentrated by rotary evaporation to yield compound 13A (18.3g of). HPLC purity 95%. LC/MS m/z 182 (M+H⁺).

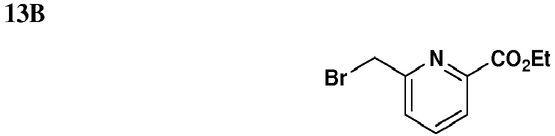

To a solution of compound 13A (2.86 g, 15.74 mmol) in DCM (100 mL) was added phosphorus tribromide (3.20 g, 11.80 mmol) at 0°C. The solution was stirred for 2h at 0°C under nitrogen, then quenched with 100 mL of saturated NaHCO₃ solution. The DCM layer was separated, and the aqueous layer was extracted with DCM (3x100mL). The combined extracts were washed with brine, dried over MgSO₄, and evaporated to yield compound 13B (2.65 g). HPLC purity 93%. LC/MS: m/z 244 (M+H).

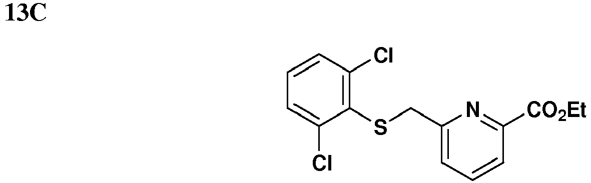

To a solution of compound 13B in THF (10 mL/mmol) was added thiophenol (1 equiv.), DIEA (2 equiv.), and CsCO₃ (1 equiv). The sealed reaction mixture was heated for 2-10 h at 60°C to push the reaction to completion. The reaction was cooled to RT and diluted with hexane. The solid CsCO₃ was removed by filtration, and the THF solvent was removed by rotary evaporation to yield compound 13C. LC/MS: m/z 342 (M+H).

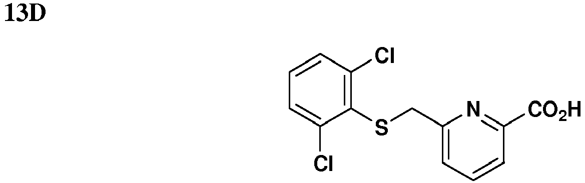

Compound 13C was dissolved in a 1:1 mixture of THF and 1N NaOH solution. The mixture stirred for 2 h at RT. The THF was removed by evaporation, and the mixture was adjusted to pH 3 by the addition of HCl. A white solid precipitated out. The precipitate was filtered and dried to give compound 13D. LC/MS m/z 313 (M+H).

### Example 13

To a solution of compound 13D (0.1 mmol) in DMF (2 mL) was added 4-(trifluoromethyl)piperidine (0.12 mmol), PyAOP (0.1 mmol), and DIEA (0.15 mmol). The reaction was stirred vigorously for 10 h. After the DMF solvent was removed by Speed Vac, the residue was purified by Prep-HPLC to give Example 13. LC/MS m/z 449 (M+H). ¹H NMR (500 MHz, CDCl₃): δ 1.57 (m, 2H), 1.80 (dd, 2H), 2.23 (m, 1H), 2.78 (t, 2H), 4.14 (s, 2H), 4.35 (dd, 2H), 7.09 (m, 2H), 7.25 (d, 2H), 7.43 (d, 1H), 7.58 (t, 1H).

### EXAMPLE 14

### N-Cyclopentyl-5-((2,6-dichlorophenylthio)methyl)nicotinamide

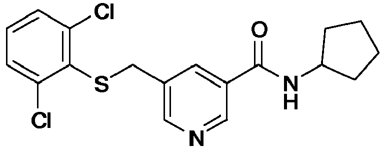

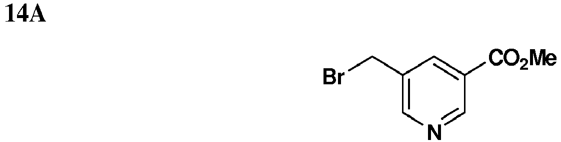

To a solution of methyl 5-methylnicotinate (5 g, 33 mmol) in carbon tetrachloride (200 mL) was added NBS (5.9 g, 1 equiv) and dibenzoyl peroxide (1.2 g, 0.15 equiv). The reaction was refluxed for 3 h, then was cooled to RT to give compound 14A. The carbon tetrachloride solution containing compound 14A was used without further purification.

### Example 14

Example 14 was prepared in three steps in a similar manner as compounds 13C to Example 13: Alkylation of compound 14A with 2,6-dichlorothiophenol, basic hydrolysis of the methyl ester, followed by amide formation provided Example 14. LC/MS m/z 381 (M+H⁺) ¹H NMR (500 MHz, CDCl₃): δ 1.47 (m, 2H), 1.72 (m, 6H), 2.08 (m, 2H), 4.11 (s, 2H), 4.36 (q, 1H), 5.93 (bs, 1H), 7.14 (t, 1H), 7.31 (d, 2H), 7.84 (s, 1H), 8.41 (s, 1H), 8.75 (s, 1H).

### EXAMPLE 15

### (4-Methylpiperidin-1-yl)(5-(m-tolylthiomethyl)pyridin-3-yl)methanone

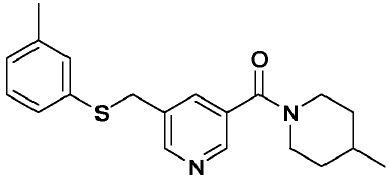

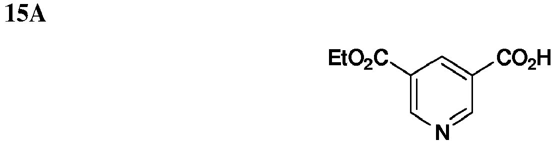

To a stirred solution of pyridine-3,5-dicarboxylic acid (25 g) in EtOH (200 mL) was added concentrated H₂SO₄ (5 mL). The reaction was stirred until all pyridine-3,5-dicarboxylic acid was gone. The reaction formed a 1:1 mixture of compound 15A and diethyl pyridine-3,5-dicarboxylate. EtOH was removed via vacuum, and the residue was dissolved in saturated NaHCO₃ solution (100 mL). Diethyl pyridine-3,5-dicarboxylate was extracted out by EtOAc (3X). The aqueous layer was adjusted to pH 3, and the product was precipitated out as a white solid. The solid was filtered and dried to give compound 15A (ca 50%). LC/MS m/z 196 (M+H⁺).

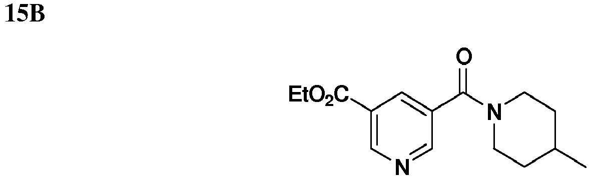

To a stirred solution of compound 15A (4.31 g) in anhydrous THF (150 mL) was added NMM (4.84 mL, 2 equiv) and isobutyl chloroformate (3.17 mL, 1.1 equiv) at 0 °C. The reaction was stirred for 1 h at 0°C, followed by addition of 4-methylpiperidine (5.2 mL, 2 equiv). The stirring was continued to for another 10 h. The white precipitated solid was filtered off, and the solvent was removed by evaporation. The crude product was purified by silica gel column chromatography (ISCO) to give compound 15B (3.56 g). LC/MS m/z 276 (M+H⁺).

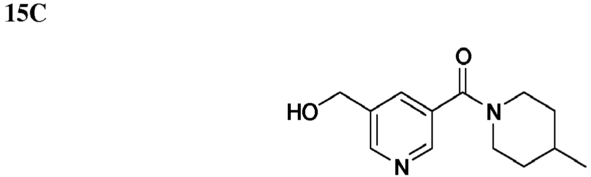

Compound 15C was prepared in a similar manner as compound 1C. Sodium borohydride reduction of compound 15B (3.56 g) gave compound 15C (2.5 g). LC/MS m/z 235 (M+H⁺). ¹H NMR (CDCl₃): δ 0.96 (d, 3H), 1.15 (m, 2H), 1.70 (m, 3H), 2.76 (t, 1H), 3.01 (t, 1H), 3.60 (d, 1H), 4.60 (d, 1H), 4.66 (s, 2H), 7.67 (s, 1H), 8.45 (s, 1H), 8.49 (s, 1H). ¹³C NMR (CDCl₃): δ 21.55, 30.95, 33.62, 34.63, 42.66, 48.18, 61.77, 131.86, 133.27, 137.09, 146.01, 148.90, 167.65.

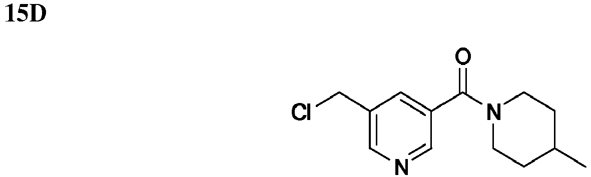

To a stirred solution of compound 15C (2.5 g, 10.6 mmol) in DCM (100 mL) was added SOCl₂ (3.9 mL, 5 equiv). The mixture stirred for 1 h at RT. DCM solvent was removed by evaporation, and a white solid was obtained as compound 15D (3.2g). LC/MS m/z 253 (M+H⁺).

### Example 15

Example 15 was prepared in a similar manner as Example 1: alkylation of compound 15D with 3-methylthiophenoyl provided Example 15. HPLC purity 99%. LC/MS: m/z 341 (M+H⁺). ¹H NMR (400 MHz, DMSO/CDCl₃): δ 0.95 (d, 3H), 1.42-1.80(m, 3H), 2.66-2.83 (m, 1H), 2.86-3.06 (m, 1H), 3.25-3.60 (m, 2H), 3.73 (s, 3H), 4.29 (s, 2H), 4.36-4.55 (m, 1H), 6.75 (d, 1H), 6.83-6.92 (m, 2H), 7.18 (t, 1H), 7.69 (s, 1H), 8.41 (s, 1H), 8.57 (s, 1H).

### EXAMPLE 16

### (2,5-Dimethylpyrrolidin-1-yl)(6-((naphthalen-1-ylsulfonyl)methyl)pyridin-2-yl)methanone

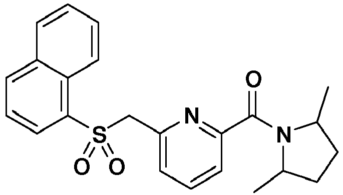

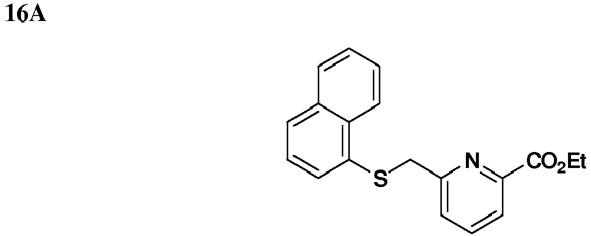

Compound 16A was prepared in a similar manner as compound 13C using appropriate starting materials. LC/MS: m/z 324 (M+H⁺).

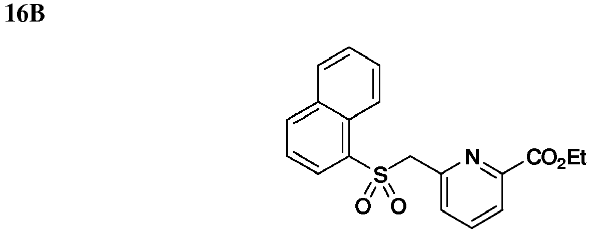

To a solution of compound 16A (1 mmol) in DCM (10 mL) was added mCPBA (4 equiv.). The mixture was stirred at RT overnight. The reaction mixture was then cooled to 0°C, followed by addition of PBr₃ (4 equiv.). The stirring was continued for 6 h at 0°C, and the reaction was then quenched with saturated NaHCO₃ solution. The DCM layer was separated, and the aqueous layer was extracted with DCM (3x100mL). The combined DCM extracts were washed with brine, dried over MgSO₄, and evaporated to give compound 16B. LC/MS: m/z 356 (M+H⁺).

### Example 16

Example 16 was prepared in two steps in a similar manner as compounds 13D to Example 13: basic hydrolysis of compound 16B, followed by amide formation provided Example 16. LC/MS: m/z 409 (M+H⁺). ¹H NMR (400 MHz, DMSO/CDCl₃): δ 0.80 (d, 3H), 1.12 (d, 3H), 0.95-4.08 (m, 6H), 5.0 (m, 2H), 7.10-7.95 (m, 7H), 8.20 (d, 1H), 8.32 (t, 1H), 8.68 (d, 1H).

### EXAMPLE 17

### (4-Methylpiperidin-1-yl)(5-(o-tolyloxymethyl)pyridin-3-yl)methanone

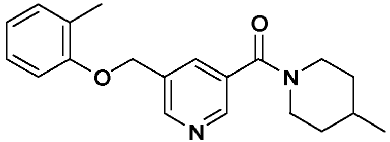

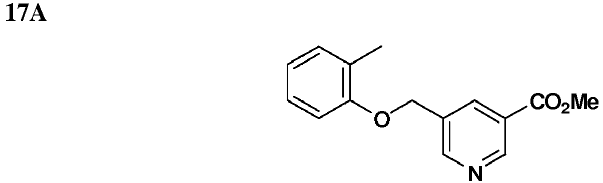

To a solution of compound 14A (ca. 1 mmol) in CCl₄ (6 mL) was added 2-methylphenol (1 equiv.) and DIEA (2 equiv). The reaction was refluxed for 1 h and then cooled to RT. The crude product was purified by silica gel column chromatography (ISCO) to give compound 17A. LC/MS: m/z 258 (M+H⁺).

### Example 17

Example 17 was prepared in two steps in a similar manner as compounds 13C to Example 13: basic hydrolysis of compound 17A, followed by amide formation provided Example 17. LC/MS: m/z 325 (M+H⁺). ¹H NMR (400 MHz, DMSO/CDCl₃): δ 1.00 (d, 3H), 2.25 (s, 3H), 1.18-4.50 (m, 9H), 5.26 (s, 2H), 6.90 (t, 1H), 7.04 (d, 1H), 7.20 (m, 2H), 7.90 (s, 1H), 8.59 (s, 1H), 8.78 (s, 1H).

### EXAMPLE 18

### (6-(2-Chlorophenyl)pyridin-2-yl)(4-methylpiperidin-1-yl)methanone

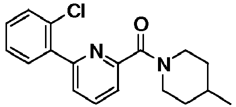

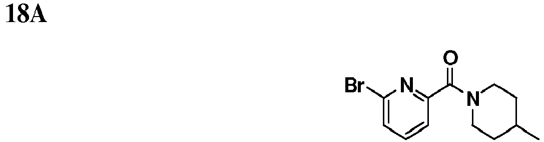

A solution of 6-bromopicolinic acid (250 mg, 1.24 mmol) in thionyl chloride (1.7 mL) was refluxed for 1.0 h, cooled, concentrated, and dried *in vacuo* for 1.0 h. The crude product was dissolved in dry DCM (15 mL), was treated with 4-methylpiperidine (96%, 0.3 mL, 2.29 mmol), and was stirred at room temperature for 20 h. The reaction mixture was concentrated and dried *in vacuo.* The solids obtained were chromatographed (ISCO, 40 g. column; CH₃OH:CH₂Cl₂ gradient- 0% to 10%) to yield compound 18A (332.9 mg, 94.8%) as a white solid (m.p. 90-92°C). HPLC: 96.6% at 1.97 and 2.07 min (retention times for rotamer mixture) (Conditions: YMC S-5 C-18 (4.6 x 50 mm), eluting with 0-100% B, 4 min gradient. (A= 90% H₂O - 10% CH₃CN - 0.1 % TFA and B= 10% H₂O - 90% CH₃CN - 0.1% TFA); Flow rate at 4 mL/min. UV detection at 220 nm. MS (ES⁺): m/z 283 [M+H]⁺.

### Example 18

A solution of compound 18A (100 mg, 0.35 mmol) in dry toluene (0.8 mL) was treated with tetrakis(triphenylphosphine)palladium(0) (14.3 mg, 0.012 mmol). The mixture stirred at room temperature for 15 min and was then treated with 2-chlorophenyl-boronic acid (70.4 mg, 0.45 mmol), 2.0 M Na₂CO₃ (0.4 mL) and absolute ethanol (0.4 mL). The reaction mixture was stirred at 80°C (oil bath) for 25 h, was cooled, and then was partitioned between H₂O (1.5 mL) and EtOAc (3 x 15 mL). The combined organic extracts were washed with brine (1.5 mL), dried over MgSO₄, filtered, and concentrated under pressure. The crude product was chromatographed (ISCO, 40 g silica gel column; EtOAc:Hexane- 0% to 50% gradient), followed by purification via preparative HPLC (YMC S5 ODS 20 x 100 mm; CH₃CN/H₂O + 0.1% TFA- 0% to 100%) to yield Example 18 as a white solid (73.6 mg, 49%). HPLC: 98% purity at 2.10 min (retention time) (Conditions: YMC S-5 C-18 (4.6 x 50 mm), eluting with 0-100% B, 4 min gradient. (A= 90% H₂O - 10% CH₃CN - 0.1% TFA and B= 10% H₂O - 90% CH₃CN - 0.1% TFA); Flow rate at 4 mL/min. UV detection at 220 nm. MS (ES⁺): m/z 315 [M+H]⁺. ¹H NMR (500 MHz, CD₃OD): δ 0.98 (d, J = 6.6 Hz, 3H), 1.20-1.27 (m, 2H), 1.62-1.80 (m, 3H), 2.84-2.88 (m, 1H), 3.09-3.13 (m, 1H), 3.81 (d, J = 13.2 Hz, 1H), 4.62 (d, J = 13.2 Hz, 1H), 7.40-7.45 (m, 2H), 7.52-7.57 (m, 3H), 7.71 (d, J = 8.8 Hz, 1H), 8.02 (t, J = 7.7 Hz, 1H).

### EXAMPLE 19

### (6-(2-Chlorophenyl)pyridin-2-yl)(3,4-dihydroquinolin-1(2H)-yl)methanone

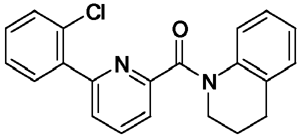

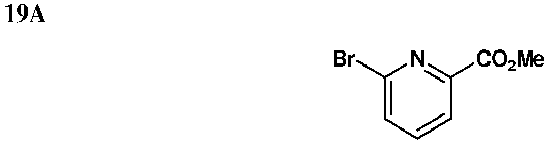

To a solution of 6-bromopicolinic acid (2.5 g) in MeOH (100 mL) was added concentrated H₂SO₄ (5 mL). The reaction was refluxed until the 6-bromopicolinic acid was gone. The mixture was dried by evaporation and then purified by silica gel column chromatography (ISCO) to give compound 19A (ca 90% yield). LC/MS: m/z 216/218 (M+H⁺).

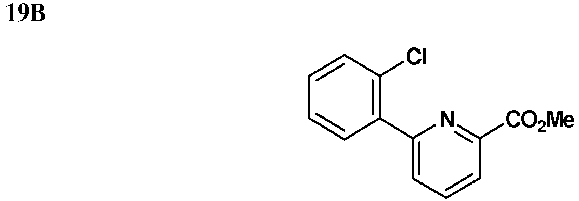

To a solution of compound 19A (300 mg) in DMA (10 mL) was added K₃PO₄ (3 equiv). Nitrogen was bubbled through the solution, and then catalyst Pd(PPh₃)₄ (0.1 equiv) was added. The mixture was placed in a sealed microwave tube, which was put on the Microwave for 30 min. at 120°C. The extra solid residues were filtered off and DMA solvent was removed by Speed-Vac. The crude product was purified by silica gel column chromatography to give compound 19B (ca 60%). LC/MS: m/z 248 (M+H⁺).

### Example 19

Example 19 was prepared in two steps in a similar manner as compounds 13D to Example 13: basic hydrolysis of compound 19B, followed by amide formation provided Example 19. LC/MS: m/z 349 (M+H⁺). ¹H NMR (400 MHz, DMSO/CDCl₃): δ 2.05 (t, 2H), 2.86 (m, 2H), 3.86 (t, 2H), 7.00 (m, 1H), 7.05 (m, 2H), 7.24 (t, 2H), 7.37 (t, 1H), 7.42 (t, 1H), 7.52 (d, 1H), 7.64 (d, 1H), 7.73 (d, 1H), 8.03 (t, 1H).

### EXAMPLES 20 TO 305

**Examples 20 to 305** in Table 2 were prepared according to the procedures described in the proceeding examples, or by other similar methods used by one skilled in the art, utilizing other appropriate reagents.

**TABLE 2**

| Example | Structure | Mass [M+H] | HPLC Purity (%) |
|---|---|---|---|
| 20 | 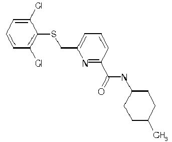 | 409.37 | 100 |
| 21 | 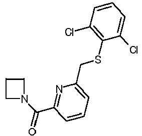 | 353.32 | 100 |
| 22 | 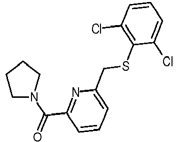 | 367.35 | 100 |
| 23 | 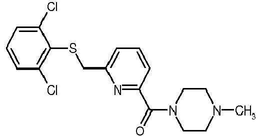 | 396.35 | 100 |
| 24 | 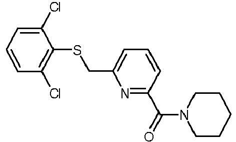 | 381.35 | 100 |
| 25 | 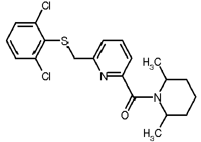 | 409.37 | 100 |
| 26 | 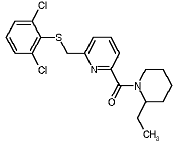 | 409.37 | 99 |
| 27 | 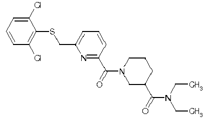 | 480.4 | 100 |
| 28 | 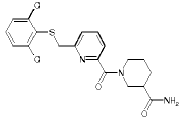 | 424.37 | 100 |
| 29 | 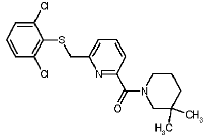 | 409.37 | 100 |
| 30 | 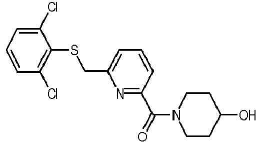 | 397.35 | 100 |
| 31 | 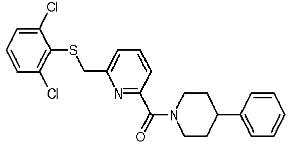 | 457.34 | 100 |
| 32 | 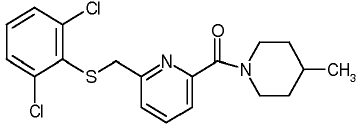 | 395.4 | 100 |
| 33 | 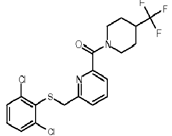 | 449.32 | 100 |
| 34 | 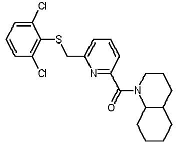 | 435.42 | 100 |
| 35 | 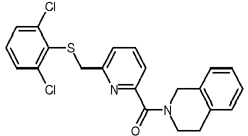 | 429.34 | 94 |
| 36 | 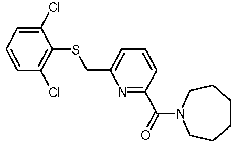 | 395.4 | 100 |
| 37 | 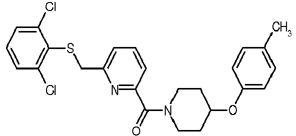 | 487.37 | 97 |
| 38 | 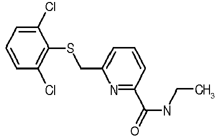 | 341.36 | 100 |
| 39 | 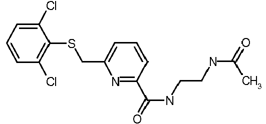 | 398.38 | 100 |
| 40 | 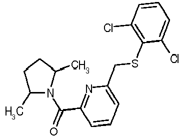 | 395.4 | 97 |
| 41 | 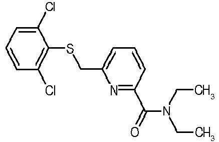 | 367.35 | 100 |
| 42 | 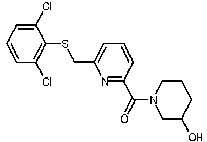 | 397.35 | 82 |
| 43 | 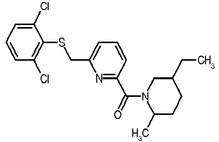 | 423.42 | 98 |
| 44 | 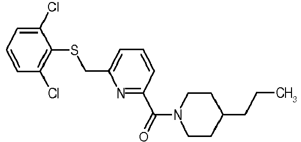 | 423.41 | 100 |
| 45 | 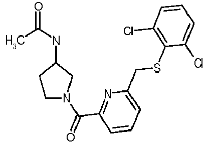 | 424.37 | 100 |
| 46 | 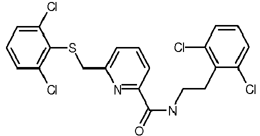 | 485.25 | 100 |
| 47 | 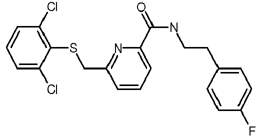 | 435.36 | 100 |
| 48 | 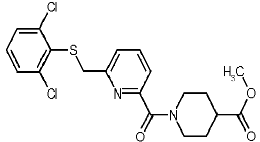 | 439.36 | 94 |
| 49 | 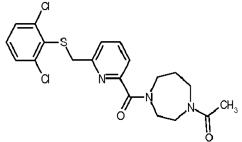 | 438.39 | 81 |
| 50 | 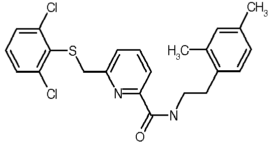 | 445.38 | 100 |
| 51 | 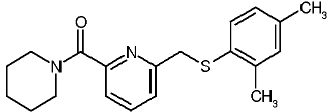 | 341.22 | 88 |
| 52 | 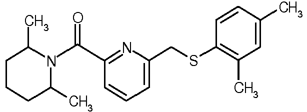 | 369.23 | 95 |
| 53 | 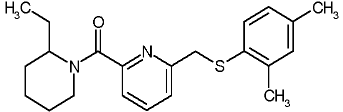 | 369.24 | 89 |
| 54 | 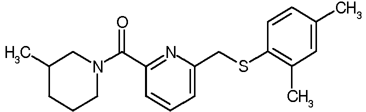 | 355.19 | 85 |
| 55 | 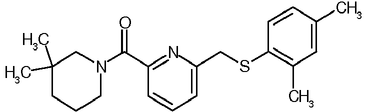 | 369.23 | 84 |
| 56 | 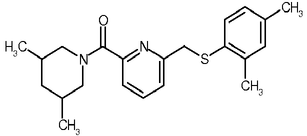 | 369.23 | 87 |
| 57 | 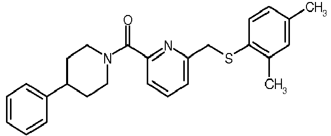 | 417.21 | 92 |
| 58 | 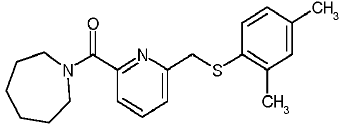 | 355.21 | 97 |
| 59 | 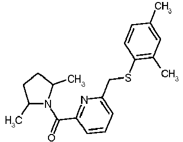 | 355.22 | 88 |
| 60 | 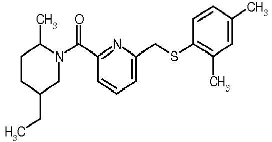 | 383.23 | 100 |
| 61 | 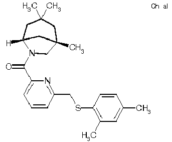 | 409.25 | 100 |
| 62 | 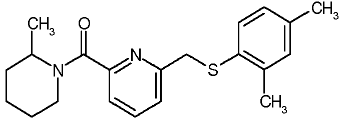 | 355.21 | 86 |
| 63 | 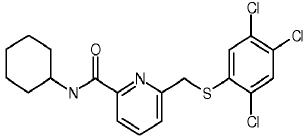 | 429.04 | 100 |
| 64 | 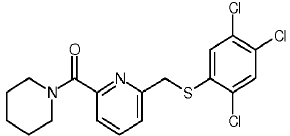 | 415.04 | 88 |
| 65 | 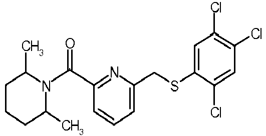 | 443.06 | 98 |
| 66 | 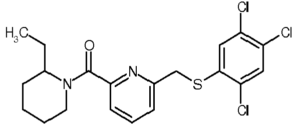 | 443.06 | 100 |
| 67 | 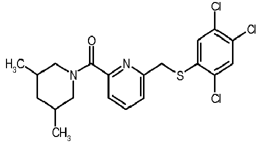 | 443.07 | 100 |
| 68 | 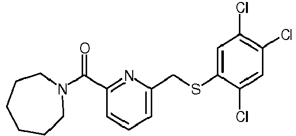 | 431.05 | 100 |
| 69 | 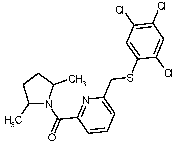 | 429.05 | 100 |
| 70 | 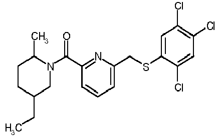 | 457.07 | 96 |
| 71 | 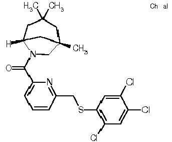 | 429.05 | 100 |
| 72 | 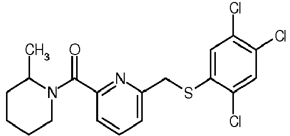 | 429.05 | 100 |
| 73 | 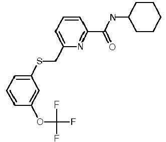 | 411.16 | 100 |
| 74 | 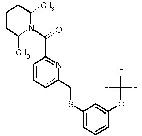 | 425.16 | 100 |
| 75 | 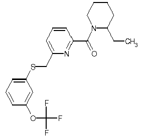 | 425.18 | 100 |
| 76 | 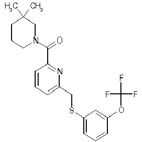 | 425.15 | 100 |
| 77 | 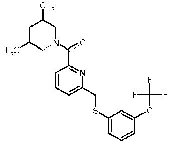 | 425.17 | 100 |
| 78 | 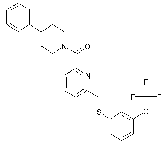 | 473.16 | 88 |
| 79 | 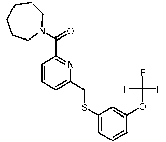 | 411.15 | 100 |
| 80 | 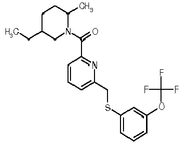 | 439.19 | 100 |
| 81 | 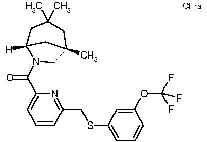 | 465.19 | 100 |
| 82 | 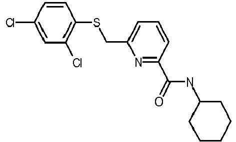 | 395.1 | 85 |
| 83 | 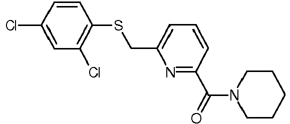 | 381.09 | 93 |
| 84 | 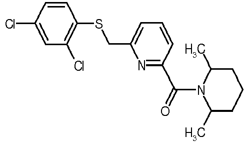 | 409.1 | 82 |
| 85 | 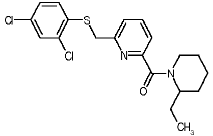 | 409.11 | 85 |
| 86 | 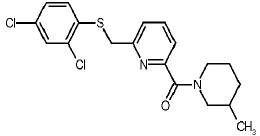 | 395.12 | 90 |
| 87 | 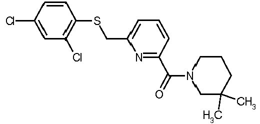 | 409.1 | 87 |
| 88 | 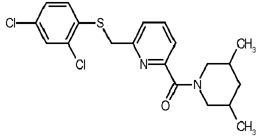 | 409.1 | 98 |
| 89 | 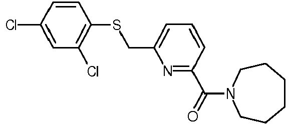 | 395.09 | 86 |
| 90 | 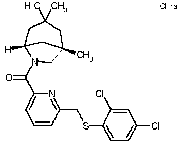 | 449.12 | 90 |
| 91 | 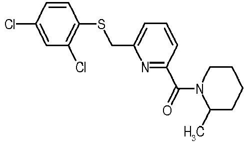 | 395.1 | 83 |
| 92 | 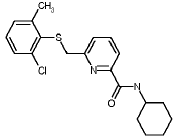 | 375.15 | 100 |
| 93 | 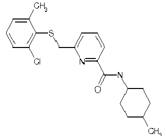 | 389.15 | 99 |
| 94 | 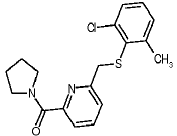 | 347.14 | 95 |
| 95 | 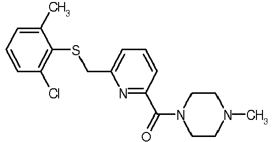 | 376.12 | 98 |
| 96 | 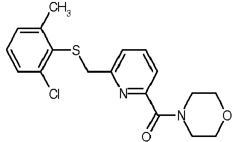 | 363.13 | 96 |
| 97 | 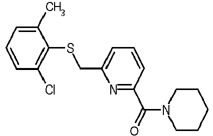 | 361.15 | 96 |
| 98 | 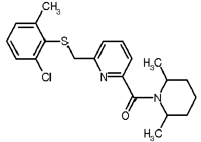 | 389.18 | 85 |
| 99 | 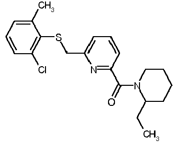 | 389.17 | 99 |
| 100 | 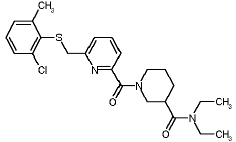 | 460.19 | 100 |
| 101 | 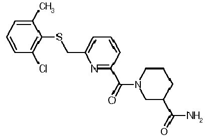 | 404.15 | 97 |
| 102 | 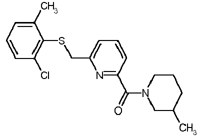 | 375.15 | 91 |
| 103 | 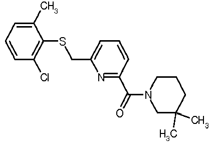 | 389.17 | 99 |
| 104 | 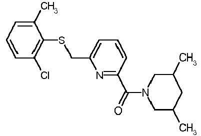 | 389.15 | 93 |
| 105 | 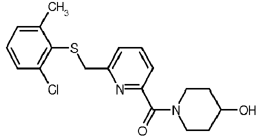 | 377.14 | 91 |
| 106 | 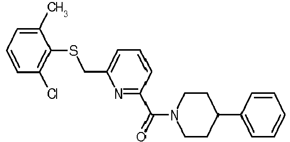 | 437.17 | 94 |
| 107 | 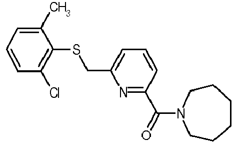 | 375.17 | 99 |
| 108 | 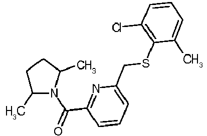 | 375.16 | 95 |
| 109 | 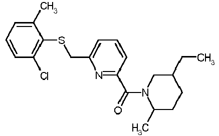 | 403.17 | 81 |
| 110 | 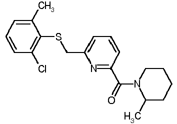 | 375.15 | 100 |
| 111 | 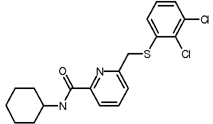 | 395.1 | 85 |
| 112 | 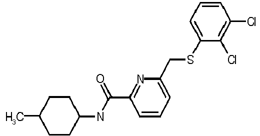 | 409.1 | 100 |
| 113 | 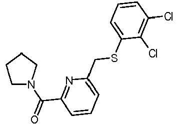 | 367.07 | 97 |
| 114 | 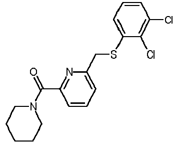 | 381.07 | 95 |
| 115 | 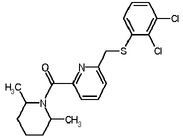 | 409.09 | 100 |
| 116 | 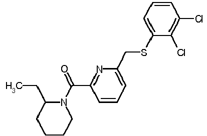 | 409.1 | 100 |
| 117 | 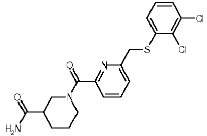 | 424.07 | 95 |
| 118 | 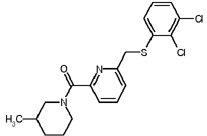 | 395.07 | 100 |
| 119 | 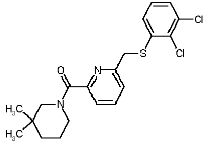 | 409.08 | 99 |
| 120 | 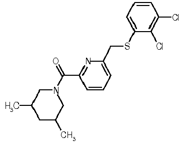 | 409.1 | 100 |
| 121 | 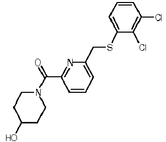 | 397.07 | 85 |
| 122 | 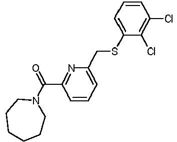 | 395.1 | 100 |
| 123 | 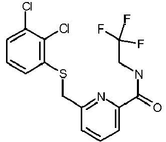 | 395.01 | 100 |
| 124 | 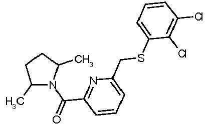 | 395.09 | 100 |
| 125 | 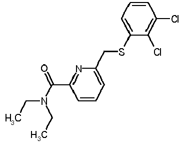 | 369.08 | 81 |
| 126 | 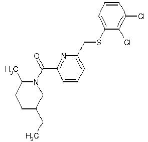 | 423.11 | 100 |
| 127 | 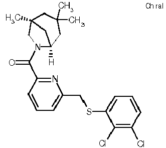 | 449.13 | 96 |
| 128 | 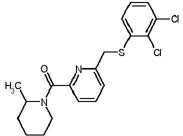 | 395.09 | 100 |
| 129 | 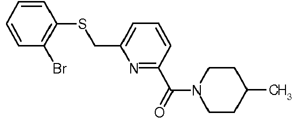 | 405.2 | 100 |
| 130 | 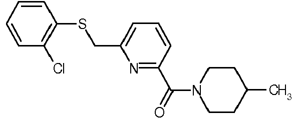 | 361.28 | 100 |
| 131 | 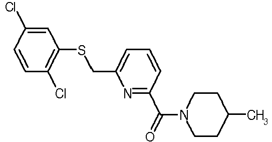 | 395.23 | 100 |
| 132 | 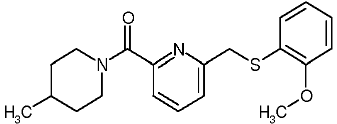 | 357.32 | 94 |
| 133 | 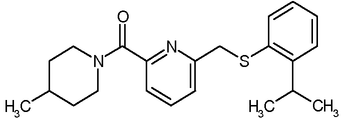 | 369.35 | 98 |
| 134 | 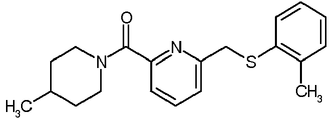 | 341.35 | 100 |
| 135 | 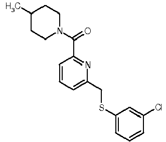 | 361.3 | 100 |
| 136 | 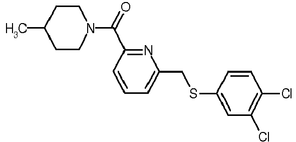 | 395.24 | 97 |
| 137 | 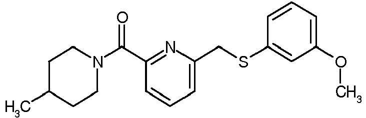 | 357.34 | 100 |
| 138 | 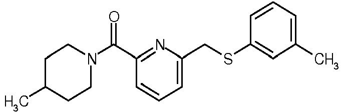 | 341.35 | 100 |
| 139 | 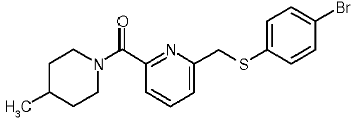 | 405.2 | 98 |
| 140 | 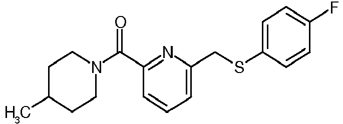 | 345.33 | 100 |
| 141 | 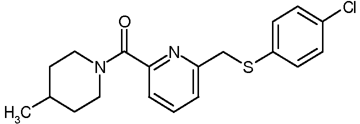 | 361.28 | 98 |
| 142 | 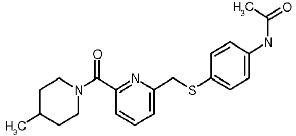 | 384.33 | 98 |
| 143 | 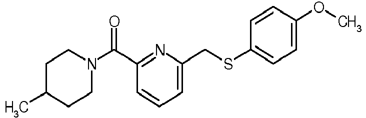 | 357.35 | 100 |
| 144 | 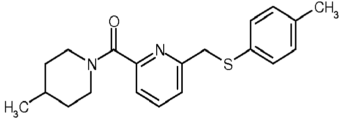 | 341.35 | 100 |
| 145 | 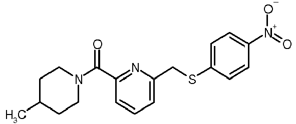 | 372.31 | 100 |
| 146 | 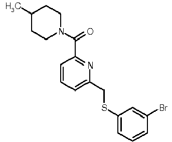 | 405.2 | 100 |
| 147 | 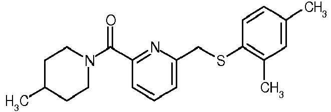 | 355.38 | 97 |
| 148 | 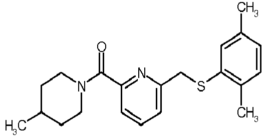 | 355.38 | 100 |
| 149 | 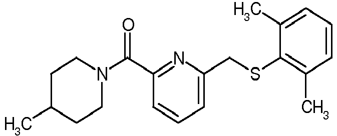 | 355.38 | 100 |
| 150 | 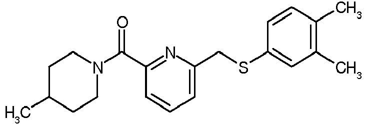 | 355.38 | 96 |
| 151 | 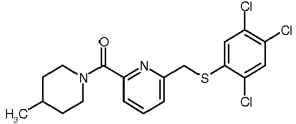 | 429.17 | 100 |
| 152 | 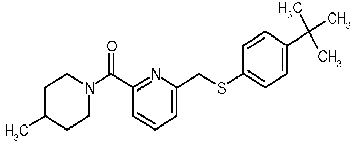 | 383.41 | 96 |
| 153 | 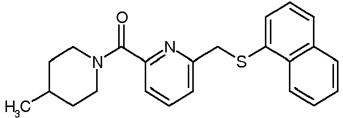 | 377.35 | 92 |
| 154 | 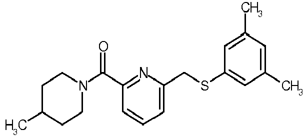 | 355.38 | 84 |
| 155 | 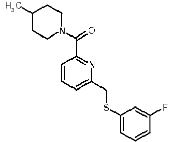 | 345.35 | 100 |
| 156 | 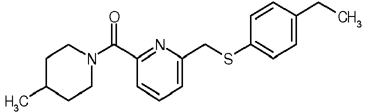 | 355.38 | 100 |
| 157 | 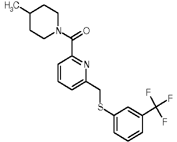 | 395.32 | 95 |
| 158 | 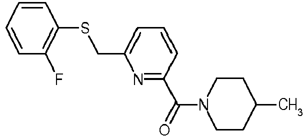 | 345.35 | 100 |
| 159 | 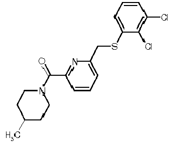 | 395.25 | 94 |
| 160 | 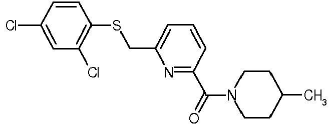 | 395.25 | 100 |
| 161 | 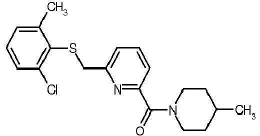 | 375.32 | 100 |
| 162 | 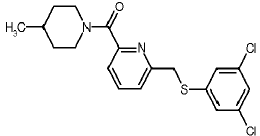 | 395.25 | 88 |
| 163 | 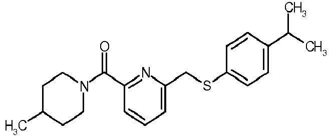 | 369.4 | 98 |
| 164 | 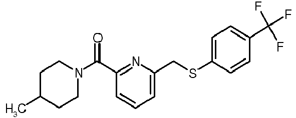 | 395.32 | 92 |
| 165 | 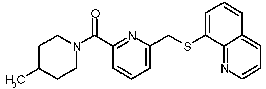 | 378.33 | 100 |
| 166 | 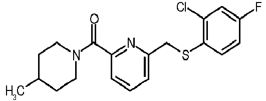 | 379.29 | 100 |
| 167 | 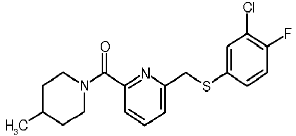 | 379.29 | 100 |
| 168 | 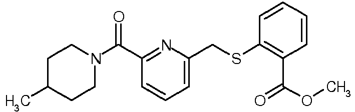 | 385.32 | 100 |
| 169 | 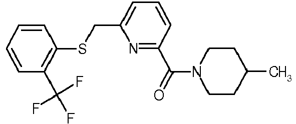 | 395.32 | 100 |
| 170 | 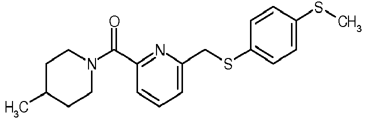 | 373.33 | 100 |
| 171 | 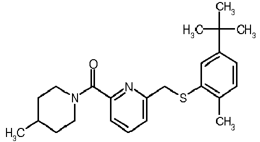 | 397.42 | 92 |
| 172 | 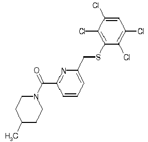 | 463.09 | 97 |
| 173 | 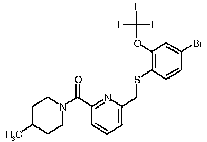 | 489.14 | 100 |
| 174 | 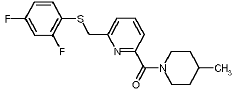 | 363.33 | 100 |
| 175 | 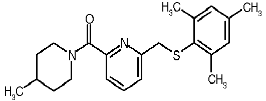 | 369.4 | 94 |
| 176 | 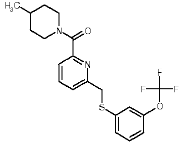 | 411.3 | 100 |
| 177 | 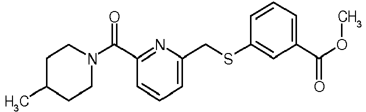 | 385.35 | 100 |
| 178 | 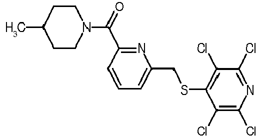 | 464.1 | 96 |
| 179 | 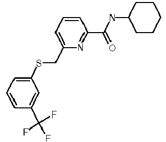 | 395.2 | 97 |
| 180 | 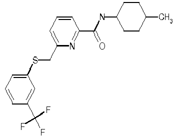 | 409.21 | 97 |
| 181 | 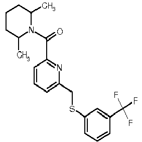 | 409.2 | 93 |
| 182 | 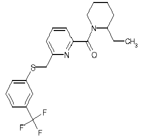 | 409.19 | 100 |
| 183 | 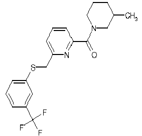 | 395.2 | 92 |
| 184 | 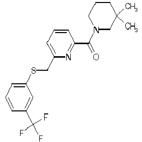 | 409.21 | 89 |
| 185 | 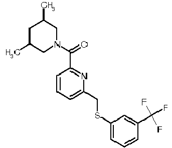 | 409.21 | 90 |
| 186 | 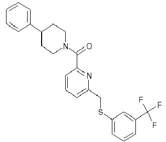 | 457.21 | 89 |
| 187 | 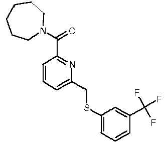 | 395.18 | 93 |
| 188 | 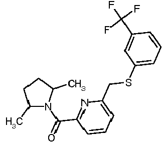 | 395.19 | 94 |
| 189 | 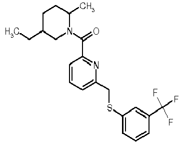 | 423.22 | 98 |
| 190 | 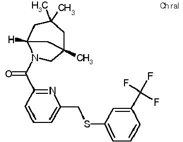 | 449.22 | 97 |
| 191 | 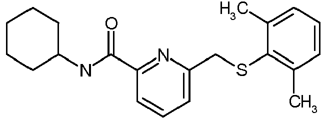 | 355.23 | 98 |
| 192 | 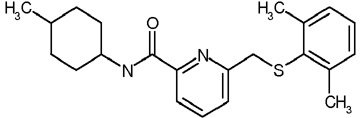 | 369.25 | 99 |
| 193 | 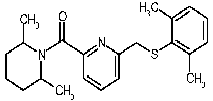 | 369.25 | 93 |
| 194 | 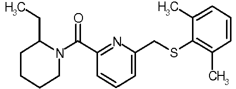 | 369.24 | 92 |
| 195 | 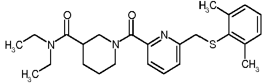 | 440.28 | 96 |
| 196 | 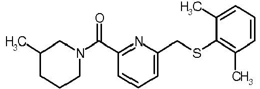 | 355.24 | 95 |
| 197 | 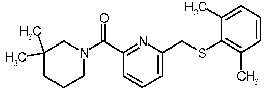 | 369.25 | 92 |
| 198 | 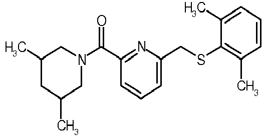 | 369.24 | 100 |
| 199 | 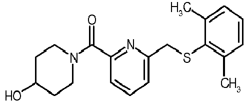 | 357.22 | 94 |
| 200 | 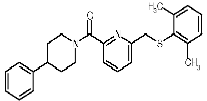 | 417.25 | 94 |
| 201 | 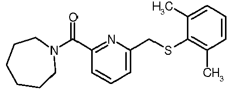 | 355.24 | 94 |
| 202 | 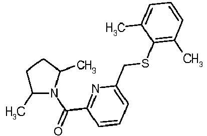 | 355.25 | 92 |
| 203 | 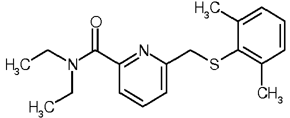 | 329.25 | 100 |
| 204 | 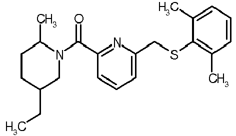 | 383.27 | 100 |
| 205 | 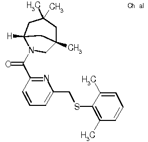 | 409.28 | 100 |
| 206 | 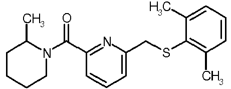 | 355.22 | 100 |
| 207 | 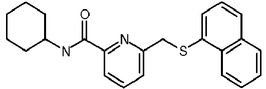 | 377.22 | 94 |
| 208 | 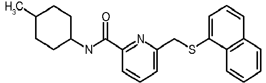 | 391.21 | 96 |
| 209 | 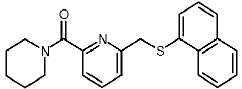 | 363.2 | 91 |
| 210 | 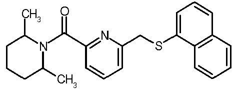 | 391.24 | 90 |
| 211 | 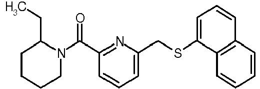 | 391.22 | 92 |
| 212 | 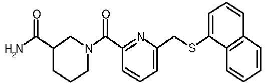 | 406.2 | 93 |
| 213 | 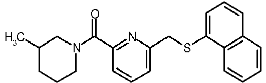 | 377.19 | 87 |
| 214 | 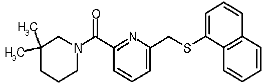 | 391.24 | 94 |
| 215 | 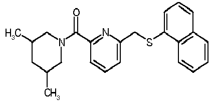 | 391.24 | 92 |
| 216 | 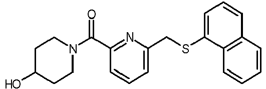 | 379.19 | 98 |
| 217 | 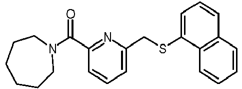 | 377.22 | 90 |
| 218 | 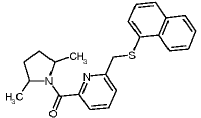 | 377.21 | 89 |
| 219 | 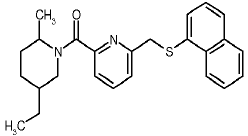 | 405.24 | 94 |
| 220 | 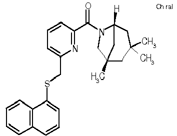 | 431.26 | 93 |
| 221 | 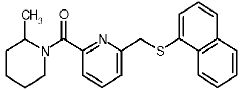 | 377.2 | 87 |
| 222 | 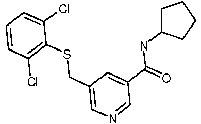 | 381.1 | 100 |
| 223 | 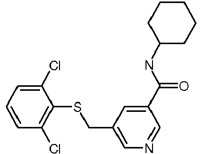 | 395.11 | 100 |
| 224 | 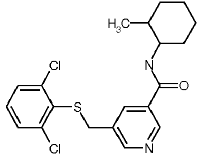 | 409.13 | 100 |
| 225 | 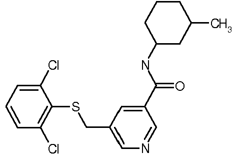 | 409.13 | 100 |
| 226 | 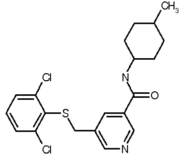 | 409.13 | 100 |
| 227 | 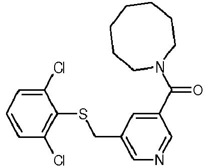 | 409.14 | 100 |
| 228 | 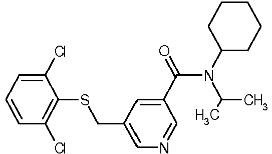 | 437.17 | 100 |
| 229 | 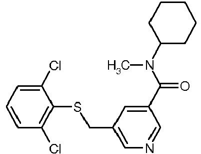 | 409.14 | 100 |
| 230 | 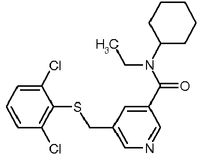 | 423.15 | 100 |
| 231 | 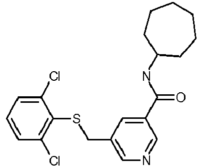 | 409.11 | 100 |
| 232 | 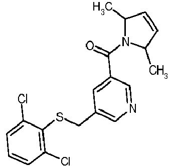 | 468.12 | 90 |
| 233 | 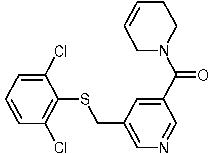 | 379.08 | 95 |
| 234 | 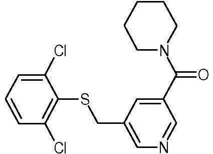 | 381.11 | 100 |
| 235 | 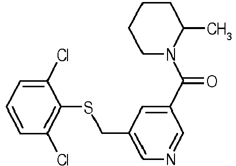 | 395.12 | 100 |
| 236 | 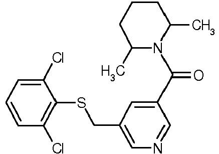 | 409.12 | 100 |
| 237 | 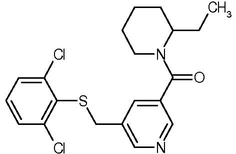 | 409.11 | 100 |
| 238 | 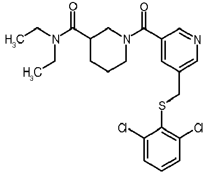 | 480.13 | 100 |
| 239 | 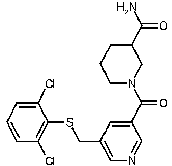 | 424.09 | 100 |
| 240 | 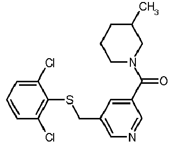 | 395.11 | 100 |
| 241 | 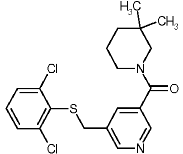 | 409.12 | 100 |
| 242 | 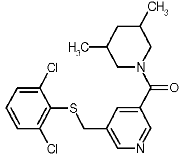 | 409.13 | 100 |
| 243 | 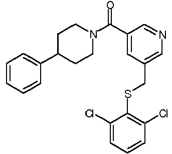 | 457.13 | 100 |
| 244 | 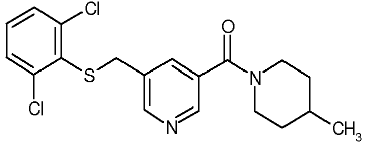 | 395.13 | 100 |
| 245 | 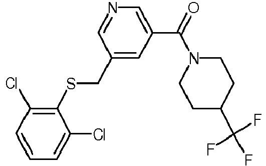 | 449.09 | 100 |
| 246 | 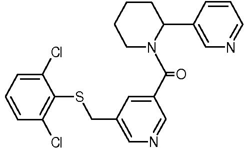 | 458.14 | 100 |
| 247 | 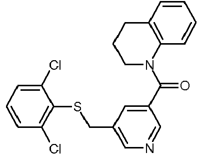 | 429.1 | 100 |
| 248 | 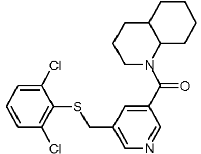 | 435.15 | 92 |
| 249 | 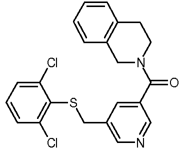 | 429.09 | 100 |
| 250 | 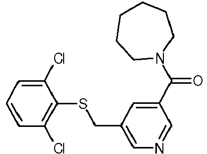 | 395.12 | 100 |
| 251 | 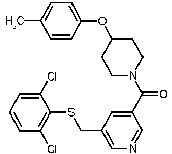 | 487.14 | 92 |
| 252 | 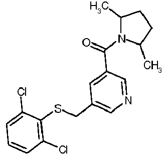 | 395.11 | 100 |
| 253 | 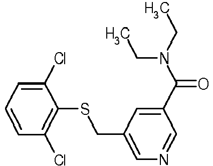 | 369.12 | 81 |
| 254 | 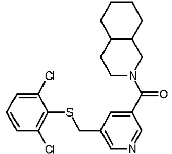 | 435.16 | 93 |
| 255 | 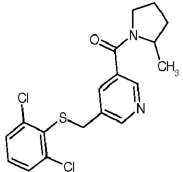 | 381.1 | 100 |
| 256 | 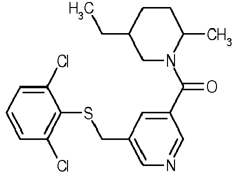 | 423.14 | 100 |
| 257 | 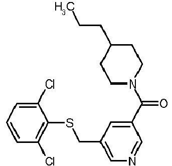 | 423.13 | 99 |
| 258 | 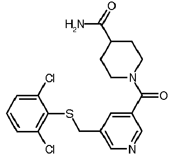 | 424.08 | 100 |
| 259 | 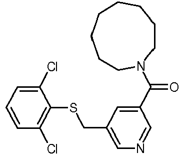 | 423.13 | 100 |
| 260 | 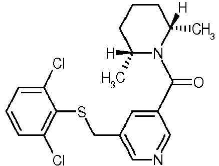 | 409.11 | 96 |
| 261 | 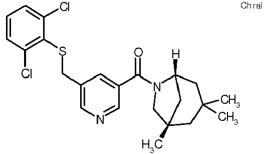 | 449.15 | 100 |
| 262 | 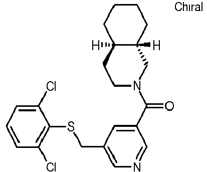 | 435.13 | 99 |
| 263 | 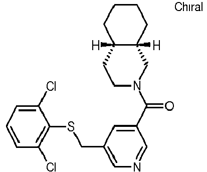 | 435.15 | 90 |
| 264 | 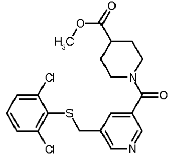 | 439.1 | 90 |
| 265 | 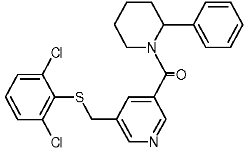 | 457.13 | 100 |
| 266 | 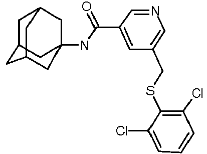 | 447.14 | 100 |
| 267 | 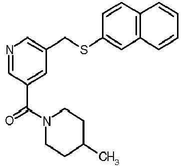 | 377.32 | 100 |
| 268 | 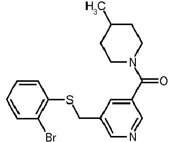 | 405.17 | 100 |
| 269 | 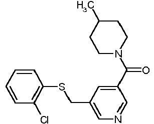 | 361.21 | 100 |
| 270 | 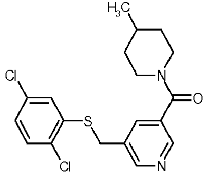 | 395.19 | 100 |
| 271 | 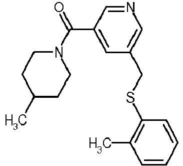 | 362.25 | 99 |
| 272 | 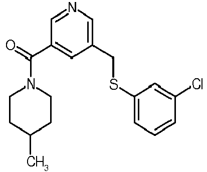 | 361.28 | 99 |
| 273 | 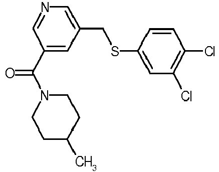 | 421.2 | 99 |
| 274 | 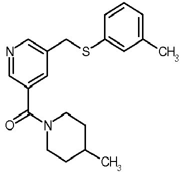 | 341.32 | 100 |
| 275 | 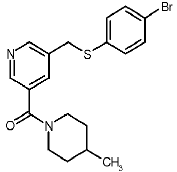 | 409.25 | 95 |
| 276 | 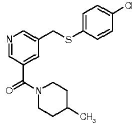 | 363.34 | 99 |
| 277 | 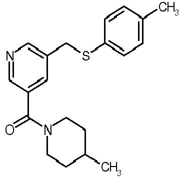 | 341.38 | 100 |
| 278 | 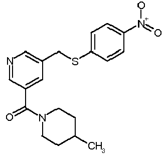 | 372.34 | 100 |
| 279 | 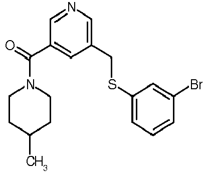 | 405.24 | 100 |
| 280 | 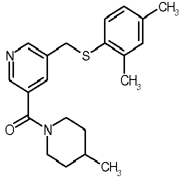 | 355.4 | 100 |
| 281 | 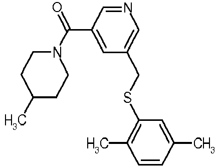 | 355.41 | 100 |
| 282 | 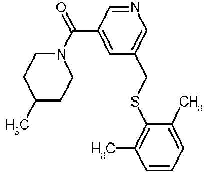 | 355.41 | 100 |
| 283 | 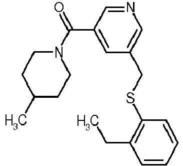 | 355.41 | 100 |
| 284 | 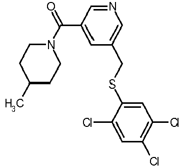 | 429.22 | 100 |
| 285 | 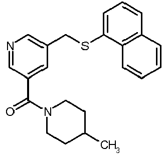 | 377.39 | 100 |
| 286 | 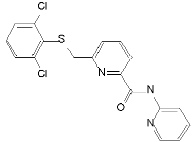 | 391 | 100 |
| 287 | 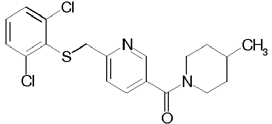 | 395 | 96 |
| 288 | 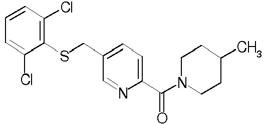 | 395 | 98 |
| 289 | 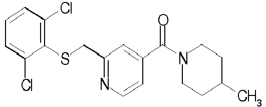 | 395 | 95 |
| 290 | 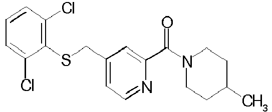 | 395 | 96 |
| 291 | 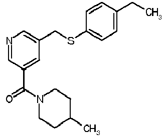 | 355.41 | 100 |
| 292 | 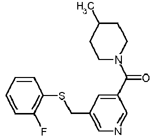 | 345.39 | 100 |
| 293 | 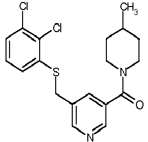 | 395.29 | 100 |
| 294 | 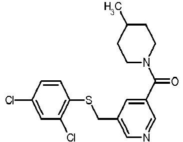 | 395.29 | 100 |
| 295 | 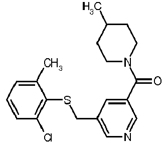 | 375.32 | 100 |
| 296 | 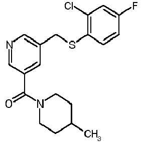 | 379.32 | 100 |
| 297 | 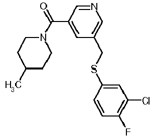 | 379.32 | 100 |
| 298 | 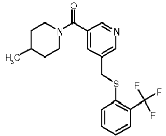 | 395.36 | 100 |
| 299 | 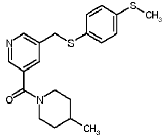 | 373.38 | 100 |
| 300 | 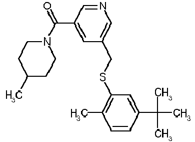 | 397.43 | 98 |
| 301 | 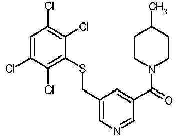 | 463.16 | 100 |
| 302 | 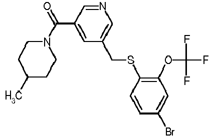 | 489.22 | 100 |
| 303 | 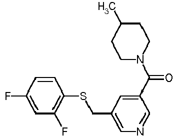 | 363.36 | 100 |
| 304 | 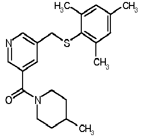 | 369.44 | 100 |
| 305 | 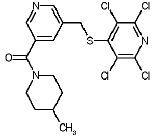 | 464.2 | 100 |

### EXAMPLES 306 TO 534

**Examples 306 to 534** were prepared according to the procedures described in **Examples 2** and **16** or other similar methods used by one skilled in the art, utilizing other appropriate reagents.

**TABLE 3**

| Example | Structure | Mass [M+H] | HPLC Purity (%) |
|---|---|---|---|
| 306 | 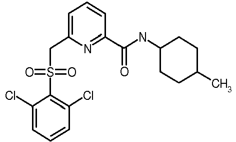 | 441.12 | 84 |
| 307 | 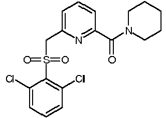 | 413.14 | 96 |
| 308 | 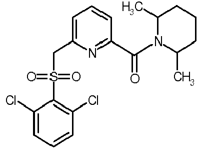 | 441.13 | 97 |
| 309 | 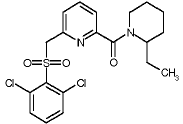 | 441.12 | 100 |
| 310 | 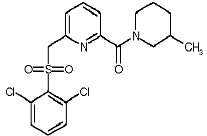 | 427.17 | 99 |
| 311 | 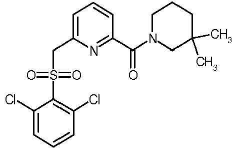 | 441.19 | 100 |
| 312 | 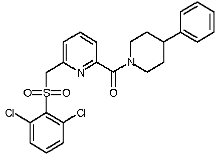 | 489.14 | 100 |
| 313 | 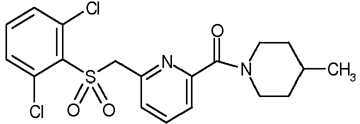 | 427.17 | 100 |
| 314 | 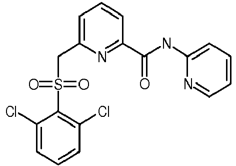 | 422.13 | 98 |
| 315 | 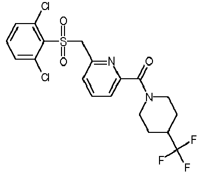 | 481.12 | 98 |
| 316 | 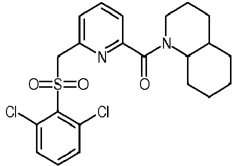 | 467.17 | 98 |
| 317 | 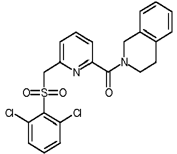 | 461.16 | 97 |
| 318 | 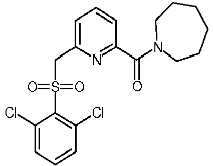 | 427.17 | 100 |
| 319 | 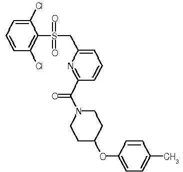 | 519.18 | 100 |
| 320 | 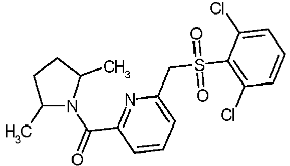 | 427.21 | 100 |
| 321 | 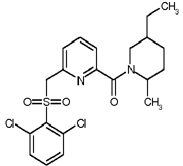 | 455.22 | 100 |
| 322 | 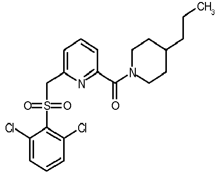 | 455.22 | 100 |
| 323 | 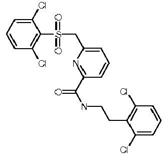 | 517.04 | 100 |
| 324 | 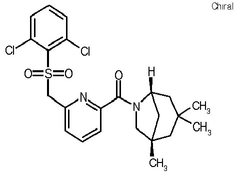 | 481.19 | 83 |
| 325 | 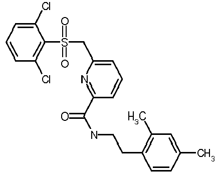 | 477.18 | 100 |
| 326 | 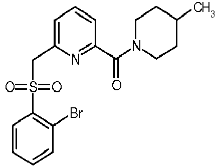 | 437.12 | 100 |
| 327 | 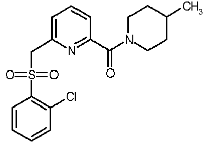 | 393.24 | 96 |
| 328 | 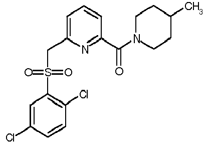 | 427.17 | 95 |
| 329 | 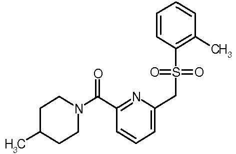 | 373.28 | 100 |
| 330 | 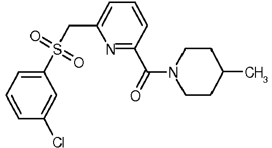 | 393.22 | 100 |
| 331 | 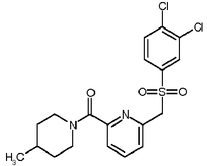 | 427.17 | 100 |
| 332 | 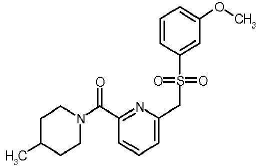 | 389.3 | 93 |
| 333 | 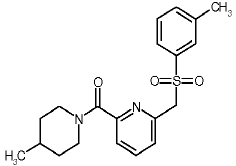 | 373.3 | 100 |
| 334 | 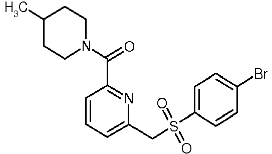 | 437.18 | 100 |
| 335 | 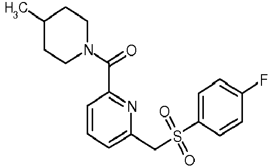 | 377.29 | 83 |
| 336 | 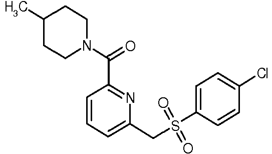 | 393.25 | 100 |
| 337 | 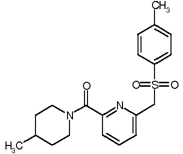 | 373.35 | 100 |
| 338 | 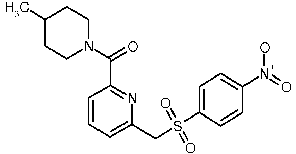 | 404.3 | 100 |
| 339 | 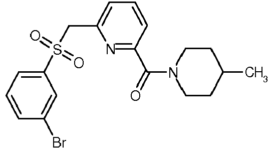 | 437.19 | 100 |
| 340 | 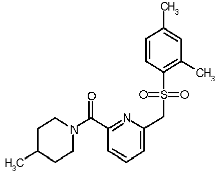 | 387.37 | 100 |
| 341 | 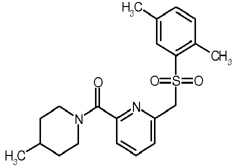 | 387.37 | 100 |
| 342 | 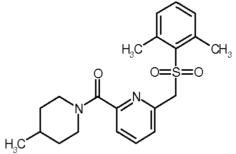 | 387.37 | 100 |
| 343 | 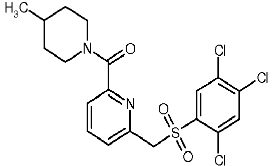 | 461.16 | 100 |
| 344 | 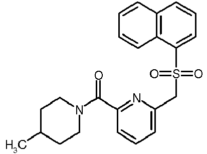 | 409.34 | 100 |
| 345 | 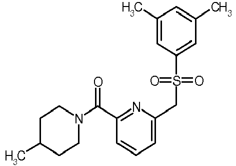 | 387.37 | 100 |
| 346 | 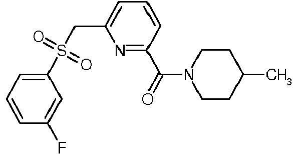 | 377.35 | 100 |
| 347 | 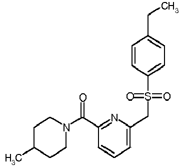 | 387.37 | 100 |
| 348 | 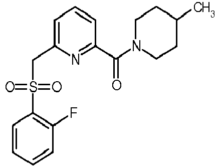 | 377.35 | 100 |
| 349 | 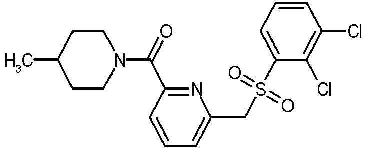 | 427.24 | 100 |
| 350 | 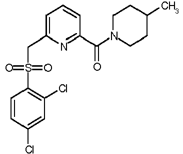 | 427.24 | 100 |
| 351 | 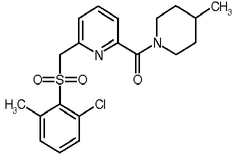 | 407.34 | 100 |
| 352 | 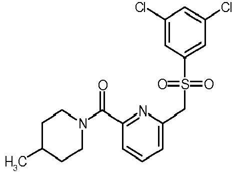 | 427.24 | 100 |
| 353 | 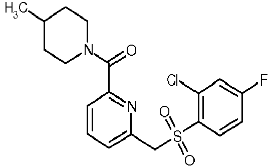 | 411.3 | 100 |
| 354 | 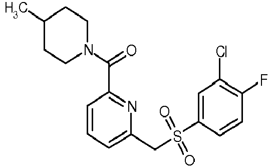 | 411.29 | 100 |
| 355 | 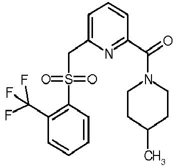 | 427.35 | 100 |
| 356 | 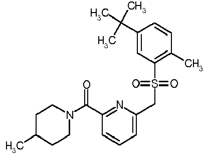 | 429.44 | 100 |
| 357 | 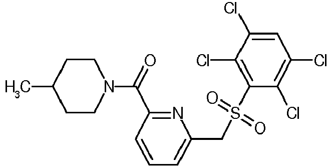 | 495.14 | 100 |
| 358 | 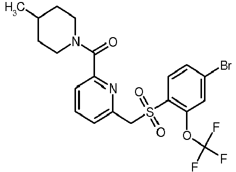 | 521.19 | 100 |
| 359 | 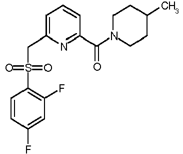 | 395.36 | 100 |
| 360 | 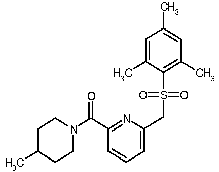 | 401.4 | 100 |
| 361 | 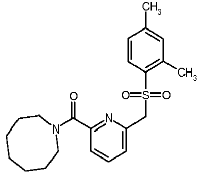 | 401.26 | 100 |
| 362 | 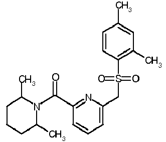 | 401.26 | 88 |
| 363 | 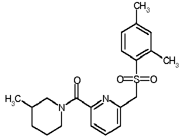 | 387.3 | 93 |
| 364 | 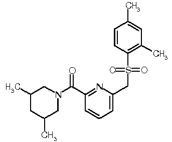 | 401.26 | 86 |
| 365 | 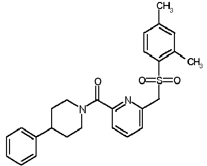 | 449.24 | 97 |
| 366 | 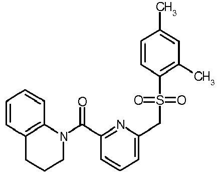 | 421.24 | 98 |
| 367 | 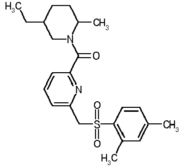 | 415.3 | 97 |
| 368 | 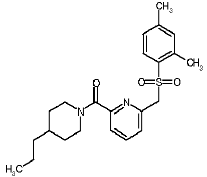 | 415.3 | 96 |
| 369 | 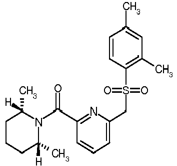 | 401.27 | 97 |
| 370 | 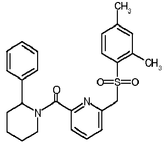 | 449.28 | 97 |
| 371 | 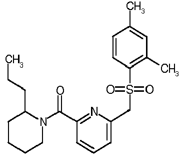 | 415.3 | 86 |
| 372 | 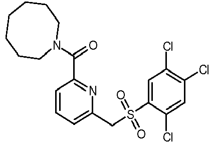 | 475.09 | 100 |
| 373 | 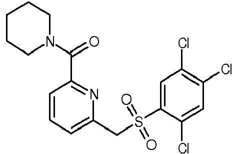 | 447.09 | 97 |
| 374 | 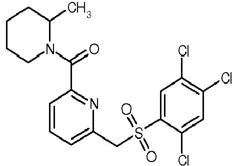 | 461.06 | 100 |
| 375 | 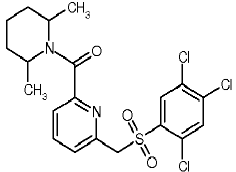 | 475.09 | 100 |
| 376 | 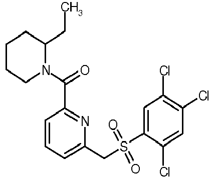 | 475.09 | 100 |
| 377 | 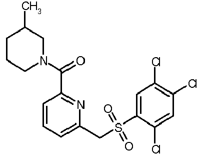 | 461.06 | 100 |
| 378 | 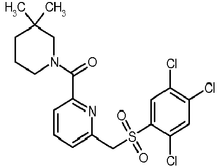 | 475.09 | 98 |
| 379 | 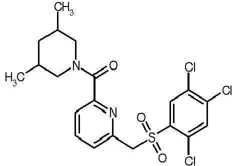 | 477.1 | 95 |
| 380 | 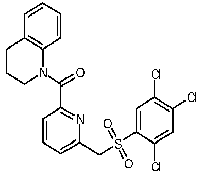 | 495.07 | 100 |
| 381 | 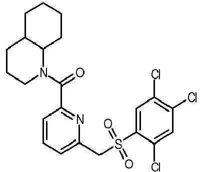 | 501.08 | 100 |
| 382 | 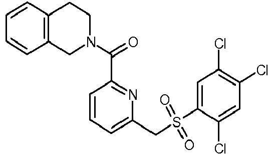 | 495.06 | 93 |
| 383 | 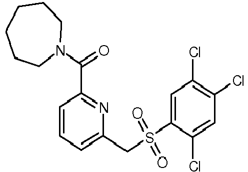 | 461.06 | 100 |
| 384 | 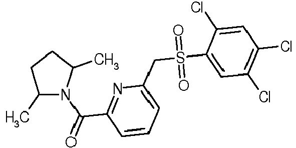 | 461.06 | 100 |
| 385 | 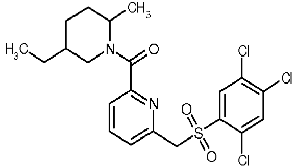 | 489.08 | 100 |
| 386 | 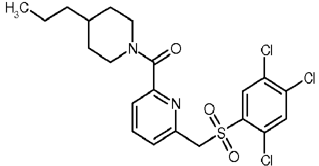 | 489.08 | 91 |
| 387 | 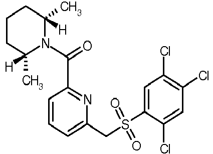 | 475.09 | 93 |
| 388 | 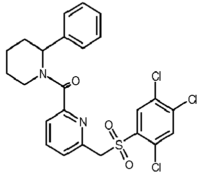 | 523.07 | 97 |
| 389 | 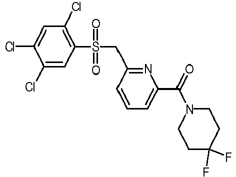 | 483.04 | 100 |
| 390 | 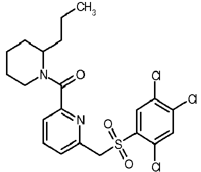 | 489.13 | 100 |
| 391 | 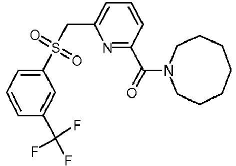 | 441.22 | 81 |
| 392 | 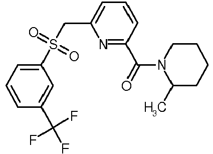 | 427.19 | 100 |
| 393 | 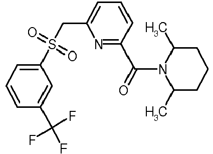 | 441.22 | 93 |
| 394 | 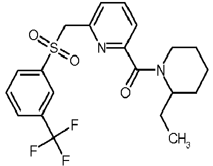 | 441.22 | 100 |
| 395 | 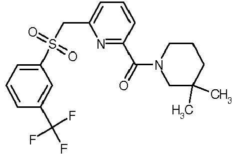 | 441.22 | 100 |
| 396 | 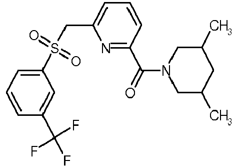 | 441.22 | 100 |
| 397 | 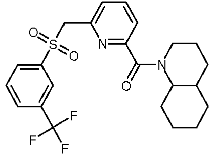 | 467.21 | 100 |
| 398 | 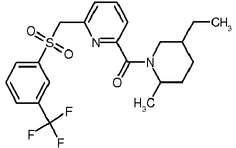 | 455.25 | 100 |
| 399 | 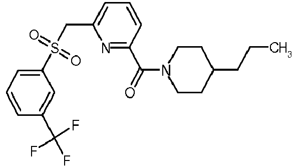 | 455.25 | 94 |
| 400 | 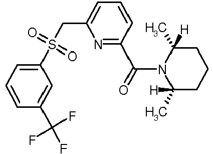 | 441.29 | 94 |
| 401 | 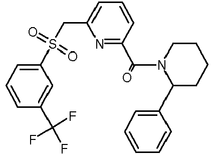 | 489.26 | 100 |
| 402 | 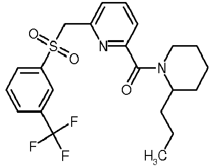 | 455.32 | 100 |
| 403 | 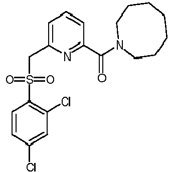 | 441.22 | 100 |
| 404 | 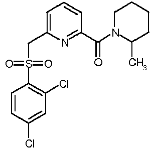 | 427.36 | 95 |
| 405 | 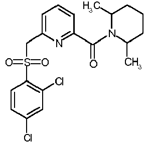 | 441.22 | 97 |
| 406 | 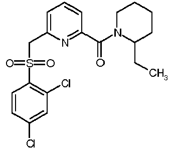 | 441.22 | 98 |
| 407 | 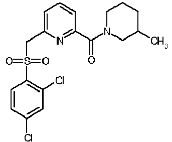 | 427.38 | 100 |
| 408 | 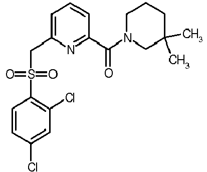 | 441.22 | 100 |
| 409 | 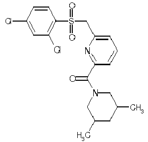 | 441.22 | 98 |
| 410 | 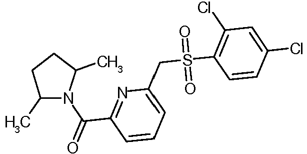 | 427.12 | 87 |
| 411 | 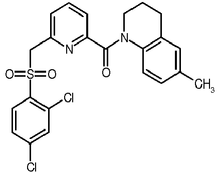 | 475.14 | 100 |
| 412 | 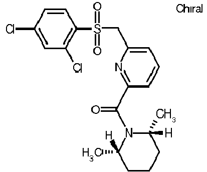 | 441.15 | 100 |
| 413 | 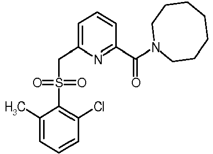 | 421.24 | 100 |
| 414 | 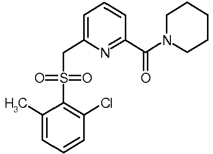 | 393.24 | 89 |
| 415 | 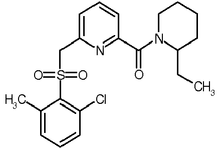 | 421.24 | 100 |
| 416 | 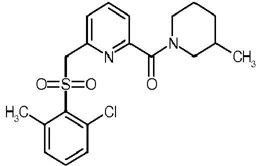 | 407.28 | 100 |
| 417 | 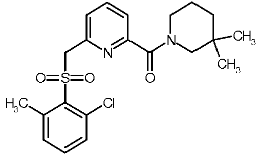 | 421.24 | 100 |
| 418 | 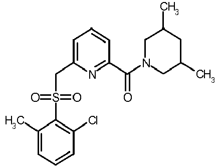 | 421.24 | 100 |
| 419 | 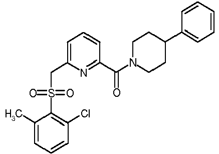 | 469.25 | 100 |
| 420 | 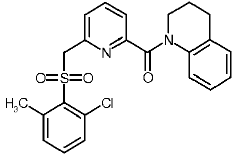 | 441.22 | 97 |
| 421 | 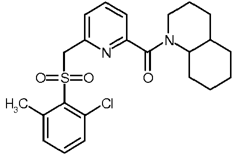 | 447.3 | 100 |
| 422 | 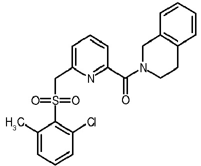 | 441.22 | 100 |
| 423 | 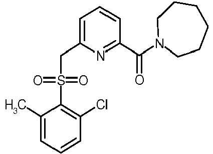 | 407.28 | 88 |
| 424 | 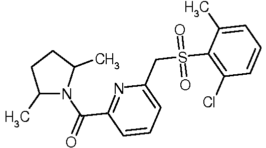 | 407.28 | 100 |
| 425 | 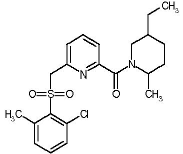 | 435.27 | 100 |
| 426 | 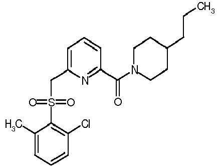 | 435.27 | 100 |
| 427 | 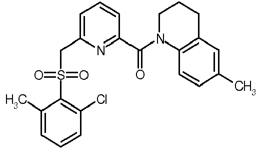 | 455.25 | 100 |
| 428 | 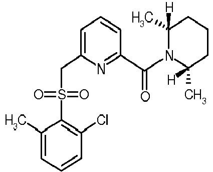 | 421.31 | 90 |
| 429 | 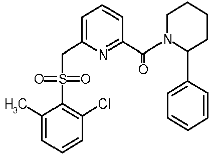 | 469.28 | 100 |
| 430 | 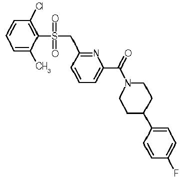 | 487.26 | 100 |
| 431 | 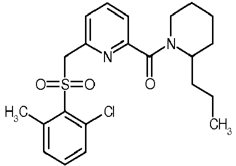 | 435.27 | 96 |
| 432 | 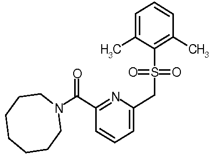 | 401.33 | 100 |
| 433 | 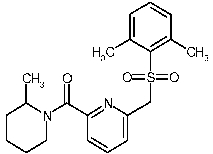 | 387.33 | 92 |
| 434 | 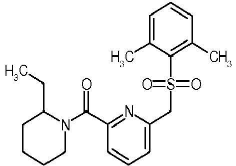 | 401.33 | 100 |
| 435 | 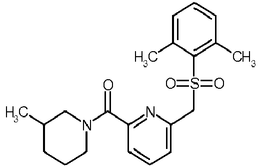 | 387.35 | 100 |
| 436 | 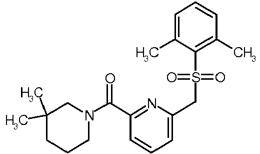 | 401.33 | 100 |
| 437 | 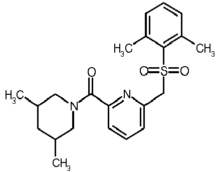 | 401.33 | 100 |
| 438 | 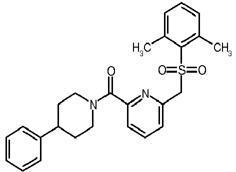 | 449.32 | 100 |
| 439 | 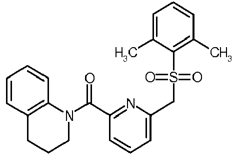 | 421.31 | 100 |
| 440 | 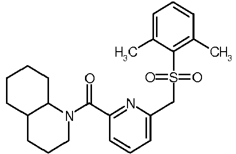 | 427.34 | 100 |
| 441 | 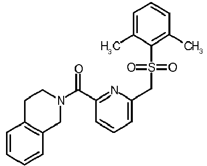 | 421.31 | 100 |
| 442 | 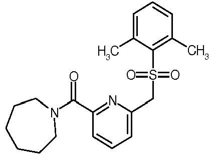 | 387.34 | 96 |
| 443 | 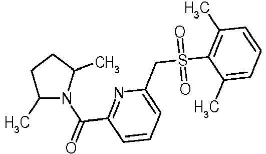 | 387.33 | 96 |
| 444 | 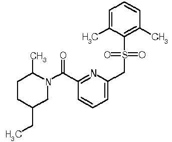 | 415.36 | 92 |
| 445 | 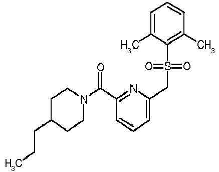 | 415.36 | 100 |
| 446 | 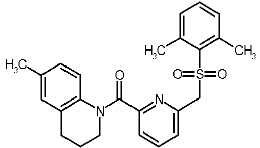 | 435.34 | 83 |
| 447 | 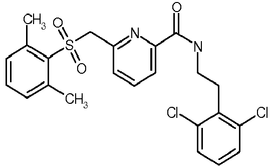 | 477.23 | 100 |
| 448 | 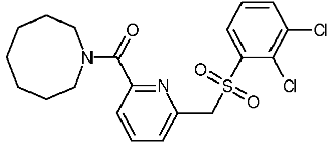 | 441.15 | 93 |
| 449 | 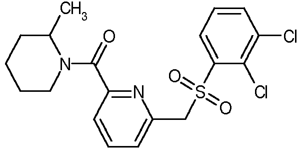 | 427.28 | 94 |
| 450 | 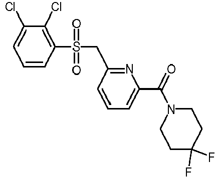 | 447.09 | 100 |
| 451 | 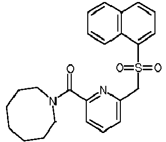 | 423.31 | 100 |
| 452 | 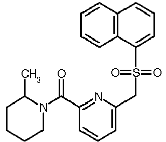 | 409.35 | 100 |
| 453 | 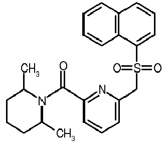 | 423.31 | 100 |
| 454 | 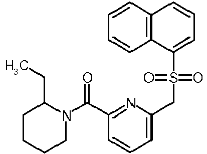 | 423.33 | 100 |
| 455 | 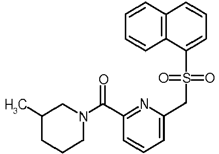 | 409.35 | 100 |
| 456 | 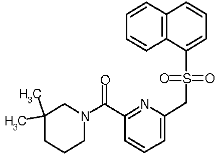 | 423.33 | 100 |
| 457 | 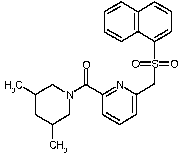 | 423.33 | 100 |
| 3458 | 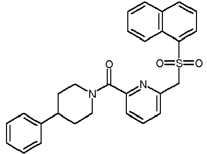 | 471.36 | 100 |
| 459 | 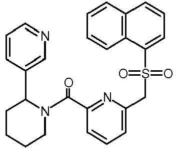 | 472.33 | 97 |
| 460 | 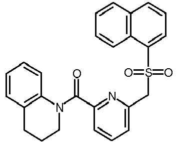 | 443.29 | 100 |
| 461 | 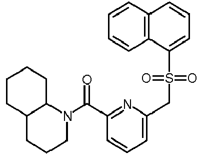 | 449.38 | 100 |
| 462 | 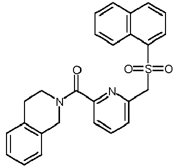 | 443.29 | 100 |
| 463 | 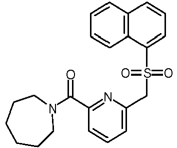 | 409.35 | 100 |
| 464 | 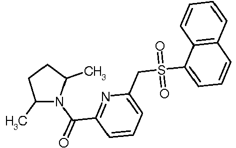 | 409.35 | 95 |
| 465 | 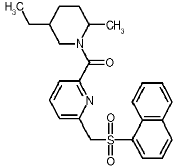 | 437.35 | 100 |
| 466 | 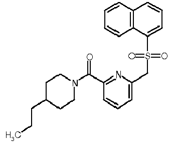 | 437.35 | 100 |
| 467 | 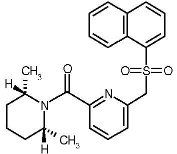 | 423.32 | 100 |
| 468 | 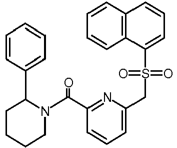 | 471.36 | 100 |
| 469 | 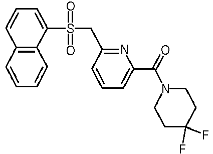 | 431.26 | 100 |
| 470 | 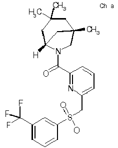 | 481.31 | 94 |
| 471 | 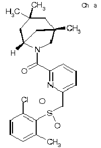 | 461.3 | 96 |
| 472 | 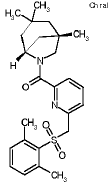 | 441.36 | 95 |
| 473 | 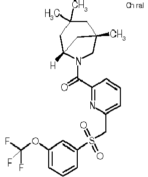 | 497.24 | 97 |
| 474 | 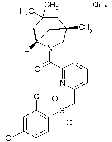 | 481.2 | 96 |
| 475 | 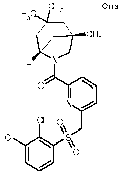 | 481.21 | 94 |
| 476 | 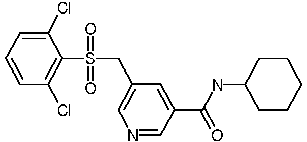 | 427.06 | 92 |
| 477 | 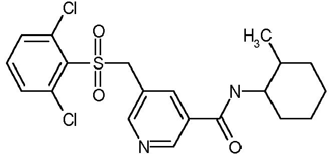 | 441.08 | 97 |
| 478 | 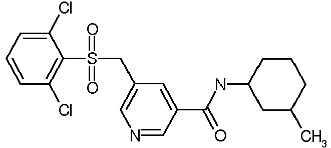 | 441.08 | 97 |
| 479 | 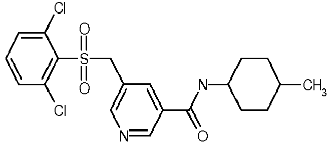 | 441.08 | 95 |
| 480 | 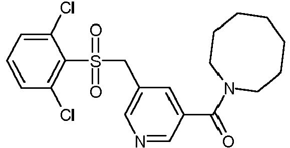 | 441.09 | 91 |
| 481 | 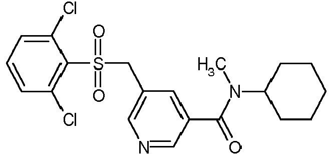 | 441.07 | 95 |
| 482 | 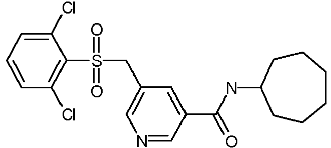 | 441.07 | 95 |
| 483 | 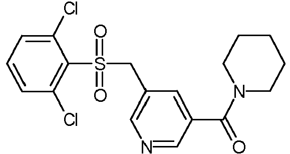 | 413.04 | 100 |
| 484 | 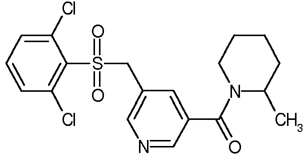 | 427.03 | 97 |
| 485 | 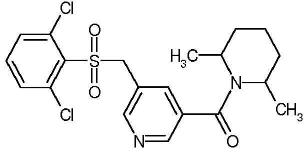 | 258.17 | 84 |
| 486 | 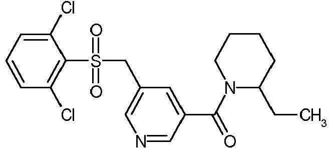 | 441.06 | 96 |
| 487 | 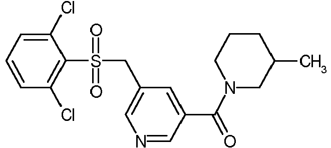 | 427.03 | 100 |
| 488 | 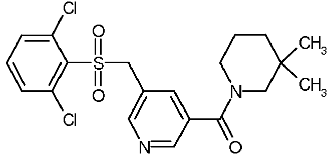 | 441.05 | 96 |
| 489 | 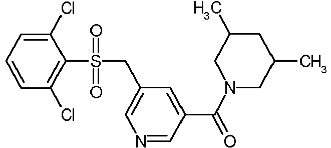 | 441.06 | 95 |
| 490 | 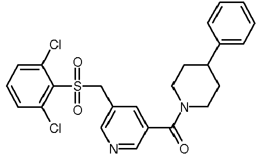 | 489.08 | 94 |
| 491 | 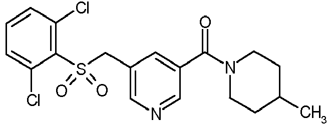 | 427.01 | 100 |
| 492 | 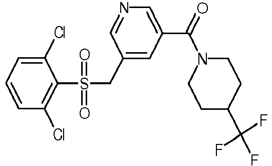 | 481.04 | 95 |
| 493 | 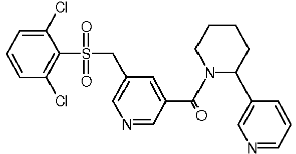 | 490.06 | 96 |
| 494 | 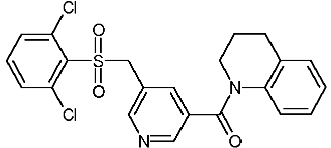 | 461.08 | 100 |
| 495 | 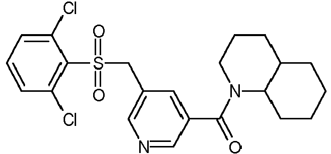 | 467.11 | 96 |
| 496 | 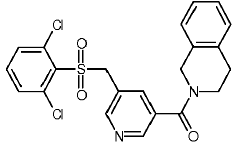 | 515.29 | 88 |
| 497 | 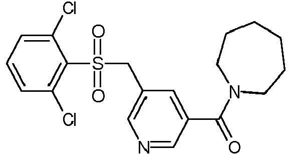 | 427.02 | 100 |
| 498 | 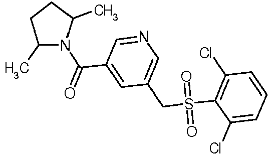 | 427.02 | 100 |
| 499 | 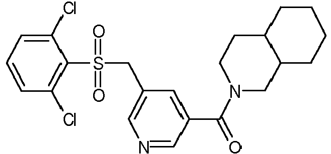 | 467.12 | 95 |
| 500 | 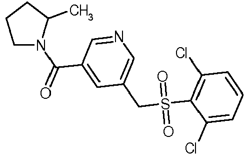 | 413.02 | 100 |
| 501 | 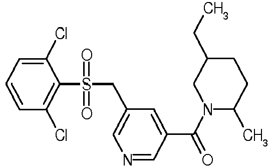 | 455.1 | 84 |
| 502 | 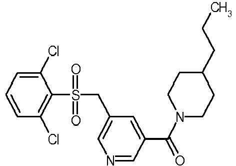 | 455.09 | 87 |
| 503 | 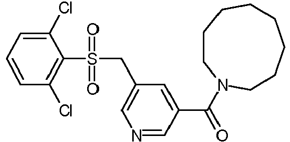 | 455.09 | 96 |
| 504 | 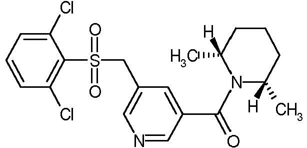 | 515.28 | 100 |
| 505 | 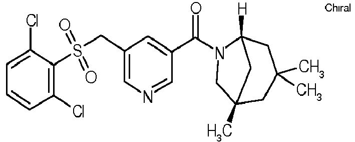 | 481.1 | 100 |
| 506 | 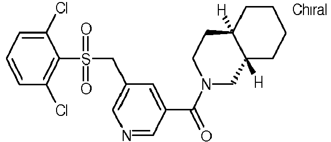 | 467.11 | 93 |
| 507 | 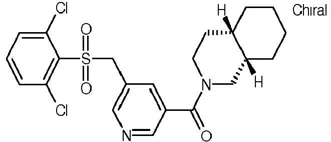 | 467.11 | 96 |
| 508 | 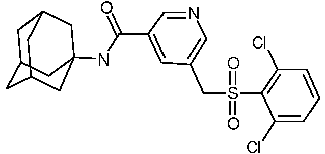 | 479.09 | 100 |
| 509 | 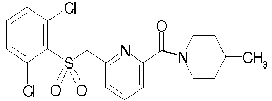 | 427 | 100 |
| 510 | 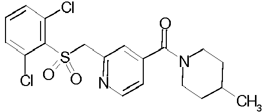 | 427 | 95 |
| 511 | 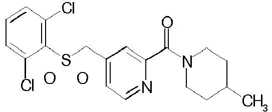 | 427 | 98 |
| 512 | 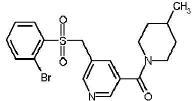 | 437.1 | 91 |
| 513 | 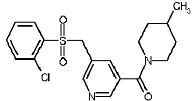 | 393.21 | 91 |
| 514 | 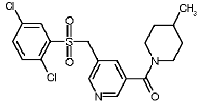 | 427.15 | 100 |
| 515 | 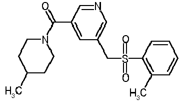 | 373.24 | 87 |
| 516 | 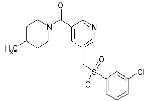 | 393.21 | 100 |
| 517 | 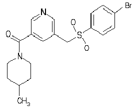 | 437.1 | 100 |
| 518 | 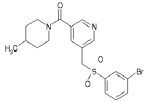 | 437.17 | 100 |
| 519 | 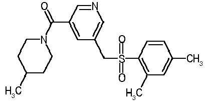 | 387.3 | 100 |
| 520 | 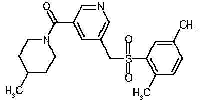 | 387.31 | 100 |
| 521 | 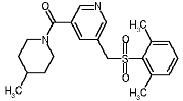 | 387.34 | 100 |
| 522 | 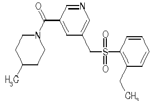 | 387.33 | 100 |
| 523 | 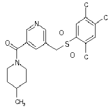 | 461.15 | 100 |
| 524 | 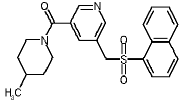 | 409.3 | 100 |
| 525 | 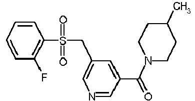 | 377.3 | 100 |
| 526 | 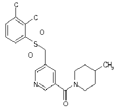 | 427.22 | 100 |
| 527 | 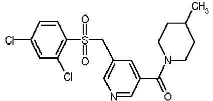 | 427.22 | 100 |
| 528 | 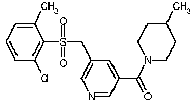 | 407.24 | 100 |
| 529 | 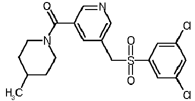 | 427.22 | 100 |
| 530 | 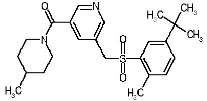 | 429.36 | 100 |
| 531 | 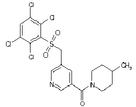 | 495.1 | 100 |
| 532 | 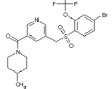 | 521.16 | 100 |
| 533 | 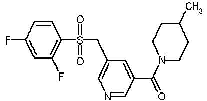 | 395.29 | 96 |
| 534 | 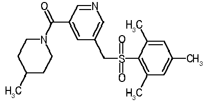 | 401.35 | 100 |

### EXAMPLES 535 TO 742

Examples 535 to 742 in Table 4 were prepared according to the procedures described in Examples 1 and 17 or other similar methods used by one skilled in the art, utilizing other appropriate reagents.

**TABLE 4**

| Example | Structure | Mass [M+H]⁺ | HPLC Purity (%) |
|---|---|---|---|
| 535 | 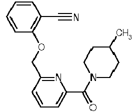 | 336.48 | 100 |
| 536 | 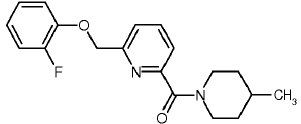 | 329.47 | 98 |
| 537 | 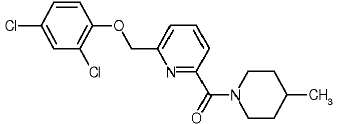 | 379.38 | 100 |
| 538 | 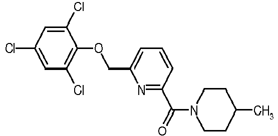 | 413.34 | 96 |
| 539 | 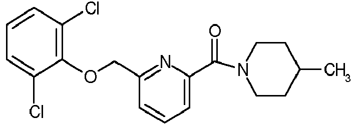 | 379.38 | 99 |
| 540 | 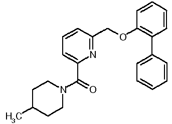 | 387.52 | 100 |
| 541 | 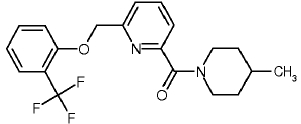 | 379.46 | 100 |
| 542 | 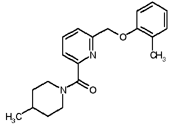 | 325.51 | 98 |
| 543 | 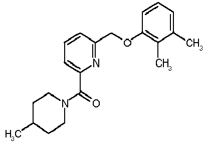 | 339.52 | 100 |
| 544 | 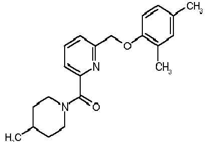 | 339.53 | 98 |
| 545 | 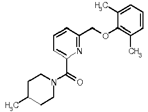 | 339.52 | 100 |
| 546 | 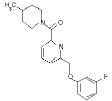 | 329.47 | 100 |
| 547 | 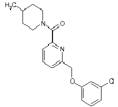 | 345.44 | 99 |
| 548 | 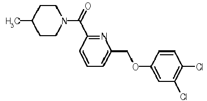 | 379.42 | 100 |
| 549 | 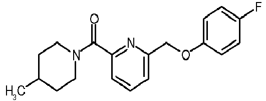 | 329.47 | 98 |
| 550 | 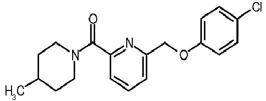 | 345.46 | 99 |
| 551 | 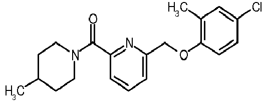 | 359.47 | 100 |
| 552 | 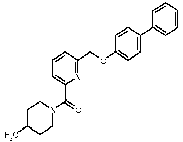 | 387.53 | 98 |
| 553 | 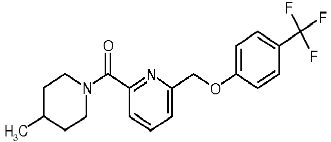 | 379.5 | 100 |
| 554 | 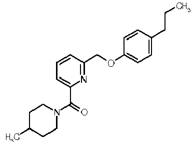 | 353.53 | 90 |
| 555 | 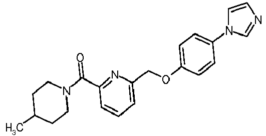 | 377.51 | 98 |
| 556 | 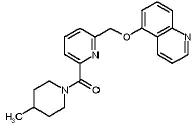 | 362.51 | 100 |
| 557 | 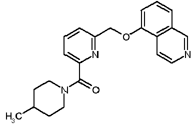 | 362.51 | 100 |
| 558 | 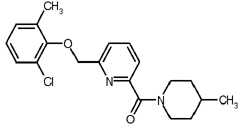 | 359.47 | 98 |
| 559 | 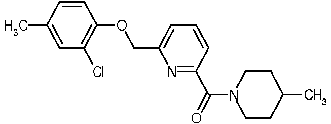 | 359.47 | 100 |
| 560 | 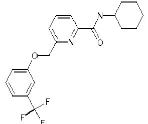 | 379.43 | 100 |
| 561 | 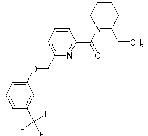 | 393.45 | 100 |
| 562 | 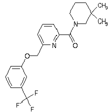 | 393.45 | 100 |
| 563 | 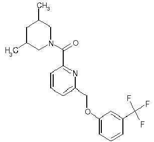 | 393.45 | 100 |
| 564 | 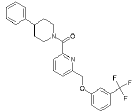 | 441.4 | 100 |
| 565 | 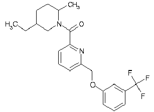 | 407.46 | 100 |
| 566 | 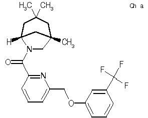 | 433.45 | |
| 567 | 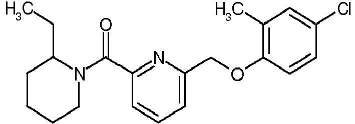 | 373.42 | 100 |
| 568 | 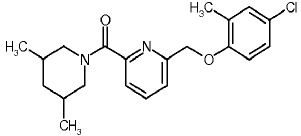 | 373.42 | 100 |
| 569 | 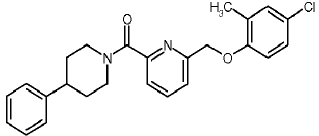 | 421.37 | 100 |
| 570 | 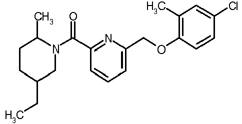 | 387.43 | 100 |
| 571 | 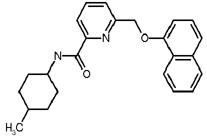 | 375.33 | 100 |
| 572 | 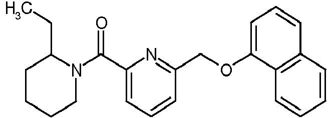 | 375.33 | 81 |
| 573 | 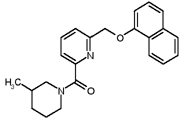 | 361.29 | 90 |
| 574 | 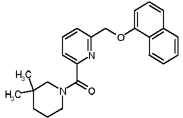 | 375.33 | 100 |
| 575 | 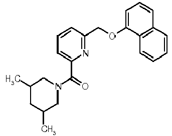 | 375.33 | 100 |
| 576 | 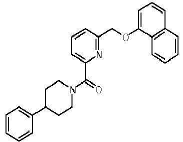 | 423.27 | 94 |
| 577 | 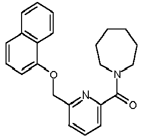 | 361.29 | 100 |
| 578 | 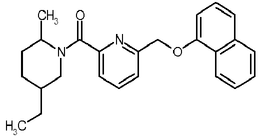 | 389.34 | 93 |
| 579 | 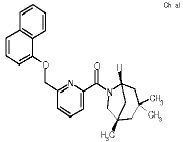 | 415.31 | 95 |
| 580 | 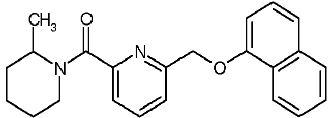 | 359.34 | 84 |
| 581 | 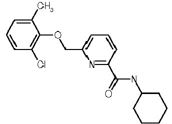 | 359.25 | 100 |
| 582 | 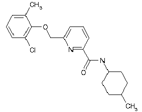 | 373.3 | 100 |
| 583 | 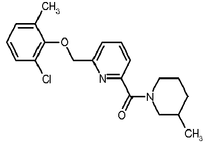 | 359.39 | 100 |
| 584 | 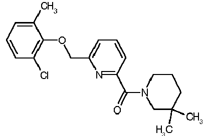 | 373.42 | 100 |
| 585 | 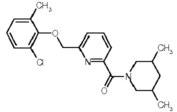 | 373.42 | 100 |
| 586 | 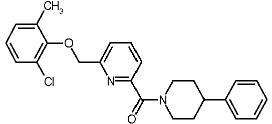 | 421.37 | 100 |
| 587 | 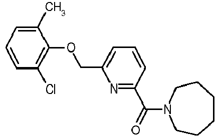 | 359.39 | 100 |
| 588 | 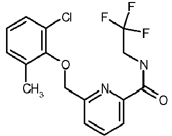 | 359.34 | 91 |
| 589 | 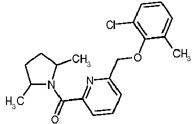 | 359.39 | 84 |
| 590 | 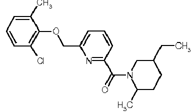 | 387.43 | 100 |
| 591 | 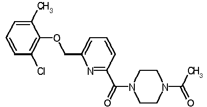 | 387.41 | 89 |
| 592 | 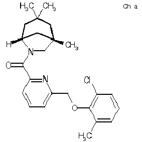 | 413.42 | 100 |
| 593 | 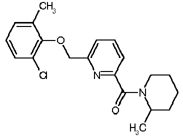 | 359.39 | 100 |
| 594 | 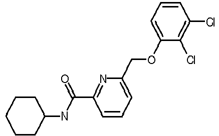 | 379.11 | 100 |
| 595 | 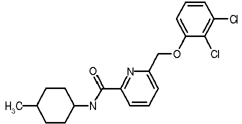 | 393.13 | 100 |
| 596 | 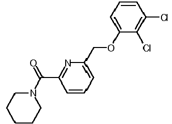 | 365.11 | 89 |
| 597 | 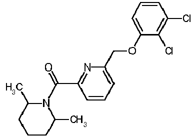 | 393.13 | 100 |
| 598 | 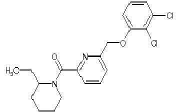 | 393.13 | 96 |
| 599 | 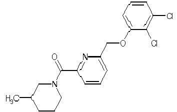 | 379.11 | 100 |
| 600 | 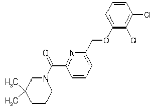 | 393.13 | 100 |
| 601 | 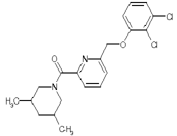 | 393.13 | 100 |
| 602 | 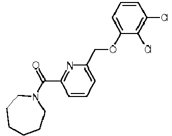 | 379.11 | 100 |
| 603 | 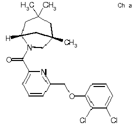 | 433.16 | 100 |
| 604 | 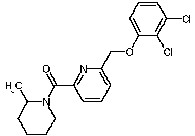 | 379.13 | 100 |
| 605 | 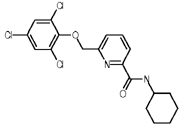 | 413.08 | 100 |
| 606 | 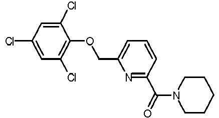 | 399.07 | 92 |
| 607 | 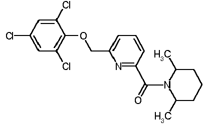 | 427.1 | 99 |
| 608 | 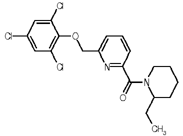 | 427.08 | 99 |
| 609 | 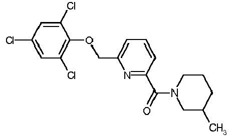 | 413.09 | 99 |
| 610 | 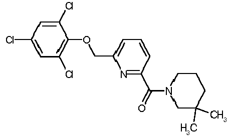 | 427.09 | 100 |
| 611 | 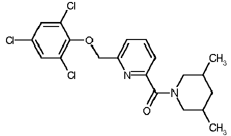 | 427.07 | 100 |
| 612 | 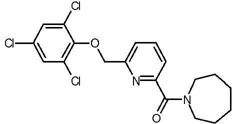 | 413.07 | 99 |
| 613 | 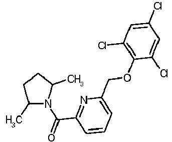 | 413.08 | 100 |
| 614 | 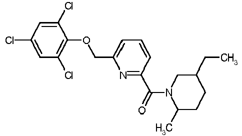 | 441.11 | 100 |
| 615 | 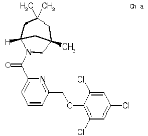 | 467.12 | 100 |
| 616 | 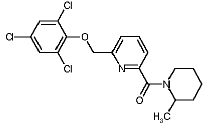 | 413.06 | 100 |
| 617 | 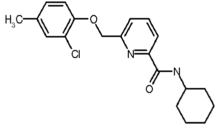 | 359.19 | 100 |
| 618 | 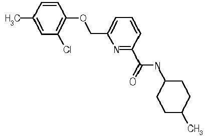 | 373.2 | 95 |
| 619 | 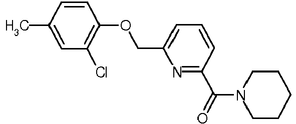 | 345.2 | 100 |
| 620 | 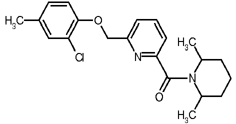 | 373.21 | 100 |
| 621 | 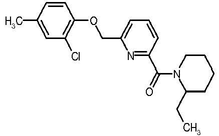 | 373.2 | 100 |
| 622 | 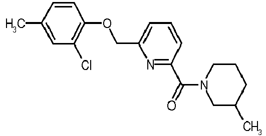 | 359.19 | 100 |
| 623 | 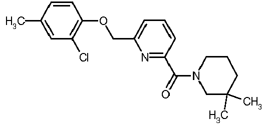 | 373.19 | 100 |
| 624 | 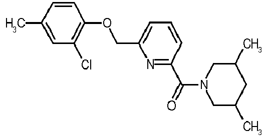 | 373.2 | 100 |
| 625 | 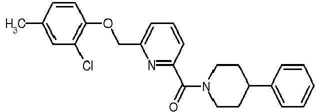 | 421.2 | 100 |
| 626 | 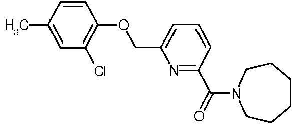 | 359.19 | 100 |
| 627 | 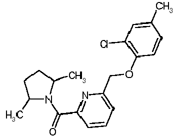 | 359.19 | 100 |
| 628 | 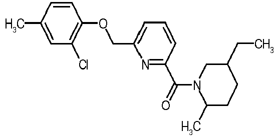 | 387.2 | 100 |
| 629 | 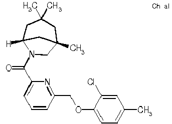 | 413.25 | 100 |
| 630 | 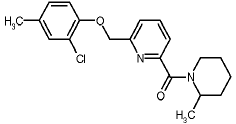 | 359.19 | 100 |
| 631 | 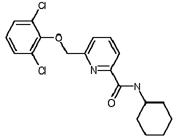 | 379.11 | 100 |
| 632 | 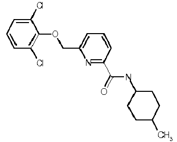 | 393.12 | 100 |
| 633 | 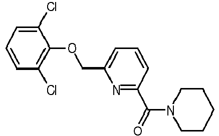 | 365.1 | 92 |
| 634 | 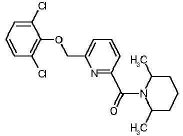 | 393.13 | 82 |
| 635 | 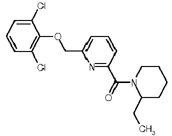 | 393.12 | 100 |
| 636 | 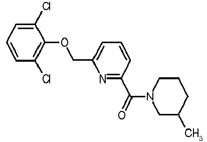 | 379.13 | 92 |
| 637 | 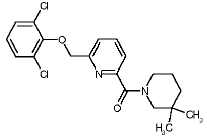 | 393.12 | 82 |
| 638 | 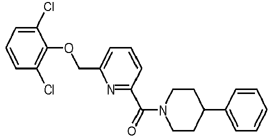 | 441.12 | 94 |
| 639 | 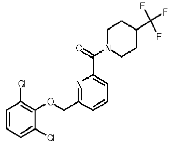 | 433.09 | 100 |
| 640 | 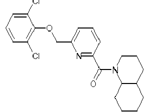 | 419.14 | 100 |
| 641 | 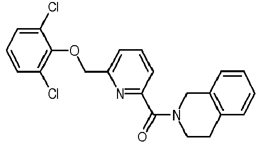 | 413.08 | 94 |
| 642 | 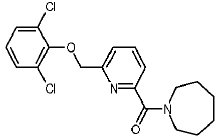 | 379.12 | 92 |
| 643 | 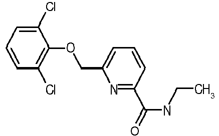 | 325.1 | 94 |
| 644 | 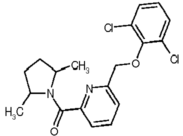 | 379.11 | 100 |
| 645 | 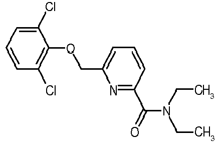 | 353.1 | 88 |
| 646 | 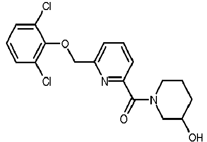 | 381.08 | 97 |
| 647 | 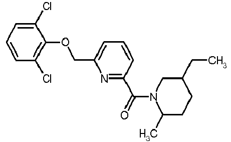 | 407.15 | 100 |
| 648 | 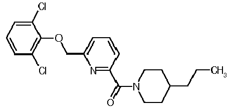 | 407.14 | 99 |
| 649 | 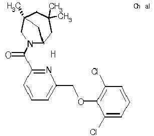 | 433.14 | 100 |
| 650 | 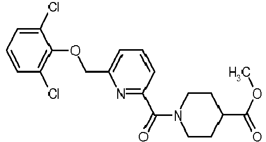 | 423.1 | 100 |
| 651 | 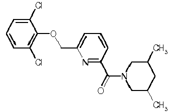 | 393.12 | 88 |
| 652 | 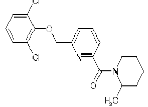 | 379.11 | 91 |
| 653 | 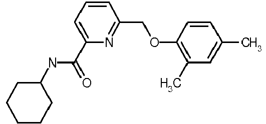 | 339.27 | 100 |
| 654 | 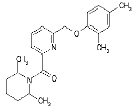 | 353.27 | 87 |
| 655 | 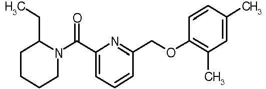 | 353.27 | 100 |
| 656 | 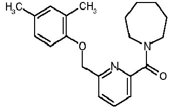 | 339.26 | 94 |
| 657 | 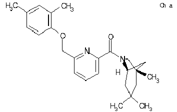 | 393.28 | 100 |
| 658 | 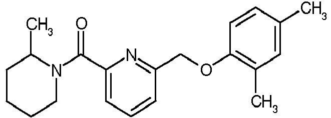 | 339.26 | 98 |
| 659 | 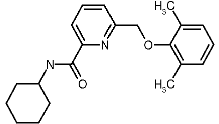 | 339.25 | 100 |
| 660 | 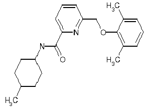 | 353.26 | 98 |
| 661 | 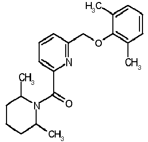 | 353.27 | 83 |
| 662 | 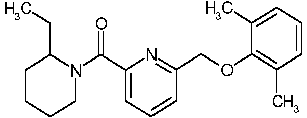 | 353.26 | 97 |
| 663 | 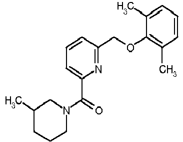 | 339.26 | 97 |
| 664 | 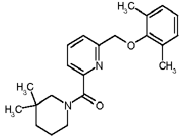 | 353.26 | 95 |
| 665 | 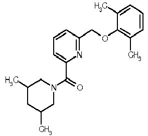 | 353.27 | 97 |
| 666 | 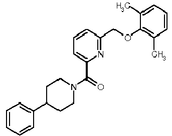 | 401.25 | 100 |
| 667 | 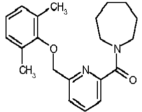 | 339.25 | 96 |
| 668 | 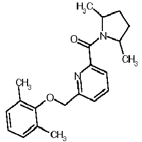 | 339.27 | 97 |
| 669 | 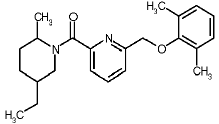 | 367.28 | 100 |
| 670 | 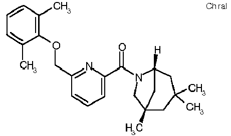 | 393.3 | 98 |
| 671 | 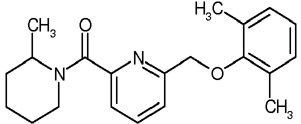 | 339.25 | 100 |
| 672 | 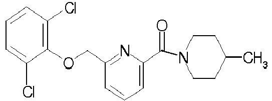 | 379 | 100 |
| 673 | 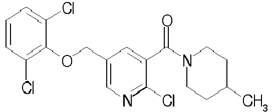 | 413 | 99.1 |
| 674 | 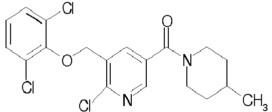 | 413 | 100 |
| 675 | 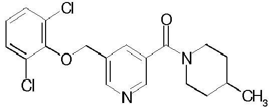 | 379 | 96 |
| 676 | 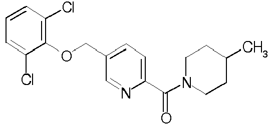 | 379 | 95 |
| 677 | 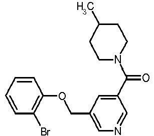 | 389.2 | 100 |
| 678 | 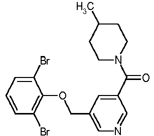 | 467.1 | 100 |
| 679 | 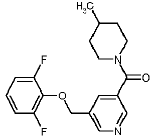 | 347.32 | 100 |
| 680 | 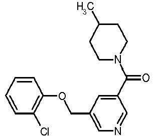 | 345.32 | 100 |
| 681 | 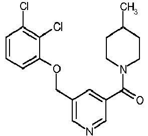 | 379.25 | 100 |
| 682 | 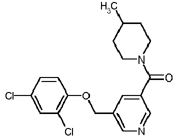 | 379.25 | 100 |
| 683 | 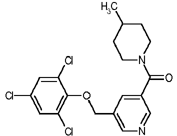 | 413.22 | 100 |
| 684 | 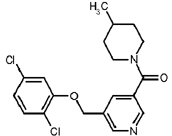 | 379.25 | 100 |
| 685 | 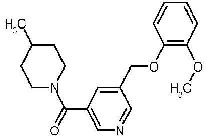 | 689.1 | 98 |
| 686 | 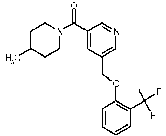 | 379.38 | 100 |
| 687 | 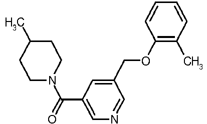 | 325.42 | 98 |
| 688 | 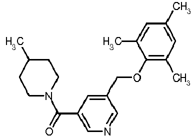 | 353.42 | 100 |
| 689 | 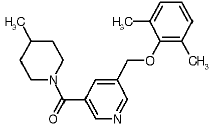 | 339.45 | 100 |
| 690 | 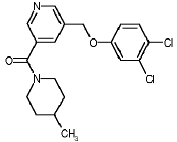 | 379.32 | 100 |
| 691 | 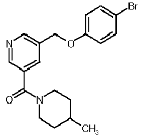 | 389.28 | 100 |
| 692 | 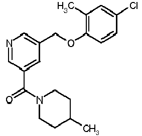 | 359.39 | 100 |
| 693 | 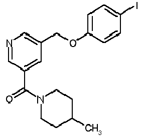 | 437.27 | 100 |
| 694 | 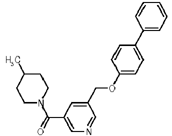 | 387.41 | 100 |
| 695 | 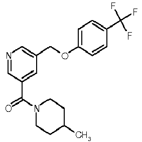 | 379.39 | 100 |
| 696 | 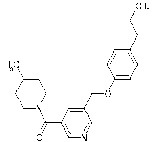 | 353.47 | 96 |
| 697 | 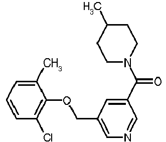 | 359.39 | 96 |
| 698 | 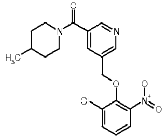 | 390.33 | 97 |
| 699 | 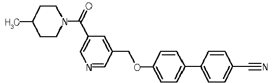 | 412.43 | 100 |
| 700 | 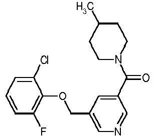 | 363.36 | 100 |
| 701 | 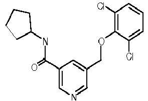 | 365.15 | 100 |
| 702 | 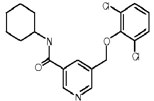 | 379.18 | 93 |
| 703 | 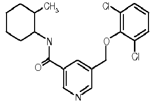 | 393.16 | 98 |
| 704 | 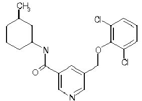 | 393.19 | 100 |
| 705 | 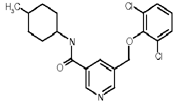 | 393.19 | 100 |
| 706 | 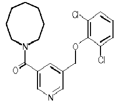 | 393.21 | 99 |
| 707 | 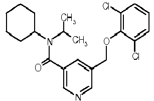 | 421.18 | 97 |
| 708 | 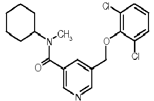 | 393.16 | 100 |
| 709 | 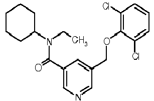 | 407.18 | 100 |
| 710 | 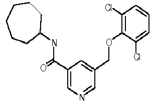 | 393.18 | 100 |
| 711 | 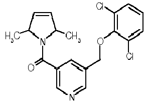 | 377.16 | 100 |
| 712 | 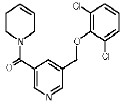 | 363.13 | 100 |
| 713 | 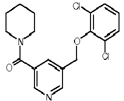 | 365.15 | 100 |
| 714 | 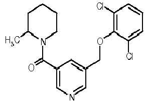 | 379.18 | 100 |
| 715 | 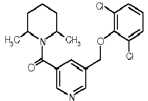 | 393.16 | 100 |
| 716 | 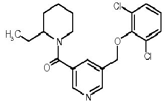 | 393.17 | 99 |
| 717 | 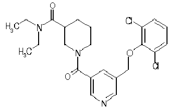 | 464.15 | 93 |
| 718 | 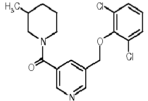 | 379.17 | 100 |
| 719 | 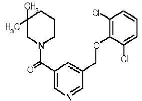 | 393.18 | 99 |
| 720 | 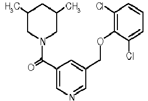 | 393.15 | 100 |
| 721 | 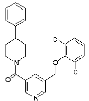 | 441.14 | 100 |
| 722 | 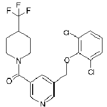 | 433.10 | 100 |
| 723 | 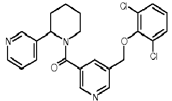 | 442.11 | 100 |
| 724 | 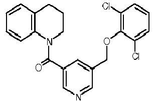 | 413.11 | 99 |
| 725 | 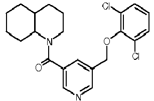 | 419.18 | 100 |
| 726 | 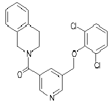 | 413.11 | 99 |
| 727 | 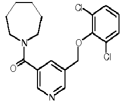 | 379.18 | 100 |
| 728 | 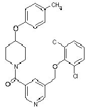 | 471.12 | 97 |
| 729 | 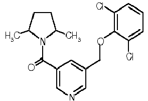 | 379.16 | 100 |
| 730 | 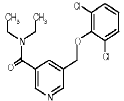 | 353.15 | 100 |
| 731 | 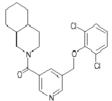 | 419.18 | 93 |
| 732 | 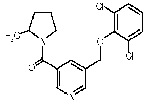 | 365.15 | 100 |
| 733 | 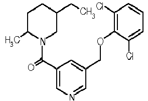 | 407.18 | 100 |
| 734 | 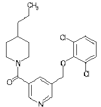 | 407.18 | 100 |
| 735 | 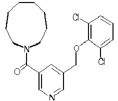 | 407.18 | 100 |
| 736 | 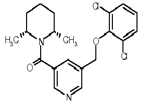 | 393.17 | 89 |
| 737 | 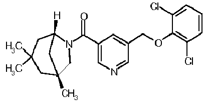 | 433.15 | 100 |
| 738 | 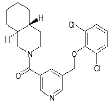 | 419.14 | 97 |
| 739 | 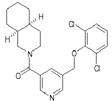 | 419.16 | 100 |
| 740 | 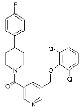 | 459.11 | 100 |
| 741 | 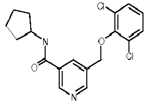 | 365.15 | 100 |
| 742 | 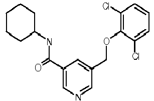 | 379.18 | 93 |

### EXAMPLES 743 TO 923

Examples 743 to 923 in Table 5 were prepared according to the procedures described in Examples 18 and 19 or other similar methods used by one skilled in the art, utilizing other appropriate reagents.

**TABLE 5**

| Example | Structure | Mass [M+H] | HPLC Purity (%) |
|---|---|---|---|
| 743 | 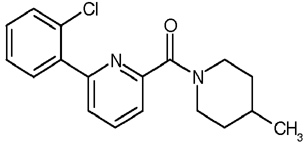 | 315.25 | 100 |
| 744 | 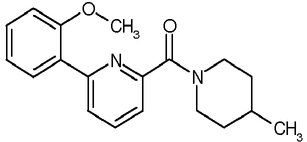 | 311 | 100 |
| 745 | 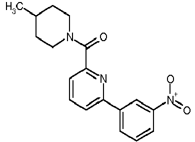 | 326.21 | 95 |
| 746 | 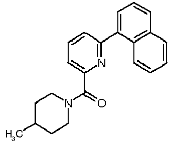 | 331.25 | 100 |
| 747 | 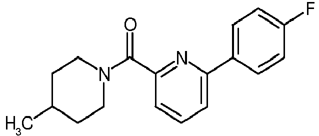 | 299.22 | 91 |
| 748 | 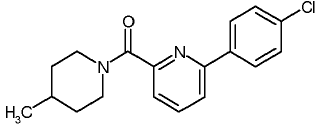 | 315.18 | 87 |
| 749 | 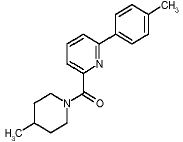 | 295.28 | 88 |
| 750 | 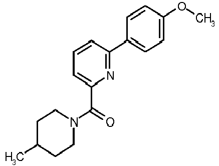 | 311.26 | 88 |
| 751 | 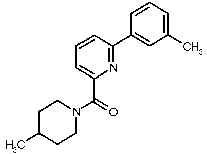 | 295.27 | 96 |
| 752 | 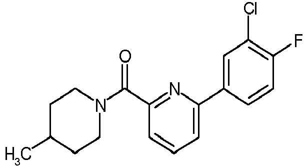 | 333.16 | 100 |
| 753 | 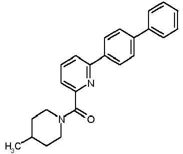 | 357.24 | 92 |
| 754 | 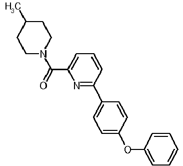 | 373.24 | 96 |
| 755 | 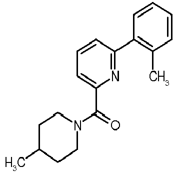 | 295.26 | 100 |
| 756 | 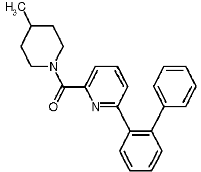 | 357.28 | 96 |
| 757 | 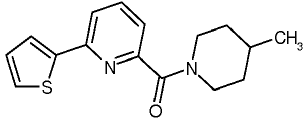 | 287.2 | 92 |
| 758 | 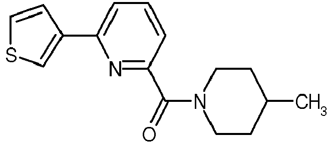 | 287.19 | 88 |
| 759 | 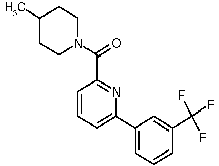 | 349.21 | 100 |
| 760 | 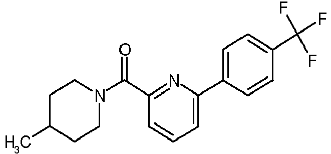 | 349.23 | 97 |
| 761 | 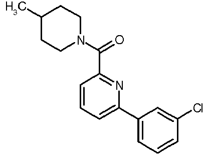 | 315.18 | 100 |
| 762 | 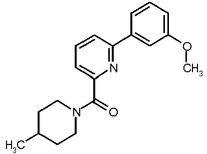 | 311.24 | 100 |
| 763 | 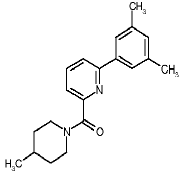 | 309.25 | 97 |
| 764 | 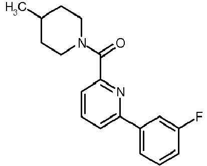 | 299.21 | 94 |
| 765 | 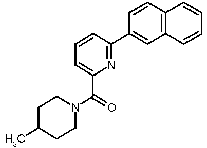 | 331.23 | 100 |
| 766 | 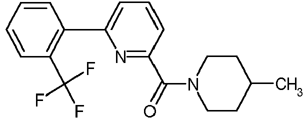 | 349.23 | 100 |
| 767 | 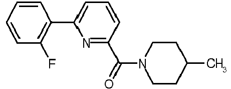 | 299.24 | 89 |
| 768 | 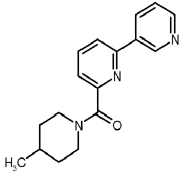 | 279.18 | 93 |
| 769 | 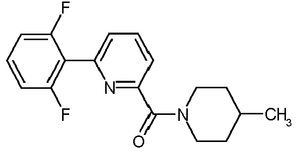 | 317.2 | 88 |
| 770 | 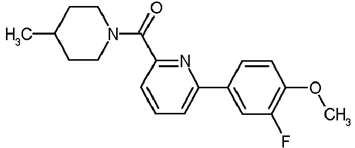 | 329.23 | 100 |
| 771 | 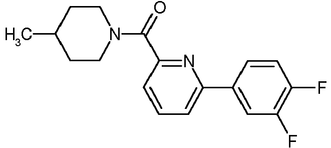 | 317.2 | 100 |
| 772 | 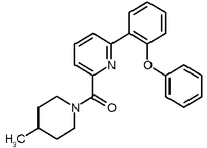 | 373.24 | 97 |
| 773 | 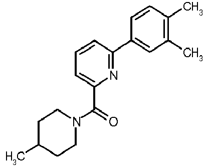 | 309.29 | 99 |
| 774 | 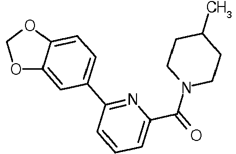 | 325.23 | 95 |
| 775 | 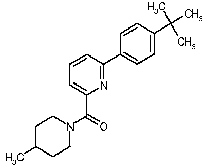 | 337.3 | 99 |
| 776 | 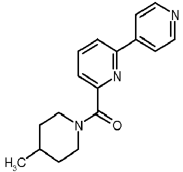 | 279.18 | 89 |
| 777 | 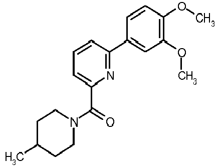 | 341.21 | 85 |
| 778 | 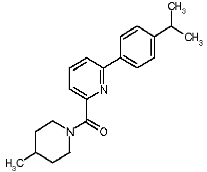 | 323.27 | 81 |
| 779 | 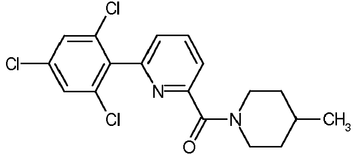 | 383.1 | 89 |
| 780 | 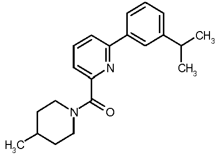 | 323.27 | 89 |
| 781 | 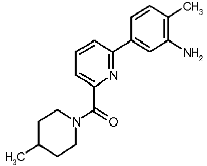 | 310.28 | 92 |
| 782 | 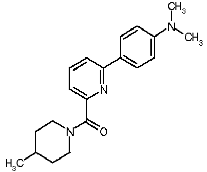 | 324.26 | 100 |
| 783 | 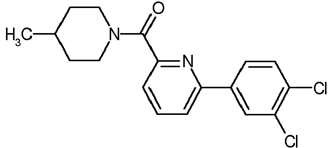 | 349.13 | 100 |
| 784 | 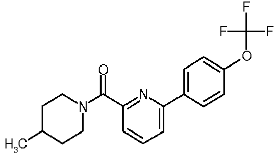 | 365.2 | 100 |
| 785 | 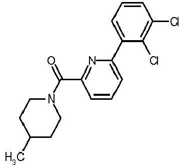 | 349.1 | 100 |
| 786 | 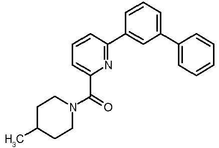 | 355.21 | 86 |
| 787 | 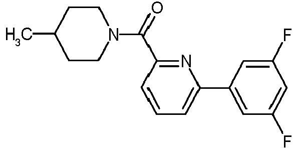 | 317.26 | 100 |
| 788 | 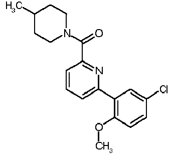 | 345.22 | 98 |
| 789 | 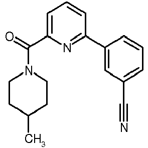 | 306.28 | 100 |
| 790 | 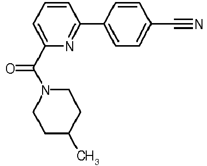 | 306.29 | 100 |
| 791 | 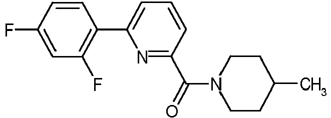 | 317.26 | 96 |
| 792 | 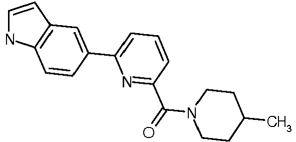 | 320.3 | 91 |
| 793 | 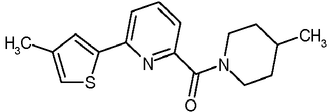 | 301.24 | 84 |
| 794 | 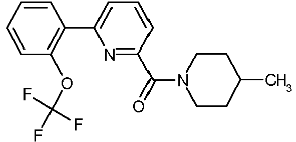 | 365.21 | 100 |
| 795 | 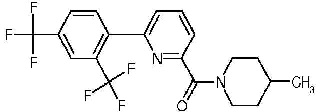 | 417.2 | 92 |
| 796 | 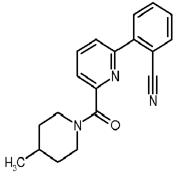 | 306.27 | 90 |
| 797 | 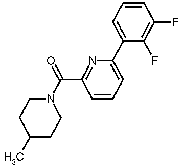 | 317.26 | 97 |
| 798 | 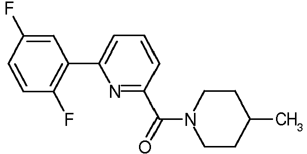 | 317.26 | 100 |
| 799 | 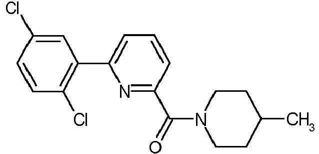 | 349.15 | 85 |
| 800 | 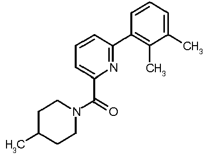 | 309.31 | 87 |
| 801 | 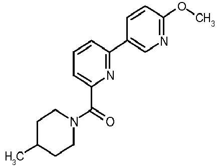 | 312.26 | 100 |
| 802 | 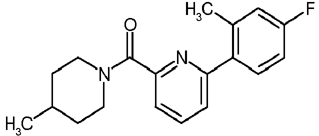 | 313.28 | 100 |
| 803 | 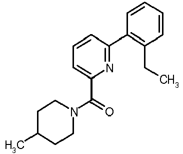 | 309.3 | 100 |
| 804 | 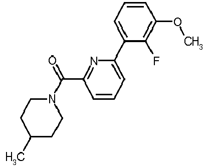 | 329.26 | 83 |
| 805 | 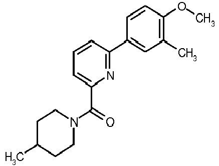 | 325.28 | 100 |
| 806 | 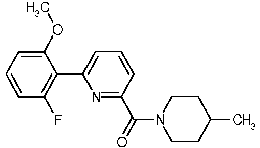 | 329.26 | 82 |
| 807 | 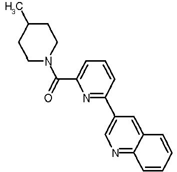 | 332.27 | 100 |
| 808 | 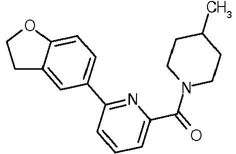 | 323.27 | 89 |
| 809 | 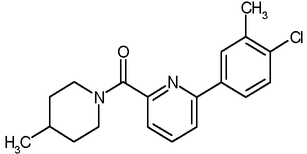 | 329.23 | 86 |
| 810 | 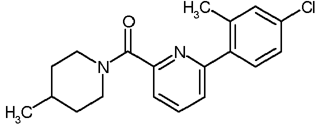 | 329.23 | 82 |
| 811 | 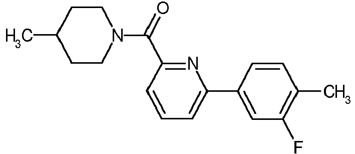 | 313.27 | 100 |
| 812 | 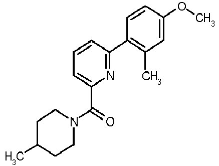 | 325.26 | 83 |
| 813 | 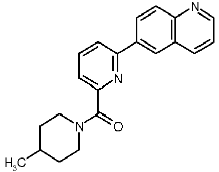 | 332.26 | 100 |
| 814 | 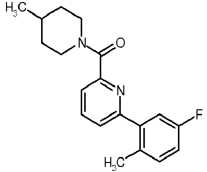 | 313.29 | 100 |
| 815 | 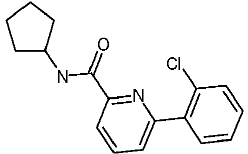 | 301.22 | 100 |
| 816 | 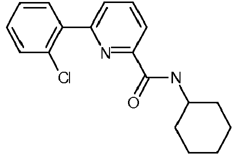 | 315.22 | 100 |
| 817 | 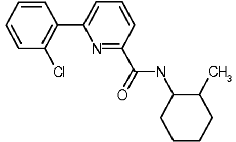 | 329.26 | 100 |
| 818 | 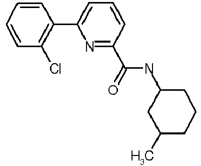 | 329.23 | 100 |
| 819 | 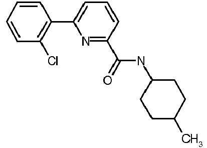 | 329.23 | 100 |
| 820 | 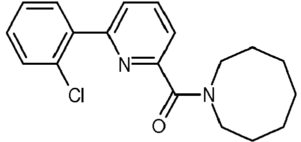 | 329.24 | 100 |
| 821 | 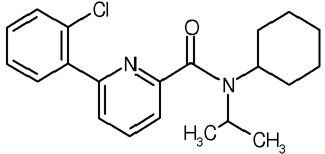 | 357.23 | 100 |
| 822 | 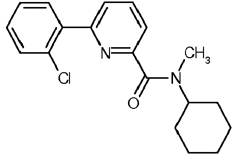 | 329.26 | 80 |
| 823 | 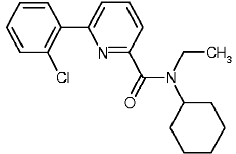 | 343.26 | 100 |
| 824 | 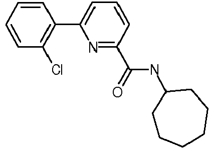 | 329.23 | 100 |
| 825 | 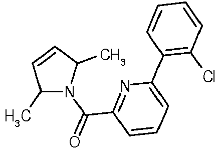 | 313.23 | 100 |
| 826 | 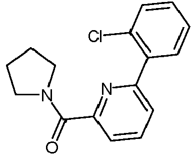 | 287.2 | 100 |
| 827 | 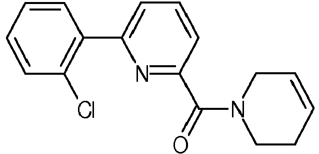 | 299.2 | 84 |
| 828 | 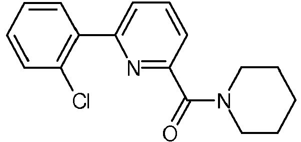 | 301.16 | 100 |
| 829 | 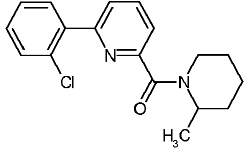 | 315.23 | 100 |
| 830 | 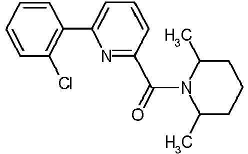 | 329.23 | 100 |
| 831 | 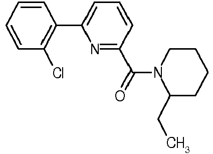 | 329.24 | 100 |
| 832 | 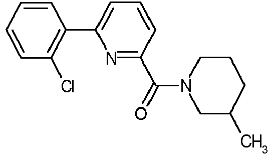 | 315.25 | 100 |
| 833 | 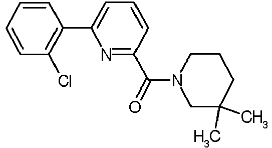 | 329.21 | 100 |
| 834 | 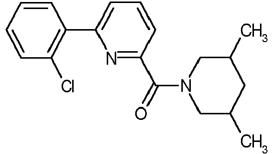 | 329.25 | 100 |
| 835 | 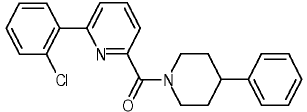 | 377.22 | 100 |
| 836 | 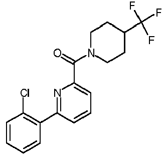 | 369.2 | 100 |
| 837 | 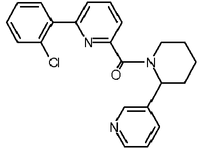 | 378.23 | 100 |
| 838 | 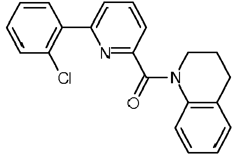 | 349.17 | 100 |
| 839 | 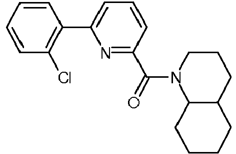 | 355.21 | 100 |
| 840 | 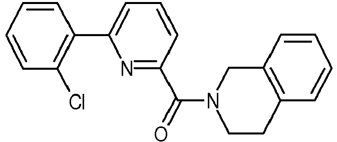 | 349.18 | 100 |
| 841 | 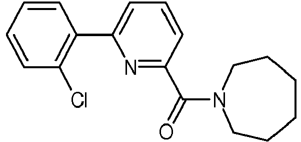 | 315.25 | 100 |
| 842 | 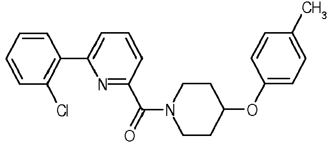 | 407.22 | 100 |
| 843 | 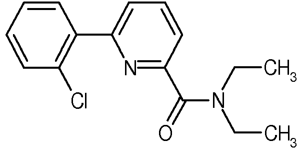 | 289.22 | 88 |
| 844 | 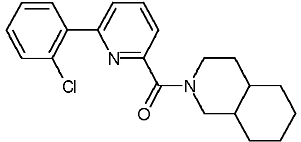 | 355.24 | 100 |
| 845 | 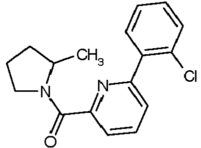 | 301.25 | 100 |
| 846 | 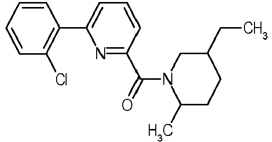 | 343.23 | 100 |
| 847 | 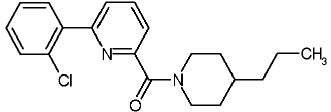 | 343.26 | 100 |
| 848 | 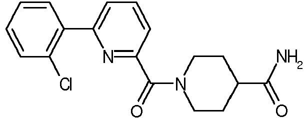 | 344.21 | 100 |
| 849 | 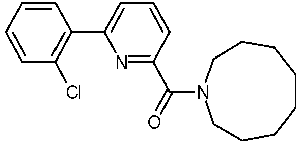 | 343.24 | 100 |
| 850 | 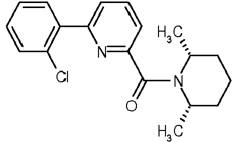 | 329.25 | 100 |
| 851 | 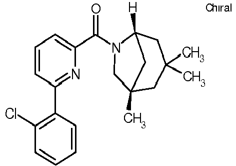 | 369.26 | 100 |
| 852 | 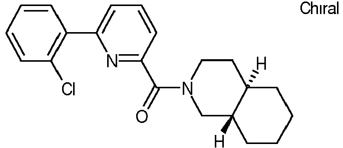 | 355.24 | 100 |
| 853 | 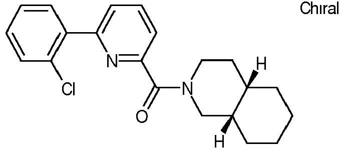 | 355.24 | 100 |
| 854 | 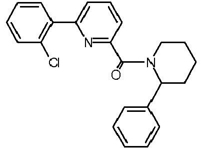 | 377.22 | 100 |
| 855 | 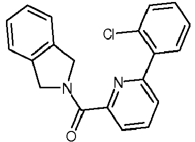 | 335.19 | 100 |
| 856 | 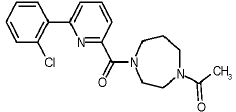 | 358.19 | 100 |
| 857 | 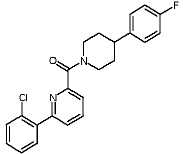 | 395.2 | 100 |
| 858 | 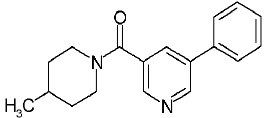 | 281 | 99.0 |
| 859 | 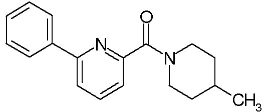 | 281 | 100 |
| 860 | 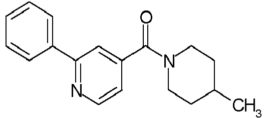 | 281 | 100 |
| 861 | 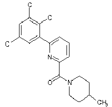 | 383.14 | 100 |
| 862 | 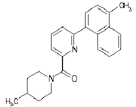 | 343.21 | 94 |
| 863 | 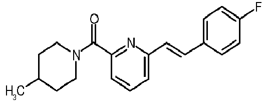 | 325.32 | 93 |
| 864 | 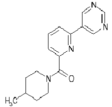 | 281.3 | 92 |
| 865 | 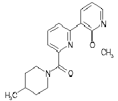 | 312.3 | 92 |
| 866 | 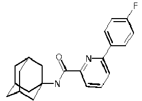 | 351.28 | 100 |
| 867 | 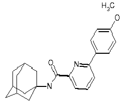 | 363.29 | 100 |
| 868 | 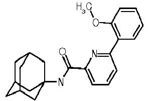 | 363.36 | 91 |
| 869 | 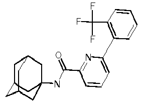 | 401.3 | 94 |
| 870 | 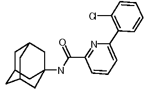 | 367.3 | 95 |
| 871 | 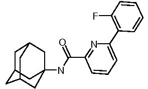 | 351.33 | 100 |
| 872 | 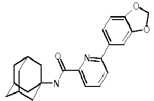 | 377.32 | 90 |
| 873 | 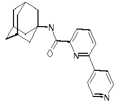 | 334.34 | 97 |
| 874 | 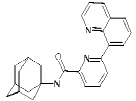 | 384.37 | 94 |
| 875 | 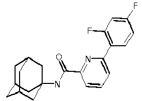 | 369.37 | 83 |
| 876 | 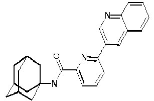 | 384.37 | 100 |
| 877 | 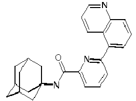 | 384.37 | 88 |
| 878 | 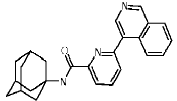 | 384.37 | 92 |
| 879 | 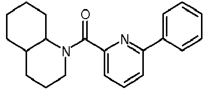 | 321.41 | 94 |
| 880 | 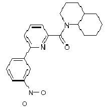 | 366.39 | 97 |
| 881 | 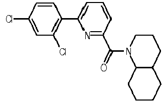 | 389.27 | 91 |
| 882 | 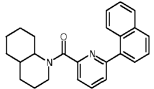 | 371.38 | 100 |
| 883 | 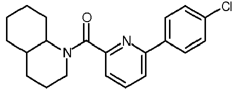 | 355.35 | 89 |
| 884 | 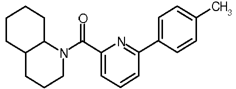 | 335.44 | 100 |
| 885 | 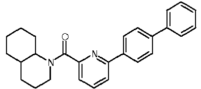 | 397.43 | 100 |
| 886 | 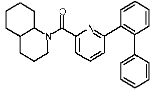 | 397.43 | 85 |
| 887 | 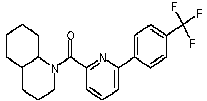 | 389.34 | 91 |
| 888 | 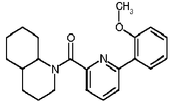 | 351.4 | 90 |
| 889 | 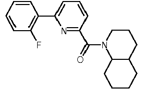 | 339.24 | 88 |
| 890 | 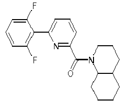 | 357.23 | 83 |
| 891 | 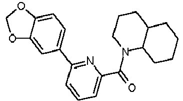 | 365.22 | 88 |
| 892 | 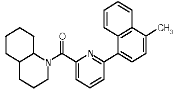 | 385.29 | 96 |
| 893 | 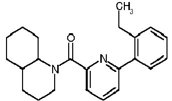 | 349.28 | 87 |
| 894 | 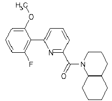 | 369.08 | 100 |
| 895 | 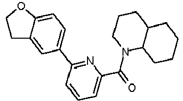 | 363.31 | 82 |
| 896 | 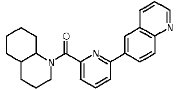 | 372.3 | 95 |
| 897 | 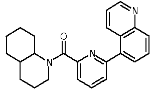 | 372.3 | 83 |
| 898 | 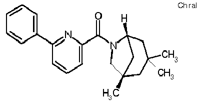 | 335.32 | 97 |
| 899 | 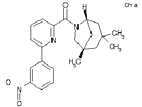 | 380.32 | 94 |
| 900 | 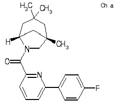 | 353.3 | 100 |
| 901 | 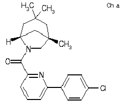 | 369.26 | 100 |
| 902 | 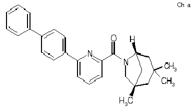 | 411.39 | 100 |
| 903 | 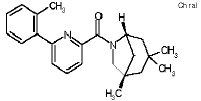 | 349.44 | 100 |
| 904 | 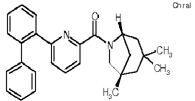 | 411.43 | 99 |
| 905 | 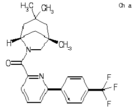 | 403.37 | 100 |
| 906 | 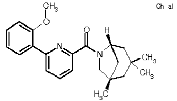 | 365.43 | 100 |
| 907 | 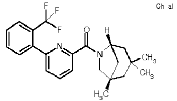 | 403.37 | 100 |
| 908 | 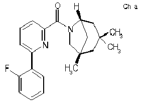 | 353.41 | 100 |
| 909 | 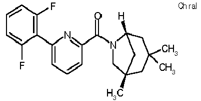 | 371.37 | 97 |
| 910 | 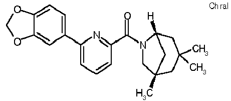 | 379.39 | 100 |
| 911 | 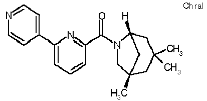 | 336.41 | 98 |
| 912 | 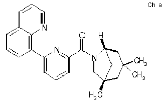 | 386.44 | 100 |
| 913 | 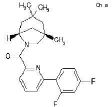 | 371.37 | 100 |
| 914 | 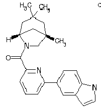 | 374.41 | 91 |
| 915 | 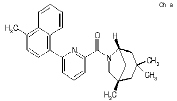 | 399.43 | 100 |
| 916 | 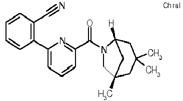 | 360.39 | 100 |
| 917 | 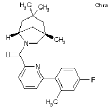 | 367.43 | 98 |
| 918 | 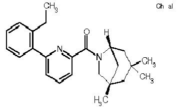 | 363.43 | 100 |
| 919 | 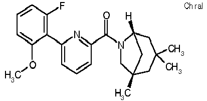 | 383.4 | 100 |
| 920 | 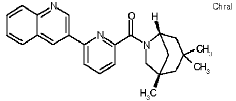 | 386.44 | 100 |
| 921 | 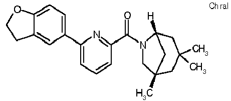 | 377.39 | 100 |
| 922 | 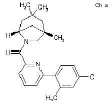 | 383.4 | 100 |
| 923 | 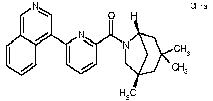 | 386.44 | 100 |

## Claims

1. A compound of formula I 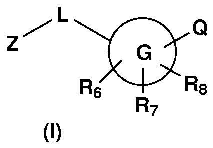 or stereoisomers or prodrugs or pharmaceutically acceptable salts thereof, for use in treating, preventing, or slowing the progression of a disease requiring 11beta-hydroxysteroid dehydrogenase type I inhibitor therapy, wherein:
Z is aryl or heterocyclyl group, and may be optionally substituted with R₁, R₂, R₃, R₄, and R₅ at any available positions;
R₁, R₂, R₃, R₄, and R₅ are independently hydrogen, halo, cyano, haloalkyl, haloalkoxy, nitro, alkyl, alkenyl, alkynyl, cycloalkyl, alkoxy, alkylthio, alkylsulfonyl, arylsulfonyl, alkylamino, -C(O)R₉, -NR₉C(O)R₉ₐ, -NR₉R₉ₐ, aryl, arylalkyl, aryloxy, or heterocyclyl, wherein the haloalkyl, haloalkoxy, alkyl, alkenyl, alkynyl, cycloalkyl, alkoxy, alkylthio, alkylsulfonyl, arylsulfonyl, alkylamino, aryl, arylalkyl, or heterocyclyl, may be optionally substituted with R₉ and R₉ₐ;
or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, alkenyl, alkynyl, cycloalkyl, -C(O)NR₉R₉ₐ, -C(O)R₉, -NR₉C(O)R₉ₐ, aryl, aryloxy, or heterocyclyl, wherein the haloalkyl, alkyl, alkenyl, alkynyl, cycloalkyl, aryl, aryloxy, or heterocyclyl may be optionally substituted with R₉ and R₉;
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl;
L is a bond, O, S, SO₂, NR₄ₐ, OCR₄ₐR_{4b}, CR₄ₐR_{4b}O, SCR₄ₐR_{4b}, CR₄ₐR_{4b}S, SO₂CR₄ₐR_{4b}, CR₄ₐR_{4b}SO₂, CR₄ₐR4_{b}CR_{4c}R_{4d}, CR₄ₐ=CR_{4b}, or OCONR_{4b};
R₄ₐ, R_{4b}, R_{4c}, and R_{4d} are independently hydrogen, alkyl or haloalkyl, wherein the alkyl and haloalkyl may be optionally substituted with R₁₀, R₁₀ₐ, R_{10b}, and R_{10c};
G is a 5- or 6-membered heteroaryl containing at least one nitrogen;
R₆, R₇, and R₈ are independently hydrogen, halo, haloalkyl, haloalkoxy, alkyl, aryl, heterocyclyl, alkoxy, aryloxy;
Q is OCONR₁₁R₁₁ₐ or CONR₁₁R₁₁ₐ;
R₁₁ and R₁₁ₐ are independently hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c};
or R₁₁ and R₁₁ₐ may be taken together with the nitrogen to which they are attached to form a heterocyclyl ring, which may be optionally substituted with R₁₀, R₁₀ₐ, R_{10b}, and R_{10c}.

2. A compound of the formula I 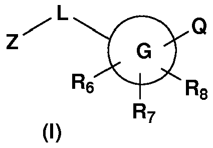 or stereoisomers or prodrugs or pharmaceutically acceptable salts thereof, for use in inhibiting the enzyme 11beta-hydroxysteroid dehydrogenase type I wherein:
Z is aryl or heterocyclyl group, and may be optionally substituted with R₁, R₂, R₃, R₄, and R₅ at any available positions;
R₁, R₂, R₃, R₄, and R₅ are independently hydrogen, halo, cyano, haloalkyl, haloalkoxy, nitro, alkyl, alkenyl, alkynyl, cycloalkyl, alkoxy, alkylthio, alkylsulfonyl, arylsulfonyl, alkylamino, -C(O)R₉, -NR₉C(O)R₉ₐ, -NR₉R₉ₐ, aryl, arylalkyl, aryloxy, or heterocyclyl, wherein the haloalkyl, haloalkoxy, alkyl, alkenyl, alkynyl, cycloalkyl, alkoxy, alkylthio, alkylsulfonyl, arylsulfonyl, alkylamino, aryl, arylalkyl, or heterocyclyl, may be optionally substituted with R₉ and R₉ₐ;
or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, alkenyl, alkynyl, cycloalkyl, -C(O)NR₉R₉ₐ, -C(O)R₉, -NR₉C(O)R₉ₐ, aryl, aryloxy, or heterocyclyl, wherein the haloalkyl, alkyl, alkenyl, alkynyl, cycloalkyl, aryl, aryloxy, or heterocyclyl may be optionally substituted with R₉ and R₉ₐ;
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl;
L is a bond, O, S, SO₂, NR₄ₐ, OCR₄ₐR_{4b}, CR₄ₐR_{4b}O, SCR₄ₐR_{4b}, CR₄ₐR_{4b}S, SO₂CR₄ₐR_{4b}, CR₄ₐR_{4b}SO₂, CR₄ₐR_{4b}CR_{4c}R_{4d}, CR₄ₐ=CR_{4b}, or OCONR_{4b};
R₄ₐ, R_{4b}, R_{4c}, and R_{4d} are independently hydrogen, alkyl or haloalkyl, wherein the alkyl and haloalkyl may be optionally substituted with R₁₀, R₁₀ₐ, R_{10b}, and R_{10c};
G is a 5- or 6-membered heteroaryl containing at least one nitrogen;
R₆, R₇, and R₈ are independently hydrogen, halo, haloalkyl, haloalkoxy, alkyl, aryl, heterocyclyl, alkoxy, aryloxy;
Q is or OCONR₁₁R₁₁ₐ, or CONR₁₁R₁₁ₐ,
R₁₁ and R₁₁ₐ are independently hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c};
or R₁₁ and R₁₁ₐ may be taken together with the nitrogen to which they are attached to form a heterocyclyl ring, which may be optionally substituted with R₁₀, R₁₀ₐ, R_{10b}, and R_{10c}.

3. A compound of the formula I 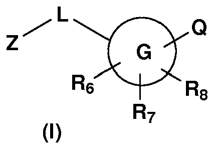 or stereoisomers or prodrugs or pharmaceutically acceptable salts thereof, wherein:
Z is aryl or heterocyclyl group, and may be optionally substituted with R₁, R₂, R₃, R₄, and R₅ at any available positions;
R₁, R₂, R₃, R₄, and R₅ are independently hydrogen, halo, cyano, haloalkyl, haloalkoxy, nitro, alkyl, alkenyl, alkynyl, cycloalkyl, alkoxy, alkylthio, alkylsulfonyl, arylsulfonyl, alkylamino, -C(O)R₉, -NR₉C(O)R₉ₐ, -NR₉R₉ₐ, aryl, arylalkyl, aryloxy, or heterocyclyl, wherein the haloalkyl, haloalkoxy, alkyl, alkenyl, alkynyl, cycloalkyl, alkoxy, alkylthio, arylsulfonyl, alkylamino, aryl, arylalkyl, or heterocyclyl, may be optionally substituted with R₉ and R₉ₐ;
or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, alkenyl, alkynyl, cycloalkyl, -C(O)NR₉R₉ₐ, -C(O)R₉, -NR₉C(O)R₉ₐ, aryl, aryloxy, or heterocyclyl, wherein the haloalkyl, alkyl, alkenyl, alkynyl, cycloalkyl, aryl, aryloxy, or heterocyclyl may be optionally substituted with R₉ and R₉ₐ;
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl;
L is a bond, O, S, SO₂, NR₄ₐ, OCR₄ₐR_{4b}, CR₄ₐR_{4b}O, SCR₄ₐR_{4b}, CR₄ₐR_{4b}S, SO₂CR₄ₐR_{4b}, CR₄ₐR_{4b}SO₂, CR₄ₐR_{4b}CR_{4c}R_{4d}, CR₄ₐ=CR_{4b}, or OCONR_{4b};
R₄ₐ, R_{4b}, R_{4c}, and R_{4d} are independently hydrogen, alkyl or haloalkyl, wherein the alkyl and haloalkyl may be optionally substituted with R₁₀, R₁₀ₐ, R_{10b}, and R_{10c};
G is a 5- or 6-membered heteroaryl containing at least one nitrogen;
R₆, R₇, and R₈ are independently hydrogen, halo, haloalkyl, haloalkoxy, alkyl, aryl, heterocyclyl, alkoxy, aryloxy;
Q is OCONR₁₁R₁₁ₐ, or CONR₁₁R₁₁a;
R₁₁ is hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c};
R₁₁ₐ is haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c};
or R₁₁ and R₁₁ₐ may be taken together with the nitrogen to which they are attached to form a heterocyclyl ring, which may be optionally substituted with R₁₀, R₁₀ₐ, R_{10b}, and R_{10c}.

4. The compound of claim 3, wherein
Z is aryl or heterocyclyl group, and may be optionally substituted with R₁, R₂, R₃, R₄, and R₅ at any available positions;
R₁, R₂, R₃, R₄, and R₅ are independently hydrogen, halo, cyano, haloalkyl, haloalkoxy, nitro, alkyl, alkenyl, alkynyl, cycloalkyl, alkoxy, alkylthio, alkylsulfonyl, arylsulfonyl, alkylamino, -C(O)R₉, -NR₉C(O)R₉ₐ, -NR₉R₉ₐ, aryl, arylalkyl, aryloxy, or heterocyclyl, wherein the haloalkyl, haloalkoxy, alkyl, alkenyl, alkynyl, cycloalkyl, alkoxy, alkylthio, alkylsulfonyl, arylsulfonyl, alkylamino, aryl, arylalkyl, or heterocyclyl, may be optionally substituted with R₉ and R₉ₐ; or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R_{10c};
L is bond, O, S, SO₂, OCR₄ₐR_{4b}, CR₄ₐR_{4b}O, SCR₄ₐR_{4b}, CR₄ₐR_{4b}S, SO₂CR₄ₐR_{4b}, CR₄ₐR_{4b}SO₂, CR₄ₐR_{4b}CR_{4c}R_{4d}, CR₄ₐ=CR_{4b}, or OCONR_{4b};
R₄ₐ, R_{4b}, R_{4c}, and R_{4d} are independently hydrogen and alkyl, wherein the alkyl may be optionally substituted with R₁₀, R₁₀ₐ, R_{10b}, and R_{10c};
G is a 5- or 6-membered heteroaryl containing at least one nitrogen;
R₆, R₇, and R₈ are independently hydrogen, halo, haloalkyl, haloalkoxy, alkyl, aryl, heterocyclyl, alkoxy, aryloxy;
Q is OCONR₁₁R₁₁ₐ or CONR₁₁R₁₁ₐ;
R₁₁ and R₁₁ₐ are independently hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c};
or R₁₁ and R₁₁ₐ may be taken together with the nitrogen to which they are attached to form a heterocyclyl ring, which may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, alkenyl, alkynyl, cycloalkyl, -C(O)NR₉R₉ₐ, -C(O)R₉, -NR₉C(O)R₉ₐ, aryl, aryloxy, or heterocyclyl, wherein the haloalkyl, alkyl, alkenyl, alkynyl, cycloalkyl, aryl, aryloxy, or heterocyclyl may be optionally substituted with R₉ and R₉ₐ; and
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl.

5. The compound of claim 3, wherein:
Z is aryl or heterocyclyl group, and may be optionally substituted with R₁, R₂, R₃, R₄, and R₅ at any available positions;
R₁, R₂, R₃, R₄, and R₅ are independently hydrogen, halo, cyano, haloalkyl, haloalkoxy, nitro, alkyl, alkenyl, alkynyl, cycloalkyl, alkoxy, alkylthio, alkylsulfonyl, arylsulfonyl, alkylamino, -C(O)R₉, -NR₉C(O)R₉ₐ, -NR₉R₉ₐ, aryl, arylalkyl, aryloxy, or heterocyclyl, wherein the haloalkyl, haloalkoxy, alkyl, alkenyl, alkynyl, cycloalkyl, alkoxy, alkylthio, alkylsulfonyl, arylsulfonyl, alkylamino, aryl, arylalkyl, or heterocyclyl, may be optionally substituted with R₉ and R₉ₐ;
or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R_{10c};
L is a bond, OCR₄ₐR_{4b}, CR₄ₐR_{4b}O, SCR₄ₐR_{4b}, CR₄aR_{4b}S, SO₂CR₄ₐR_{4b}, CR₄ₐR_{4b}SO₂, CR₄ₐR_{4b}CR_{4c}R_{4dc}, or CR₄ₐ=CR_{4b};
R₄ₐ, R_{4b}, R_{4c}, and R_{4d} are independently hydrogen, alkyl or haloalkyl, wherein the alkyl or haloalkyl may be optionally substituted with R₁₀, R₁₀ₐ, R_{10b}, and R_{10c};
G is a 5- or 6-membered heteroaryl containing at least one nitrogen;
R₆, R₇, and R₈ are independently hydrogen, halo, haloalkyl, haloalkoxy, alkyl, aryl, heterocyclyl, alkoxy, aryloxy;
Q is OCONR₁₁R₁₁ₐ;
R₁₁ and R₁₁ₐ are independently hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c};
or R₁₁ and R₁₁ₐ may be taken together with the nitrogen to which they are attached to form a heterocyclyl ring, which may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, alkenyl, alkynyl, cycloalkyl, -C(O)NR₉R₉ₐ, - C(O)R₉, -NR₉C(O)R₉ₐ, aryl, aryloxy, or heterocyclyl, wherein the haloalkyl, alkyl, alkenyl, alkynyl, cycloalkyl, aryl, aryloxy, or heterocyclyl may be optionally substituted with R₉ and R₉ₐ; and
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl.

6. The compound of claim 3, wherein:
Z is an aryl or heteroaryl of the following structure: 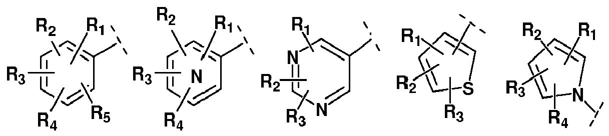 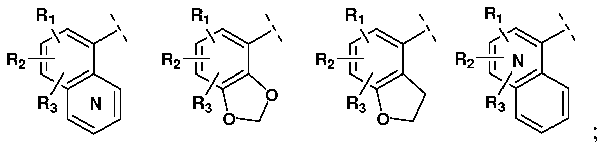
L is a bond, OCR₄ₐR_{4b}, CR₄ₐR_{4b}O, SCR₄ₐR_{4b}, CR₄ₐR_{4b}S, SO₂CR₄ₐR_{4b}, CR₄ₐR_{4b}SO₂, CR₄ₐR_{4b}CR_{4c}R_{4d}, or CR₄ₐ=CR_{4b}; and
G is a 5- or 6-membered heteroaryl containing at least one nitrogen of the following structure:

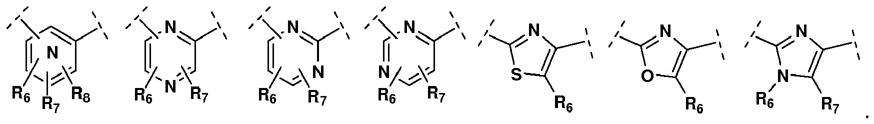

7. The compound of claim 3, wherein:
Z is 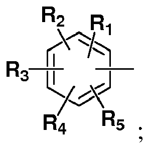
R₁, R₂, R₃, R₄, and R₅ are independently hydrogen, halo, cyano, haloalkyl, haloalkoxy, nitro, alkyl, alkenyl, alkynyl, cycloalkyl, alkoxy, alkylthio, alkylsulfonyl, arylsulfonyl, alkylamino, -C(O)R₉, -NR₉C(O)R₉ₐ, -NR₉R₉ₐ, aryl, arylalkyl, aryloxy, or heterocyclyl, wherein the haloalkyl, haloalkoxy, alkyl, alkenyl, alkynyl, cycloalkyl, alkoxy, alkylthio, alkylsulfonyl, arylsulfonyl, alkylamino, aryl, arylalkyl, or heterocyclyl, may be optionally substituted with R₉ and R₉ₐ;
or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R_{10c};
L is a bond, OCR₄ₐR_{4b}, SCR₄ₐR_{4b}, or SO₂CR₄ₐR_{4b};
R₄ₐ and R_{4b} are independently hydrogen, alkyl, or haloalkyl;
G is a 5- or 6-membered heteroaryl containing at least one nitrogen of the following structure: 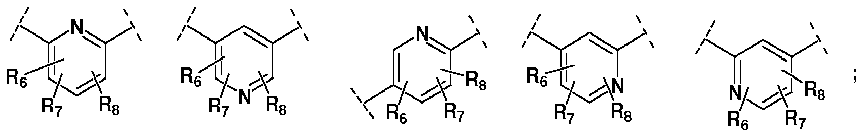
R₆, R₇, and R₈ are independently hydrogen, halo, haloalkyl, haloalkoxy, alkyl, aryl, heterocyclyl, alkoxy, aryloxy;
Q is OCONR₁₁R₁₁ₐ;
R₁₁ and R₁₁ₐ are independently hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c};
or R₁₁ and R₁₁ₐ may be taken together with the nitrogen to which they are attached to form a heterocyclyl ring, which may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, alkenyl, alkynyl, cycloalkyl, -C(O)NR₉R₉ₐ, -C(O)R₉, -NR₉C(O)R₉ₐ, aryl, aryloxy, or heterocyclyl, wherein the haloalkyl, alkyl, alkenyl, alkynyl, cycloalkyl, aryl, aryloxy, or heterocyclyl may be optionally substituted with R₉ and R₉ₐ; and
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl.

8. The compound of claim 3, wherein:
Z is 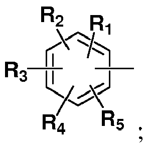
R₁, R₂, R₃, R₄, and R₅ are independently hydrogen, halo, cyano, haloalkyl, haloalkoxy, nitro, alkyl, cycloalkyl, alkoxy, alkylthio, alkylsulfonyl, arylsulfonyl, alkylamino, -C(O)R₉, -NR₉C(O)R₉ₐ, -NR₉R₉ₐ, aryl, arylalkyl, aryloxy, or heterocyclyl, wherein the haloalkyl, haloalkoxy, alkyl, alkenyl, alkynyl, cycloalkyl, alkoxy, alkylthio, alkylsulfonyl, arylsulfonyl, alkylamino, aryl, arylalkyl, or heterocyclyl, may be optionally substituted with R₉ and R₉ₐ;
or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R_{10c};
L is OCR₄ₐR_{4b}, SCR₄ₐR_{4b}, or SO₂CR₄ₐR_{4b};
R₄ₐ and R_{4b} are independently hydrogen, alkyl or haloalkyl;
G is a 5- or 6-membered heteroaryl containing at least one nitrogen of the following structure: 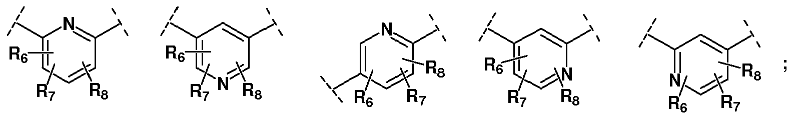
R₆, R₇, and R₈ are independently hydrogen, halo, haloalkyl, haloalkoxy, alkyl, aryl, heterocyclyl, alkoxy, aryloxy;
Q is OCONR₁₁R₁₁ₐ;
R₁₁ and R₁₁ₐ are independently hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c};
or R₁₁ and R₁₁ₐ may be taken together with the nitrogen to which they are attached to form a heterocyclyl ring, which may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, alkenyl, alkynyl, cycloalkyl, -C(O)NR₉R₉ₐ, -C(O)R₉, -NR₉C(O)R₉ₐ, aryl, aryloxy, or heterocyclyl, wherein the haloalkyl, alkyl, alkenyl, alkynyl, cycloalkyl, aryl, aryloxy, or heterocyclyl may be optionally substituted with R₉ and R₉ₐ; and
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl.

9. The compound of claim 3, wherein:
Z is 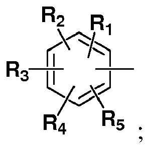
R₁, R₂, R₃, R₄, and R₅ are independently hydrogen, halo, cyano, haloalkyl, haloalkoxy, nitro, alkyl, cycloalkyl, alkoxy, alkylthio, alkylsulfonyl, arylsulfonyl, alkylamino, -C(O)R₉, -NR₉C(O)R₉ₐ, -NR₉R₉ₐ, aryl, arylalkyl, aryloxy, or heterocyclyl, wherein the haloalkyl, haloalkoxy, alkyl, alkenyl, alkynyl, cycloalkyl, alkoxy, alkylthio, alkylsulfonyl, arylsulfonyl, alkylamino, aryl, arylalkyl, or heterocyclyl, may be optionally substituted with R₉ and R₉ₐ;
or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R_{10c};
L is OCR₄ₐR_{4b} or SO₂CR₄ₐR_{4b};
R₄ₐ and R_{4b} are independently hydrogen, alkyl, or haloalkyl;
G is a 5- or 6-membered heteroaryl containing at least one nitrogen of the following structure: 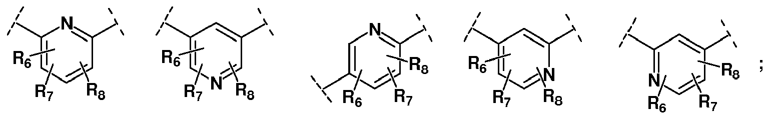
R₆, R₇, and R₈ are independently hydrogen, halo, haloalkyl, haloalkoxy, alkyl, aryl, heterocyclyl, alkoxy, aryloxy;
Q is OCONR₁₁R₁₁a;
R₁₁ and R₁₁ₐ are independently hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c};
or R₁₁ and R₁₁ₐ may be taken together with the nitrogen to which they are attached to form a heterocyclyl ring, which may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, alkenyl, alkynyl, cycloalkyl, -C(O)NR₉R₉ₐ, -C(O)R₉, -NR₉C(O)R₉ₐ, aryl, aryloxy, or heterocyclyl, wherein the haloalkyl, alkyl, alkenyl, alkynyl, cycloalkyl, aryl, aryloxy, or heterocyclyl may be optionally substituted with R₉ and R₉ₐ; and
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl.

10. The compound of 3, wherein:
Z is 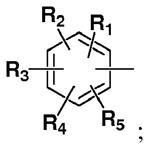
R₁, R₂, R₃, R₄, and R₅ are independently hydrogen, halo, cyano, haloalkyl, haloalkoxy, nitro, alkyl, cycloalkyl, alkoxy, alkylthio, alkylsulfonyl, arylsulfonyl, alkylamino, aryl, arylalkyl, aryloxy, or heterocyclyl, wherein the haloalkyl, haloalkoxy, alkyl, cycloalkyl, alkoxy, alkylthio, alkylsulfonyl, arylsulfonyl, alkylamino, aryl, arylalkyl, or heterocyclyl, may be optionally substituted with R₉ and R₉ₐ; or independently any two adjoining R₁, R₂, R₃, R₄, and/or R₅ may be taken together to form a fused aryl or heterocyclyl ring, which may be may be optionally substituted with R₁₀, R₁₀ₐ, R_{10b}, and R₁₀c;
L is OCR₄ₐR_{4b} or SO₂CR₄ₐR_{4b};
R₄ₐ and R_{4b} are independently hydrogen or alkyl;
G is a 5- or 6-membered heteroaryl containing at least one nitrogen of the following structure: 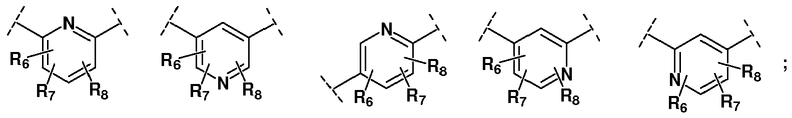
R₆, R₇, and R₈ are independently hydrogen, halo, haloalkyl, haloalkoxy, alkyl, aryl, or heterocyclyl;
Q is OCONR₁₁R₁₁ₐ;
R₁₁ and R₁₁ₐ are independently hydrogen, haloalkyl, alkyl, cycloalkyl, aryl, arylalkyl, or heterocyclyl, wherein the alkyl, cycloalkyl, aryl, arylalkyl, or heterocyclyl may be optionally substituted with R₁₀, R₁₀ₐ, R_{10b}, and R_{10c};
or R₁₁ and R₁₁ₐ may be taken together with the nitrogen to which they are attached to form a heterocyclyl ring, which may be optionally substituted with R₁₀, R₁₀ₐ, R_{10b}, and R_{10c};
R₁₀, R₁₀ₐ, R_{10b}, and R_{10c} are independently selected from hydrogen, halo, hydroxy, nitro, cyano, haloalkyl, alkyl, cycloalkyl, aryl, aryloxy, or heterocyclyl, wherein the haloalkyl, alkyl, cycloalkyl, aryl, aryloxy, or heterocyclyl may be optionally substituted with R₉ and R₉ₐ; and
R₉ and R₉ₐ are independently hydrogen, alkyl, alkoxy, cycloalkyl, aryl, or heterocyclyl, wherein the alkyl, alkoxy, cycloalkyl, aryl, or heterocyclyl may be optionally substituted with halo, haloalkyl, alkyl, aryl, or heterocyclyl.

11. A compound claim selected from the compounds exemplified in Examples 1 to 923.

12. A compound of any of claims 3 to 11, for use in therapy.

13. A compound of any of claims 3 to 11, for use in treating, preventing or slowing the progression of diabetes, macrovascular complications of diabetes, microvascular complications of diabetes, cardiovascular diseases, or Metabolic Syndrome.

14. A compound of any of claims 3 to 11, for use in treating, preventing or slowing the progression of diabetes, macrovascular complications of diabetes, hyperglycemia, impaired glucose tolerance, insulin resistance, hyperinsulinemia, retinopathy, neuropathy, nephropathy, delayed wound healing, atherosclerosis and its sequelae, abnormal heart function, myocardial ischemia, stroke, Metabolic Syndrome, hypertension, obesity, dislipidemia, dylsipidemia, hyperlipidemia, hypertriglyceridemia, hypercholesterolemia, low HDL, high LDL, non-cardiac ischemia, infection, cancer, vascular restenosis, pancreatitis, neurodegenerative disease, lipid disorders, cognitive impairment and dementia, bone disease, HIV protease associated lipodystrophy and glaucoma.

15. A pharmaceutical composition comprising a compound of claims 3 to 11.
